(19) Europäisches Patentamt
European Patent Office
Office européen des brevets

(11) EP 3 556 388 A1

(12) **EUROPEAN PATENT APPLICATION**
published in accordance with Art. 153(4) EPC

(43) Date of publication:
**23.10.2019 Bulletin 2019/43**

(21) Application number: **17881449.7**

(22) Date of filing: **19.06.2017**

(51) Int Cl.:
***A61K 38/48*** *(2006.01)* ***A61P 13/12*** *(2006.01)*

(86) International application number:
**PCT/CN2017/089060**

(87) International publication number:
**WO 2018/107700 (21.06.2018 Gazette 2018/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.12.2016 PCT/CN2016/110169**
**15.12.2016 PCT/CN2016/110174**

(71) Applicant: **Talengen International Limited**
**Wanchai**
**Hong Kong (CN)**

(72) Inventor: **LI, Jinan**
**Shenzhen**
**Guangdong 518020 (CN)**

(74) Representative: **karo IP**
**Kahlhöfer Rößler Kreuels**
**Patentanwälte PartG mbB**
**Postfach 32 01 02**
**DE-40416 Düsseldorf (DE)**

# (54) METHOD FOR PREVENTING AND TREATING PATHOLOGICAL RENAL TISSUE INJURY

(57) The present invention relates a method for preventing and/or treating a renal tissue injury in a subject, comprising administering an effective amount of plasminogen to the subject, wherein the subject has a risk of the renal tissue injury, is suspected of having the renal tissue injury or suffers from the renal tissue injury. The present invention further relates to a medicament, a pharmaceutical composition, an article of manufacture, and a kit comprising plasminogen which are useful for preventing and/or treating a renal tissue injury and its related conditions in a subject.

EP 3 556 388 A1

## Description

## Technical Field

The present invention relates to the effect of plasminogen in the prevention and treatment of nephropathy, thereby providing a brand new therapeutic strategy for treating nephropathy and its related conditions caused by different reasons.

## Background Art

Nephropathy is a renal structural change and dysfunction caused by various reasons. Nephropathy causes injuries to a structure of a renal tissue, thereby affecting its function. Nephropathy can be primary, such as glomerulonephritis, chronic pyelonephritis, nephrotic syndrome, renal insufficiency, glomerular sclerosis, glomerular mesangial hyperplasia, tubulointerstitial lesions, renal tubular atrophy and the like caused by an infection, an inflammation, an allergic reaction, and the like; and can also be secondary to other diseases, for instance, nephropathy can be caused by ischemia, metabolic disorders such as a glucose metabolism disorder and a fat metabolism disorder, and other diseases such as tumors.

For instance, hypertension, diabetes mellitus, atherosclerosis, and other diseases are often accompanied by nephropathy. Hypertension is one of common chronic diseases, the main manifestation of which is increased systemic arterial pressure. If the blood pressure of hypertensive patients is not well controlled, complications are easily developed such as cerebral stroke, coronary heart disease, retinopathy, and a chronic kidney disease. In addition, prolonged hypertension can affect more and more tissues and organs. Therefore, we should strengthen and prevent hypertension and its complications, so as to reduce the harm of hypertension[1].

Diabetic nephropathy (DN) is a common and important complication of diabetes mellitus, and is a major cause of mortality and disability in diabetic patients. If the diagnosis and treatment is not timely, when DN develops to end-stage renal disease, only dialysis or even kidney transplantation will be adopted. The main pathological features of DN in an early stage are glomerular hypertrophy, thickening of glomerular and tubular basement membrane, and progressive accumulation of extracellular matrix in the mesangial area; and those in a later stage are glomerular and tubulointerstitial fibrosis. Early clinical manifestations comprise a reduced glomerular filtration rate, followed by microalbuminuria, an elevated arterial blood pressure, proteinuria, and fluid retention, ultimately leading to renal failure[2].

Diabetic nephropathy is a diabetic microvascular complication, the development of which is related to factors such as hyperglycemia and oxidative stress, wherein hyperglycemia is an important factor in the production of microalbuminuria[3]. Microalbuminuria may predict the progression of diabetic nephropathy. Diabetic atherosclerosis is a macrovascular complication of diabetes mellitus, which is closely related to hyperglycemia, vascular endothelial dysfunction, insulin resistance, and other factors. Recent studies have found that proteinuria is closely related to atherosclerosis[4].

In developed countries, diabetic nephropathy and hypertensive renal arteriolosclerosis have become the main causes of a chronic kidney disease. In China, these two diseases still rank behind primary glomerulonephritis in various causes, but there is a significant uptrend in recent years.

Systemic allergic diseases, such as systemic sclerosis, systemic lupus erythematosus, systemic vasculitis, anaphylactoid purpura, polymyositis, and thrombotic microangiopathy, often affect the kidneys.

Most drugs and their metabolites are excreted by the kidneys, and thus the incidence of a drug-induced renal injury is very high. Studies have shown that the incidence of drug-induced acute tubular necrosis (ATN) or acute interstitial nephritis (AIN) is up to 18.3%, and the incidence of an antibiotic-induced renal injury is up to 36%[5].

Anti-infective drugs, such as aminoglycoside antibiotics, are widely used to treat Gram-negative bacterial infections, but nephrotoxicity limits their clinical application[6].

The antiviral agent, aciclovir (ACV), is a cyclic analogue of deoxyguanosine against herpes virus. Parenteral administration of large doses of aciclovir can cause acute renal failure (ARF) in 10% to 48% of patients, which may be caused by renal obstruction due to aciclovir deposition in renal tubules, toxic immune response or hypersensitivity reaction, etc. [7]Adedovir (ADV) and cidofovir (CDV), as nucleoside analogues, are commonly used clinically to treat hepatitis B and AIDS. When nephrotoxicity occurs, it is usually manifested as tubular necrosis and interstitial fibrosis[8].

An immunosuppressive agent, such as cyclosporine A (CsA), is widely used in organ transplantation and treatment of an autoimmune disease as an immunosuppressive agent. It has been reported that about 30% of patients treated with CsA develop moderate to severe renal dysfunction. The mechanism of a renal injury comprises ischemia due to renal vasoconstriction and an endothelial cell injury, and the direct toxic effect of CsA on renal tubular epithelial cells[9].

Anti-tumor drugs, such as cisplatin (Cis) as a cell proliferation inhibitor, are widely used in the treatment of testicular cancer, ovarian cancer, prostate cancer, lung cancer, bone cancer, head and neck cancer, and other solid tumors. Cis has a high anti-tumor efficiency, but it also has dose-dependent nephrotoxicity, etc.[10], which is substantially manifested as azotemia, polyuria and renal failure, characterized by injuries of the glomeruli and renal tubules.

A non-steroidal anti-inflammatory drug, such as acetylsalicylic acid (ASA), is the most widely used antipyretic and analgesic anti-inflammatory drug in the world. The dose used for antipyretic and analgesic purposes rarely causes adverse reactions; however, the long-term heavy drug use can easily lead to side effects, the manifestations of which are escape of potassium ions from renal tubular cells due to decoupling of oxidative phosphorylation, resulting in potassium deficiency and excessive excretion of uric acid in urine, and in the case of a greater injury, interstitial nephritis, renal papillary necrosis, and renal hypofunction may occur[11].

Aristolochic acid (AA) as an active component of a natural product is a nephrotoxin derived from plants of Aristolochiaceae, and is the cause of the well-known aristolochic acid nephropathy (AAN)[12]. AA induces a renal injury mainly at sites of renal tubules, results in cell apoptosis or death, and inhibits the proliferation of renal interstitial fibroblasts, leading to renal tubular atrophy and oligo-cellular interstitial fibrosis.

Diuretics are drugs that increase urine output by inhibiting the reabsorption of water and electrolytes in renal tubules. All kinds of diuretics have potential nephrotoxicity, and might cause a renal injury after application. The nephrotoxicity caused by diuretics is related to the cytotoxicity, immune response, an allergic reaction, a metabolism disorder and other adverse reactions caused by such kinds of drugs, and the diuretics should be avoided to be used in combination with nephrotoxic drugs to prevent aggravation of the renal injury. In addition, there are a variety of drugs that can cause a renal injury, for instance, sulfonamides often cause the formation of sulfonamide crystals blocking the ureter and thus cause obstructive nephropathy[13]. Hypolipidemic drugs such as statins can cause rhabdomyolysis, which in turn leads to tubular necrosis[14].

Nephropathy will cause partial or total loss of renal function in a later stage, which is a pathological state leading to renal failure. Renal failure can be divided into acute renal failure and chronic renal failure. The condition of acute renal failure progresses rapidly, and generally due to insufficient supply of blood in the kidney, impaired renal function caused by obstruction due to a certain factor, or a toxin-induced renal injury, acute renal failure occurs. Chronic renal failure is mainly caused by long-term nephropathy, and as time goes on and the disease progresses, the renal function gradually declines, leading to the occurrence of renal failure.

Chronic renal failure refers to slow progressive renal function impairment caused by various kidney diseases, ultimately causing uremia and complete loss of renal function, and leading to a series of clinical symptoms as well as a clinical syndrome composed of a biochemical endocrine disorder and other metabolism disorders; and the interval from primary onset to start of renal failure may be several years to more than a decade.

Uremia is the end stage of chronic renal failure. Chronic renal failure is a result of renal injury and progressive deterioration caused by various causes. Common basic diseases comprise primary glomerulonephritis, tubulointerstitial nephritis, diabetic nephropathy, etc. Its clinical manifestations are mainly renal hypofunction, metabolic waste retention, water, electrolyte and acid-base imbalance, and thus failure of maintaining stability of the environment in the body. Chronic renal failure can be divided into four stages according to the degree of renal function impairment: compensatory stage of renal insufficiency, decompensatory stage of renal insufficiency, renal failure stage, and uremia stage.

In an early stage of chronic renal insufficiency, only the symptoms of primary diseases are observed clinically, and only a decrease in creatinine clearance can be observed in an examination. In patients in the compensatory stage of uremia, renal function often deteriorates abruptly under stress, accompanied by water, electrolyte and acid-base metabolism disorder, protein, glucose, fat and vitamin metabolism disorders, loss of appetite or dyspepsia; in the case of condition aggravated, anorexia, nausea, vomiting or diarrhea and other gastrointestinal symptoms, and other early symptoms of uremia may occur, clinically known as reversible uremia. Once the stress factors are removed, renal function can often be restored to the compensatory stage. If the condition progresses to a point where the "intact" nephron is unable to meet the minimum requirements of the body, uremic symptoms will gradually appear even without stress factors, and are manifested as systemic symptoms such as a water and electrolyte metabolism disorder, accumulation of metabolites in the body, etc., and moderate symptoms of the cardiovascular system, respiratory system, and blood system.

The drug therapies of chronic renal insufficiency mainly focuses on three aspects: relieving symptoms, delaying disease progression, and preventing and treating complications, specifically for instance, correction of acidosis as well as water and electrolyte disorder, treatment of hypertension, prevention and treatment of infections, treatment of hyperlipemia, and treatment of complications of the cardiovascular, respiratory, and blood systems, as well as alternative dialysis treatment for uremia at the same time, such as hemodialysis or peritoneal dialysis, or kidney transplantation.

"Acute renal injury (AKI)" is a new term proposed in recent years, which is used to replace the acute renal failure (ARF) used for many years, and has been widely recognized. It is a common clinical syndrome, which is substantially manifested by rapid decrease of renal function and accumulation of metabolic wastes. The incidence of AKI is high and is increasing year by year. It has been reported in foreign countries that the incidence of AKI has increased from 0.65‰ to 5%o in the past decade, and the incidence of AKI requiring dialysis is 0.295‰ [15-16]. The incidence of AKI in inpatients is 1.9%, and can be up to 60% in intensive care units[17]. Currently, there is no specific drug for AKI treatment, and renal replacement therapy is required for severe patients[18]. The prognosis of AKI is also not optimistic. The ATN and RENAL studies[119-20] reported that the mortality rates of AKI in critically ill patients were 53.0% and 44.7%, respectively, and the surviving patients were also prone to develop to chronic nephropathy and even to end-stage nephropathy[21]. Therefore, AKI has attracted more and more attention from clinicians.

For a renal tissue injury caused by various reasons, people have been eager to find more effective therapeutic drugs. The studies of the present invention found that plasminogen can promote repair of an injured renal tissue and recovery of renal function, and can further inhibit apoptosis of an injured renal tissue and reduce its fibrosis. Therefore, plasminogen is expected to become a novel drug for treating kidney diseases.

## Summary of the Invention

The present invention relates to the following items:

In one aspect, the present invention relates to: Item 1. A method for preventing and/or treating a renal tissue injury in a subject, comprising administering an effective amount of plasminogen to the subject, wherein the subject has a risk of the renal tissue injury, is suspected of having the renal tissue injury or suffers from the renal tissue injury.

Item 2. The method of item 1, wherein the renal tissue injury comprises a renal tissue injury caused by an infection, an inflammation, an allergic reaction, autoimmunity, ischemia, microangiopathy, a thrombus, a trauma, a radiation injury, a glucose metabolic disorder, an electrolyte disorder, a fat metabolism disorder, and a cancer.

Item 3. The method of item 1 or 2, wherein the renal tissue injury is a renal tissue injury caused by a systemic disease selected from hypertension, diabetes mellitus, atherosclerosis, systemic sclerosis, systemic lupus erythematosus, hyperlipemia, non-Hodgkin's lymphoma, multiple myeloma, systemic vasculitis, anaphylactoid purpura, polymyositis, and thrombotic microangiopathies.

Item 4. The method of item 1 or 2, wherein the renal tissue injury is a renal tissue injury caused by a chronic kidney disease.

Item5. The method of item 4, wherein the chronic kidney disease is chronic glomerulonephritis, chronic pyelonephritis, nephrotic syndrome, renal insufficiency, renal failure or uremia.

Item 6. The method of item 1 or 2, wherein the chronic kidney disease is a drug-induced chronic renal injury.

Item 7. The method of item 6, wherein the drug comprises a chemotherapeutic drug, an antihypertensive drug, a hypolipidemic drug, a hypoglycemic drug, a nonsteroid anti-inflammatory drug, an antibiotic drug, and an antiviral drug.

Item 8. The method of item 7, wherein the drug is a chemotherapeutic drug, in particular cisplatin.

In another aspect, the present invention relates to: Item 9. A method for preventing and/or treating an acute renal tissue injury in a subject, comprising administering an effective amount of plasminogen to the subject to protect the renal tissues.

Item 10. The method of item 9, wherein the plasminogen alleviates apoptosis in a renal tissue caused by an acute renal tissue injury.

Item 11. The method of item 9 or 10, wherein the plasminogen promotes repair of an injured renal tissue.

Item 12. The method of any one of items 9 to 11, wherein the plasminogen alleviates fibrosis of the injured renal tissue.

Item 13. The method of any one of items 9 to 12, wherein the plasminogen promotes recovery of renal function.

Item 14. The method of any one of items 1 to 13, wherein the renal injury is acute glomerulonephritis, acute pyelonephritis, an acute renal injury, acute renal failure, acute renal insufficiency, and acute tubular necrosis.

Item 15. The method of items 1 to 13, wherein the acute renal injury is an acute renal injury induced by a chemotherapeutic drug.

In another aspect, the present invention relates to: Item 16. A method for preventing and/or treating a chronic renal tissue injury in a subject, comprising administering an effective amount of plasminogen to the subject to protect the renal tissues.

Item 17. The method of item 16, wherein the plasminogen alleviates apoptosis in a renal tissue.

Item 18. The method of item 16 or 17, wherein the plasminogen promotes repair of an injured renal tissue.

Item 19. The method of any one of items 16 to 18, wherein the plasminogen alleviates fibrosis of the injured renal tissue.

Item 20. The method of any one of items 16 to 19, wherein the plasminogen promotes recovery of renal function.

Item 21. The method of any one of items 16 to 20, wherein the chronic renal injury is a renal tissue injury caused by a chronic renal tissue disease.

Item 22. The method of any one of items 16 to 20, wherein the chronic renal injury is a renal tissue injury elicited or accompanied by other diseases.

Item 23. The method of item 22, wherein the other diseases comprise hypertension, diabetes mellitus, atherosclerosis, hyperlipemia, hepatitis, hepatic cirrhosis, coronary heart disease, angina pectoris, and myocardial infarction.

Item 24. The method of item 23, wherein the other diseases are hypertension, diabetes mellitus, atherosclerosis, and hyperlipemia.

In another aspect, the present invention relates to: Item 25. A method for preventing and/or treating a lipid deposition-induced renal tissue injury in a subject, comprising administering an effective amount of plasminogen to the subject.

Item 26. The method of item 25, wherein the lipid deposition is induced by hyperlipemia caused by abnormal fat or glucose metabolism in the subject.

In another aspect, the present invention relates to: Item 27. A method for preventing and/or treating a renal tissue injury elicited or accompanied by diabetes mellitus in a subject, comprising administering an effective amount of plasminogen to the subject.

In another aspect, the present invention relates to: Item 28. A method for preventing and/or treating a renal tissue injury elicited or accompanied by hyperlipemia in a subject, comprising administering an effective amount of plasminogen to the subject.

In another aspect, the present invention relates to: Item 29. A method for preventing and/or treating a renal tissue injury elicited or accompanied by atherosclerosis in a subject, comprising administering an effective amount of plasminogen to the subject.

In another aspect, the present invention relates to: Item 30. A method for preventing and/or treating an ischemic renal tissue injury in a subject, comprising administering an effective amount of plasminogen to the subject.

Item 31. The method of item 30, wherein the plasminogen alleviates apoptosis in a renal tissue.

Item 32. The method of item 30 or 31, wherein the plasminogen promotes repair of an injured renal tissue.

Item 33. The method of any one of items 30 to 32, wherein the plasminogen alleviates fibrosis of the injured renal tissue.

Item 34. The method of any one of items 30 to 33, wherein the plasminogen promotes recovery of renal function.

Item 35. The method of any one of items 30 to 34, wherein the ischemia is caused by stenosis of a vascular lumen.

Item 36. The method of any one of items 30 to 34, wherein the ischemia is caused by a thrombus blocking a blood vessel.

Item 37. The method of item 35 or 36, wherein the ischemia is caused by hypertension, diabetes mellitus, atherosclerosis, and a heart disease.

In another aspect, the present invention relates to: Item 38. A method for preventing and/or treating a renal tissue injury induced by ischemic reperfusion in a subject, comprising administering an effective amount of plasminogen to the subj ect.

In another aspect, the present invention relates to: Item 39. A method for preventing and/or treating a renal tissue injury induced by an autoimmune response in a subject, comprising administering an effective amount of plasminogen to the subj ect.

Item 40. The method of item 39, wherein the renal tissue injury induced by an autoimmune response is caused by systemic sclerosis.

In another aspect, the present invention relates to: Item 41. A method for preventing and/or treating an inflammation-induced renal tissue injury in a subject, comprising administering an effective amount of plasminogen to the subject.

Item 42. The method of item 41, wherein the inflammation is an acute and chronic renal parenchymal inflammation or renal interstitial inflammation.

Item 43. The method of item 42, wherein the plasminogen alleviates apoptosis in a renal tissue.

Item 44. The method of item 42 or 43, wherein the plasminogen promotes repair of an injured renal tissue.

Item 45. The method of any one of items 41 to 44, wherein the plasminogen alleviates fibrosis of the injured renal tissue.

Item 46. The method of any one of items 41 to 45, wherein the plasminogen promotes recovery of renal function.

Item 47. The method of any one of items 1 to 46, wherein the plasminogen has at least 75%, 80%, 85%, 90%, 95%, 96%, 97%, 98% or 99% sequence identity with SEQ ID No. 2, 6, 8, 10 or 12, and still has the plasminogen activity.

Item 48. The method of any one of items 1 to 47, wherein the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No. 2, 6, 8, 10 or 12, and still has the plasminogen activity.

Item 49. The method of any one of items 1 to 48, wherein the plasminogen is a protein that comprises a plasminogen active fragment and still has the plasminogen activity.

Item 50. The method of any one of items 1 to 49, wherein the plasminogen is selected from Glu-plasminogen, Lys-plasminogen, mini-plasminogen, micro-plasminogen, delta-plasminogen or their variants that retain the plasminogen activity.

Item 51. The method of any one of items 1 to 50, wherein the plasminogen is administered in combination with one or more drugs selected from an antihypertensive drug, a hypolipidemic drug, an antibiotic drug, an anticoagulant drug, a thrombolytic drug, a diuretic drug, an anti-tumor drug, a hypoglycemic drug, a non-steroidal anti-inflammatory drug, an immunomodulatory drug, an anti-infective drug, an antiviral drug, a hormone, and an active ingredient of a natural product.

Item 52. The method of any one of items 1 to 51, wherein the plasminogen is a natural or synthetic human plasminogen, or a variant or fragment thereof that still retains the plasminogen activity.

Item 53. The method of any one of items 1 to 51, wherein the plasminogen is an ortholog of human plasminogen from a primate or a rodent, or a variant or fragment thereof that still retains the plasminogen activity.

Item 54. The method of any one of items 1 to 53, wherein the amino acids of the plasminogen are as shown in SEQ ID No. 2, 6, 8, 10 or 12.

Item 55. The method of any one of items 1 to 54, wherein the plasminogen is a natural human plasminogen.

Item 56. The method of any one of items 1 to 55, wherein the subject is a human.

Item 57. The method of any one of items 1 to 26, wherein the subject has a lack or deficiency of plasminogen.

Item 58. The method of item 57, wherein the lack or deficiency is congenital, secondary and/or local.

In another aspect, the present invention relates to: Item 59. A plasminogen for use in the method of any one of items 1 to 58.

In another aspect, the present invention relates to: Item 60. A pharmaceutical composition, comprising a pharmaceutically acceptable carrier and the plasminogen for use in the method of any one of items 1 to 58.

In another aspect, the present invention relates to: Item 61. A preventive or therapeutic kit comprising: (i) the plasminogen for use in the method of any one of items 1 to 58, and (ii) a means for delivering the plasminogen to the subject.

Item 62. The kit of item 61, wherein the means is a syringe or a vial.

Item 63. The kit of item 61 or 62, further comprising a label or an instruction for use indicating the administration of the plasminogen to the subject to implement the method of any one of items 1 to 58.

In another aspect, the present invention relates to: Item 64. An article of manufacture, comprising:
a container comprising a label; and

(i) the plasminogen for use in the method of any one of items 1 to 58 or a pharmaceutical composition comprising the plasminogen, wherein the label indicates the administration of the plasminogen or the composition to the subject to implement the method of any one of items 1 to 58.

Item 65. The kit of any one of items 61 to 63 or the article of manufacture of item 64, further comprising one or more additional means or containers containing other drugs.

Item 66. The kit or the article of manufacture of item 65, wherein the other drugs are selected from a group of: a hypolipidemic drug, an anti-platelet drug, an antihypertensive drug, a vasodilator, a hypoglycemic drug, an anticoagulant drug, a thrombolytic drug, a hepatoprotective drug, an anti-arrhythmia drug, a cardiotonic drug, a diuretic drug, an anti-infective drug, an antiviral drug, an immunomodulatory drug, an inflammatory regulatory drug, an antitumor drug, a hormone drug, and thyroxine.

The present invention further relates to the use of plasminogen for implementing the method of any one of items 1 to 58.

The present invention further relates to the use of plasminogen in the preparation of a medicament, a pharmaceutical composition, an article of manufacture, and a kit for the method of any one of items 1 to 58.

In some embodiments of the above-mentioned method, the plasminogen is administered by systemic or topical route, preferably by the following routes: intravenous, intramuscular, and subcutaneous administration of plasminogen for treatment. In some embodiments of the above-mentioned method, the plasminogen is administered in combination with a suitable polypeptide carrier or stabilizer. In some embodiments of the above-mentioned method, the plasminogen is administered at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 $mg/cm^2$, 0.001-800 $mg/cm^2$, 0.01-600 $mg/cm^2$, 0.1-400 $mg/cm^2$, 1-200 $mg/cm^2$, 1-100 $mg/cm^2$ or 10-100 $mg/cm^2$ (by per square centimeter of body surface area) daily, preferably the dosage is repeated at least once, preferably the dosage is administered at least daily.

The present invention explicitly encompasses all the combinations of technical features belonging to the embodiments of the present invention, and these combined technical solutions have been explicitly disclosed in the present application, as if the above-mentioned technical solutions were individually and explicitly disclosed. In addition, the present invention also explicitly encompasses all the combinations between various embodiments and elements thereof, and the combined technical solutions are explicitly disclosed herein.

## Detailed Description of Embodiments

"Nephropathy" is renal structural changes and dysfunction caused by various causes.

"Plasmin" is a very important enzyme that exists in the blood and can hydrolyze fibrin clots into fibrin degradation products and D-dimers.

"Plasminogen" is the zymogenic form of plasmin, and based on the sequence in the swiss prot and calculated from the amino acid sequence (SEQ ID No.4) of the natural human plasminogen containing a signal peptide, is a glycoprotein composed of 810 amino acids, which has a molecular weight of about 92 kD and is synthesized mainly in the liver and capable of circulating in the blood; and the cDNA sequence encoding this amino acid sequence is as shown in SEQ ID No.3. Full-length plasminogen contains seven domains: a C-terminal serine protease domain, an N-terminal Pan Apple (PAp) domain and five Kringle domains (Kringles 1-5). Referring to the sequence in the swiss prot, the signal peptide comprises residues Met1-Gly19, PAp comprises residues Glu20-Val98, Kringle 1 comprises residues Cys103-Cys181, Kringle 2 comprises residues Glu184-Cys262, Kringle 3 comprises residues Cys275-Cys352, Kringle 4 comprises residues Cys377-Cys454, and Kringle 5 comprises residues Cys481-Cys560. According to the NCBI data, the serine protease domain comprises residues Val581-Arg804.

Glu-plasminogen is a natural full-length plasminogen and is composed of 791 amino acids (without a signal peptide of 19 amino acids); the cDNA sequence encoding this sequence is as shown in SEQ ID No.1; and the amino acid sequence is as shown in SEQ ID No.2. *In vivo,* Lys-plasminogen, which is formed by hydrolysis of amino acids at positions 76-77 of Glu-plasminogen, is also present, as shown in SEQ ID No.6; and the cDNA sequence encoding this amino acid sequence is as shown in SEQ ID No.5. δ-plasminogen is a fragment of full-length plasminogen that lacks the structure of Kringle 2-Kringle 5 and contains only Kringle 1 and the serine protease domain[22,23]. The amino acid sequence (SEQ ID No. 8) of δ-plasminogen has been reported in the literature[23], and the cDNA sequence encoding this amino acid sequence is as shown in SEQ ID No. 7. Mini-plasminogen is composed of Kringle 5 and the serine protease domain, and has been reported in the literature to comprise residues Val443-Asn791 (with the Glu residue of the Glu-plasminogen sequence that does not contain a signal peptide as the starting amino acid)[24]; the amino acid sequence is as shown in SEQ ID No. 10; and the cDNA sequence encoding this amino acid sequence is as shown in SEQ ID No.9. Micro-plasminogen comprises only the serine protease domain, the amino acid sequence of which has been reported in the literature to comprise residues Ala543-Asn791 (with the Glu residue of the Glu-plasminogen sequence that does not contain a signal peptide as the starting amino acid)[25], and the sequence of which has been also reported in patent document CN 102154253 A to comprise residues Lys531-Asn791 (with the Glu residue of the Glu-plasminogen sequence that does not contain a signal peptide as the starting amino acid) (the sequence in this patent application refers to the patent document CN 102154253 A); the amino acid sequence is as shown in SEQ ID No. 12; and the cDNA sequence encoding this amino acid sequence is as shown in SEQ ID No.11.

In the present invention, "plasmin" is used interchangeably with "fibrinolysin" and "fibrinoclase", and the terms have the same meaning; and "plasminogen" is used interchangeably with "plasminogen" and "fibrinoclase zymogen", and the terms have the same meaning.

In the present application, the meaning of "lack" in plasminogen is that the content or activity of plasminogen in the body of a subject is lower than that of a normal person, which is low enough to affect the normal physiological function of the subject; and the meaning of "deficiency" in plasminogen is that the content or activity of plasminogen in the body of a subject is significantly lower than that of a normal person, or even the activity or expression is extremely small, and only through exogenous supply can the normal physiological function be maintained.

Those skilled in the art can understand that all the technical solutions of the plasminogen of the present invention are suitable for plasmin. Therefore, the technical solutions described in the present invention cover plasminogen and plasmin.

In the course of circulation, plasminogen is in a closed, inactive conformation, but when bound to thrombi or cell surfaces, it is converted into an active plasmin in an open conformation under the mediation of a plasminogen activator (PA). The active plasmin can further hydrolyze the fibrin clots to fibrin degradation products and D-dimers, thereby dissolving the thrombi. The PAp domain of plasminogen comprises an important determinant that maintains plasminogen in an inactive, closed conformation, and the KR domain is capable of binding to lysine residues present on receptors and substrates. A variety of enzymes that can serve as plasminogen activators are known, including: tissue plasminogen activator (tPA), urokinase plasminogen activator (uPA), kallikrein, coagulation factor XII (Hagmann factor), and the like.

"Plasminogen active fragment" refers to an active fragment in the plasminogen protein that is capable of binding to a target sequence in a substrate and exerting the proteolytic function. The technical solutions of the present invention involving plasminogen encompass technical solutions in which plasminogen is replaced with a plasminogen active fragment. The plasminogen active fragment of the present invention is a protein comprising a serine protease domain of plasminogen. Preferably, the plasminogen active fragment of the present invention comprises SEQ ID No. 14, or an amino acid sequence having an amino acid sequence identity of at least 80%, 90%, 95%, 96%, 97%, 98% or 99% with SEQ ID No. 14. Therefore, plasminogen of the present invention comprises a protein containing the plasminogen active fragment and still having the plasminogen activity.

At present, methods for determining plasminogen and its activity in blood include: detection of tissue plasminogen activator activity (t-PAA), detection of tissue plasminogen activator antigen (t-PAAg) in plasma, detection of tissue plasminogen activity (plgA) in plasma, detection of tissue plasminogen antigen (plgAg) in plasma, detection of activity of the inhibitor of tissue plasminogen activators in plasma, detection of inhibitor antigens of tissue plasminogen activators in plasma and detection of plasmin-anti-plasmin (PAP) complex in plasma. The most commonly used detection method is the chromogenic substrate method: streptokinase (SK) and a chromogenic substrate are added to a test plasma, the plasminogen in the test plasma is converted into plasmin by the action of SK, the plasmin acts on the chromogenic substrate, and then it is determined that the increase in absorbance is directly proportional to plasminogen activity using a spectrophotometer. In addition, plasminogen activity in blood can also be determined by immunochemistry, gel electrophoresis, immunonephelometry, radioimmuno-diffusion and the like.

"Orthologues or orthologs" refer to homologs between different species, including both protein homologs and DNA homologs, and are also known as orthologous homologs and vertical homologs. The term specifically refers to proteins or genes that have evolved from the same ancestral gene in different species. The plasminogen of the present invention includes human natural plasminogen, and also includes orthologues or orthologs of plasminogens derived from different species and having plasminogen activity.

"Conservatively substituted variant" refers to one in which a given amino acid residue is changed without altering the overall conformation and function of the protein or enzyme, including, but not limited to, replacing an amino acid in the amino acid sequence of the parent protein by an amino acid with similar properties (such as acidity, alkalinity and hydrophobicity). Amino acids with similar properties are well known. For example, arginine, histidine and lysine are hydrophilic basic amino acids and are interchangeable. Similarly, isoleucine is a hydrophobic amino acid that can be replaced by leucine, methionine or valine. Therefore, the similarity of two proteins or amino acid sequences with similar functions may be different. For example, the similarity (identity) is 70%-99% based on the MEGALIGN algorithm. "Conservatively substituted variant" also includes a polypeptide or enzyme having amino acid identity of 60% or more, preferably 75% or more, more preferably 85% or more, even more preferably 90% or more as determined by the BLAST or FASTA algorithm, and having the same or substantially similar properties or functions as the natural or parent protein or enzyme.

"Isolated" plasminogen refers to the plasminogen protein that is isolated and/or recovered from its natural environment. In some embodiments, the plasminogen will be purified (1) to a purity of greater than 90%, greater than 95% or greater than 98% (by weight), as determined by the Lowry method, such as more than 99% (by weight); (2) to a degree sufficiently to obtain at least 15 residues of the N-terminal or internal amino acid sequence using a spinning cup sequenator; or (3) to homogeneity, which is determined by sodium dodecyl sulfate-polyacrylamide gel electrophoresis (SDS-PAGE) under reducing or non-reducing conditions using Coomassie blue or silver staining. Isolated plasminogen also includes plasminogen prepared from recombinant cells by bioengineering techniques and separated by at least one purification step.

The terms "polypeptide", "peptide" and "protein" are used interchangeably herein and refer to polymeric forms of amino acids of any length, which may include genetically encoded and non-genetically encoded amino acids, chemically or biochemically modified or derivatized amino acids, and polypeptides having modified peptide backbones. The term includes fusion proteins, including, but not limited to, fusion proteins having heterologous amino acid sequences, fusions having heterologous and homologous leader sequences (with or without N-terminal methionine residues); and the like.

The "percent amino acid sequence identity (%)" with respect to the reference polypeptide sequence is defined as the percentage of amino acid residues in the candidate sequence identical to the amino acid residues in the reference polypeptide sequence when a gap is introduced as necessary to achieve maximal percent sequence identity and no conservative substitutions are considered as part of sequence identity. The comparison for purposes of determining percent amino acid sequence identity can be achieved in a variety of ways within the skill in the art, for example using publicly available computer softwares, such as BLAST, BLAST-2, ALIGN or Megalign (DNASTAR) software. Those skilled in the art can determine appropriate parameters for aligning sequences, including any algorithm needed to achieve the maximum comparison over the full length of the sequences being compared. However, for purposes of the present invention, the percent amino acid sequence identity value is generated using the sequence comparison computer program ALIGN-2.

In the case of comparing amino acid sequences using ALIGN-2, the % amino acid sequence identity of a given amino acid sequence A relative to a given amino acid sequence B (or may be expressed as a given amino acid sequence A having or containing a certain % amino acid sequence identity relative to, with or for a given amino acid sequence B) is calculated as follows:

$$\text{fraction } X/Y \times 100$$

wherein X is the number of identically matched amino acid residues scored by the sequence alignment program ALIGN-2 in the alignment of A and B using the program, and wherein Y is the total number of amino acid residues in B. It will be appreciated that where the length of amino acid sequence A is not equal to the length of amino acid sequence B, the % amino acid sequence identity of A relative to B will not be equal to the % amino acid sequence identity of B relative to A. Unless specifically stated otherwise, all the % amino acid sequence identity values used herein are obtained using the ALIGN-2 computer program as described in the previous paragraph.

As used herein, the terms "treatment" and "treating" refer to obtaining a desired pharmacological and/or physiologic effect. The effect may be complete or partial prevention of a disease or its symptoms and/or partial or complete cure of the disease and/or its symptoms, and includes: (a) prevention of the disease from developing in a subject that may have a predisposition to the disease but has not been diagnosed as having the disease; (b) suppression of the disease, i.e., blocking its formation; and (c) alleviation of the disease and/or its symptoms, i.e., eliminating the disease and/or its symptoms.

The terms "individual", "subject" and "patient" are used interchangeably herein and refer to mammals, including, but not limited to, murine (rats and mice), non-human primates, humans, dogs, cats , hoofed animals (e.g., horses, cattle, sheep, pigs, goats) and so on.

"Therapeutically effective amount" or "effective amount" refers to an amount of plasminogen sufficient to achieve the prevention and/or treatment of a disease when administered to a mammal or another subject to treat the disease. The "therapeutically effective amount" will vary depending on the plasminogen used, the severity of the disease and/or its symptoms, as well as the age, body weight of the subject to be treated, and the like.

## 2. Preparation of the plasminogen of the present invention

Plasminogen can be isolated and purified from nature for further therapeutic uses, and can also be synthesized by standard chemical peptide synthesis techniques. When chemically synthesized, a polypeptide can be subjected to liquid or solid phase synthesis. Solid phase polypeptide synthesis (SPPS) is a method suitable for chemical synthesis of plasminogen, in which the C-terminal amino acid of a sequence is attached to an insoluble support, followed by the sequential addition of the remaining amino acids in the sequence. Various forms of SPPS, such as Fmoc and Boc, can be used to synthesize plasminogen. Techniques for solid phase synthesis are described in Barany and Solid-Phase Peptide Synthesis; pp. 3-284 in The Peptides: Analysis, Synthesis, Biology. Vol. 2: Special Methods in Peptide Synthesis, Part A., Merrifield, et al. J. Am. Chem. Soc., 85: 2149-2156 (1963); Stewart et al. Solid Phase Peptide Synthesis, 2nd ed. Pierce Chem. Co., Rockford, Ill. (1984); and Ganesan A. 2006 Mini Rev. Med Chem. 6:3-10 and Camarero JA et al. 2005 Protein Pept Lett. 12:723-8. Briefly, small insoluble porous beads are treated with a functional unit on which a peptide chain is constructed. After repeated cycles of coupling/deprotection, the attached solid phase free N-terminal amine is coupled to a single N-protected amino acid unit. This unit is then deprotected to expose a new N-terminal amine that can be attached to another amino acid. The peptide remains immobilized on the solid phase before it is cut off.

Standard recombinant methods can be used to produce the plasminogen of the present invention. For example, a nucleic acid encoding plasminogen is inserted into an expression vector, so that it is operably linked to a regulatory sequence in the expression vector. Expression regulatory sequence includes, but is not limited to, promoters (e.g., naturally associated or heterologous promoters), signal sequences, enhancer elements and transcription termination sequences. Expression regulation can be a eukaryotic promoter system in a vector that is capable of transforming or transfecting eukaryotic host cells (e.g., COS or CHO cells). Once the vector is incorporated into a suitable host, the host is maintained under conditions suitable for high-level expression of the nucleotide sequence and collection and purification of plasminogen.

A suitable expression vector is usually replicated in a host organism as an episome or as an integral part of the host chromosomal DNA. In general, an expression vector contains a selective marker (e.g., ampicillin resistance, hygromycin resistance, tetracycline resistance, kanamycin resistance or neomycin resistance) to facilitate detection of those exogenous cells transformed with a desired DNA sequence.

*Escherichia coli* is an example of prokaryotic host cells that can be used to clone a polynucleotide encoding the subject antibody. Other microbial hosts suitable for use include *Bacillus,* for example, *Bacillus subtilis* and other species of *Enterobacteriaceae* (such as *Salmonella* spp. and *Serratia* spp.), and various *Pseudomonas* spp. In these prokaryotic hosts, expression vectors can also be generated which will typically contain an expression control sequence (e.g., origin of replication) that is compatible with the host cell. In addition, there will be many well-known promoters, such as the lactose promoter system, the tryptophan (trp) promoter system, the beta-lactamase promoter system or the promoter system from phage lambda. Optionally in the case of manipulation of a gene sequence, a promoter will usually control expression, and has a ribosome binding site sequence and the like to initiate and complete transcription and translation.

Other microorganisms, such as yeast, can also be used for expression. *Saccharomyces* (e.g., *S. cerevisiae)* and *Pichia* are examples of suitable yeast host cells, in which a suitable vector has an expression control sequence (e.g., promoter), an origin of replication, a termination sequence and the like, as required. A typical promoter comprises 3-phosphoglycerate kinase and other glycolytic enzymes. Inducible yeast promoters specifically include promoters derived from alcohol dehydrogenase, isocytochrome C, and enzymes responsible for maltose and galactose utilization.

In addition to microorganisms, mammalian cells (e.g., mammalian cells cultured in cell culture *in vitro*) can also be used to express and generate the plasminogen of the present invention (e.g., a polynucleotide encoding a subject anti-Tau antibody). See Winnacker, From Genes to Clones, VCH Publishers, N.Y., N.Y. (1987). Suitable mammalian host cells include CHO cell lines, various Cos cell lines, HeLa cells, myeloma cell lines and transformed B cells or hybridomas. Expression vectors for these cells may comprise an expression control sequence, such as an origin of replication, promoter and enhancer (Queen et al. Immunol. Rev. 89:49 (1986)), as well as necessary processing information sites, such as a ribosome binding site, RNA splice site, polyadenylation site and transcription terminator sequence. Examples of suitable expression control sequences are promoters derived from white immunoglobulin gene, SV40, adenovirus, bovine papilloma virus, cytomegalovirus and the like. See Co et al. J. Immunol. 148:1149 (1992).

Once synthesized (chemically or recombinantly), the plasminogen of the present invention can be purified according to standard procedures in the art, including ammonium sulfate precipitation, affinity column, column chromatography, high performance liquid chromatography (HPLC), gel electrophoresis and the like. The plasminogen is substantially pure, e.g., at least about 80% to 85% pure, at least about 85% to 90% pure, at least about 90% to 95% pure, or 98% to 99% pure or purer, for example free of contaminants such as cell debris, macromolecules other than the subject antibody and the like.

## 3. Pharmaceutical formulations

A therapeutic formulation can be prepared by mixing plasminogen of a desired purity with an optional pharmaceutical carrier, excipient or stabilizer (Remington's Pharmaceutical Sciences, 16th edition, Osol, A. ed. (1980)) to form a lyophilized preparation or an aqueous solution. Acceptable carriers, excipients and stabilizers are non-toxic to the recipient at the dosages and concentrations employed, and include buffers, such as phosphates, citrates and other organic acids; antioxidants, including ascorbic acid and methionine; preservatives (e.g., octadecyl dimethyl benzyl ammonium chloride; hexane chloride diamine; benzalkonium chloride and benzethonium chloride; phenol, butanol or benzyl alcohol; alkyl p-hydroxybenzoates, such as methyl or propyl p-hydroxybenzoate; catechol; resorcinol; cyclohexanol; 3-pentanol; and m-cresol); low molecular weight polypeptides (less than about 10 residues); proteins, such as serum albumin, gelatin or immunoglobulins; hydrophilic polymers, such as polyvinylpyrrolidone; amino acids, such as glycine, glutamine, asparagine, histidine, arginine or lysine; monosaccharides, disaccharides and other carbohydrates, including glucose, mannose or dextrins; chelating agents, such as EDTA; sugars, such as sucrose, mannitol, fucose or sorbitol; salt-forming counterions, such as sodium; metal complexes (e.g., zinc-protein complexes); and/or non-ionic surfactants, such as TWEENTM, PLURONICSTM or polyethylene glycol (PEG). Preferred lyophilized anti-VEGF antibody formulations are described in WO 97/04801, which is incorporated herein by reference.

The formulations of the invention may also comprise one or more active compounds required for the particular disorder to be treated, preferably those that are complementary in activity and have no side effects with one another, for example anti-hypertensive drugs, anti-arrhythmic drugs, drugs for treating diabetes mellitus, and the like.

The plasminogen of the present invention may be encapsulated in microcapsules prepared by techniques such as coacervation or interfacial polymerization, for example, it may be incorporated in a colloid drug delivery system (e.g., liposomes, albumin microspheres, microemulsions, nanoparticles and nanocapsules), or incorporated in hydroxymethylcellulose or gel-microcapsules and poly-(methyl methacrylate) microcapsules in macroemulsions. These techniques are disclosed in Remington's Pharmaceutical Sciences, 16th edition, Osol, A. Ed. (1980).

The plasminogen of the present invention for *in vivo* administration must be sterile. This can be easily achieved by filtration through a sterile filtration membrane before or after freeze drying and reconstitution.

The plasminogen of the present invention can be prepared into a sustained-release preparation. Suitable examples of sustained-release preparations include solid hydrophobic polymer semi-permeable matrices having a shape and containing glycoproteins, such as films or microcapsules. Examples of sustained-release matrices include polyesters, hydrogels (e.g., poly(2-hydroxyethylmethacrylate)) (Langer et al. J. Biomed. Mater. Res., 15: 167-277 (1981); and Langer, Chem. Tech., 12:98-105 (1982)), or poly(vinyl alcohol), polylactides (US Patent 3773919, and EP 58, 481), copolymer of L-glutamic acid and □ ethyl-L-glutamic acid (Sidman et al. Biopolymers 22:547(1983)), nondegradable ethylene-vinyl acetate (Langer et al. *supra*), or degradable lactic acid-glycolic acid copolymers such as Lupron DepotTM (injectable microspheres composed of lactic acid-glycolic acid copolymer and leuprolide acetate), and poly D-(-)-3-hydroxybutyric acid. Polymers, such as ethylene-vinyl acetate and lactic acid-glycolic acid, are able to persistently release molecules for 100 days or longer, while some hydrogels release proteins for a shorter period of time. A rational strategy for protein stabilization can be designed based on relevant mechanisms. For example, if the aggregation mechanism is discovered to be formation of an intermolecular S-S bond through thio-disulfide interchange, stability is achieved by modifying sulfhydryl residues, lyophilizing from acidic solutions, controlling moisture content, using appropriate additives, and developing specific polymer matrix compositions.

## 4. Administration and dosage

The pharmaceutical composition of the present invention is administered in different ways, for example by intravenous, intraperitoneal, subcutaneous, intracranial, intrathecal, intraarterial (e.g., via carotid), intramuscular, intranasal, topical or intradermal administration or spinal cord or brain delivery. An aerosol preparation, such as a nasal spray preparation, comprises purified aqueous or other solutions of the active agent along with a preservative and isotonic agent. Such preparations are adjusted to a pH and isotonic state compatible with the nasal mucosa.

In some cases, the plasminogen pharmaceutical composition of the present invention may be modified or formulated in such a manner to provide its ability to cross the blood-brain barrier. Such plasminogen compositions is administered to an individual suffering from thrombosis and/or a thrombosis-related disease via a variety of enteral and parenteral routes of administration, including oral, intravenous and the like.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, and alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include liquid and nutrient supplements, electrolyte supplements and the like. Preservatives and other additives may also be present, for example, such as antimicrobial agents, antioxidants, chelating agents and inert gases.

In some embodiments, the plasminogen of the invention is formulated with an agent that promotes the plasminogen to cross the blood-brain barrier. In some cases, the plasminogen of the present invention is fused directly or via a linker to a carrier molecule, peptide or protein that promotes the fusion to cross the blood brain barrier. In some embodiments, the plasminogen of the present invention is fused to a polypeptide that binds to an endogenous blood-brain barrier (BBB) receptor. The polypeptide that is linked to plasminogen and binds to an endogenous BBB receptor promotes the fusion to cross the BBB. Suitable polypeptides that bind to endogenous BBB receptors include antibodies (e.g., monoclonal antibodies) or antigen-binding fragments thereof that specifically bind to endogenous BBB receptors. Suitable endogenous BBB receptors include, but are not limited to, insulin receptors. In some cases, antibodies are encapsulated in liposomes. See, for example, US Patent Publication No. 2009/0156498.

The medical staff will determine the dosage regimen based on various clinical factors. As is well known in the medical field, the dosage of any patient depends on a variety of factors, including the patient's size, body surface area, age, the specific compound to be administered, sex, frequency and route of administration, overall health and other drugs administered simultaneously. The dosage range of the pharmaceutical composition comprising plasminogen of the present invention may be, for example, about 0.0001 to 2000 mg/kg, or about 0.001 to 500 mg/kg (such as 0.02 mg/kg, 0.25 mg/kg, 0.5 mg/kg, 0.75 mg/kg, 10 mg/kg and 50 mg/kg) of the subject's body weight daily. For example, the dosage may be 1 mg/kg body weight or 50 mg/kg body weight, or in the range of 1 mg/kg-50 mg/kg, or at least 1 mg/kg. Dosages above or below this exemplary range are also contemplated, especially considering the above factors. The intermediate dosages in the above range are also included in the scope of the present invention. A subject may be administered with such dosages daily, every other day, weekly or based on any other schedule determined by empirical analysis. An exemplary dosage schedule includes 1-10 mg/kg for consecutive days. During administration of the drug of the present invention, the therapeutic effect and safety of thrombosis and a thrombosis-related disease are required to be assessed real-timely and regularly.

## 5. Treatment efficacy

One embodiment of the present invention relates to the judgment of treatment efficacy and treatment safety after treating a subject with plasminogen. Clinically, the methods for judging treatment efficacy include, but are not limited to, detection of the following indexes to assess renal function: serum creatinine level, creatinine clearance, 24-hour urinary protein excretion rate (UAER), glomerular filtration rate, urinary albumin/creatinine ratio, albumin secretion rate, renal biopsy, etc. For example, the glomerular filtration rate can indicate glomerular hyperfiltration and hyperperfusion, indicating the degree of relief of the early symptoms of diabetic nephropathy. The glomerular filtration rate is the volume of filtrate produced per minute by the kidneys and can be determined by a variety of methods, such as measurement of urinary clearance of filtration markers, such as glycans, iothalamates or iohexols. A more commonly used method can be estimating glomerular filtration rate by determining creatinine (a protein produced by muscle and released into the blood) clearance. The creatinine clearance (usually expressed in milliliters per minute) can be determined by comparing the level of creatinine collected in the urine with the level of creatinine in the blood over a given time (e.g., 12 or 24 hours). The typical creatinine clearance in adult males is approximately 97-137 ml/min, and that in adult females is approximately 88-128 ml/min. The creatinine clearance is directly proportional to urinary creatinine excretion and inversely proportional to serum creatinine concentration.

Creatinine clearance/glomerular filtration rate or urinary albumin excretion rate is usually used as the main efficacy assessment index. Furthermore, other secondary indexes can be added to assess the efficacy of the drug of the present invention on related complications, for example, detection of triglyceride, total cholesterol, low-density lipoprotein and the like is added to assess blood lipid changes; detection of systolic blood pressure and diastolic blood pressure before and after treatment is added to assess the degree of relief of hypertension; and so on.

**6. Articles of manufacture or kits**

One embodiment of the present invention relates to an article of manufacture or a kit comprising plasminogen of the present invention useful in the treatment of diabetic nephropathy. The article preferably includes a container, label or package insert. Suitable containers include bottles, vials, syringes and the like. The container can be made of various materials, such as glass or plastic. The container contains a composition that is effective to treat the disease or disorder of the present invention and has a sterile access (for example, the container may be an intravenous solution bag or vial containing a plug that can be pierced by a hypodermic injection needle). At least one active agent in the composition is plasminogen. The label on or attached to the container indicates that the composition is used to treat the diabetic nephropathy of the present invention and diabetic nephropathy-related diseases. The article may further comprise a second container containing a pharmaceutically acceptable buffer, such as phosphate buffered saline, Ringer's solution and glucose solution. It may further comprise other substances required from a commercial and user perspective, including other buffers, diluents, filters, needles and syringes. In addition, the article comprises a package insert with instructions for use, including, for example, instructions to a user of the composition to administer the plasminogen composition and other drugs to treat an accompanying disease to a patient.

**Brief Description of the Drawings**

**Figure 1** shows a representative image of HE staining of kidney after administration of plasminogen to purine-induced chronic renal injury model mice for 10 days. A represents the control group administered with vehicle PBS, B represents the group administered with plasminogen, and C and D represent the $PLG^{-/-}$ group. The results showed that kidneys of $PLG^{-/-}$mice were most heavily injured, in which a large number of pus casts (indicated by a thick arrow), a small number of purine crystals (indicated by a triangle), great atrophy areas of renal tubules and flattened epithelial cells were observed; compared with $PLG^{-/-}$mice, kidneys of mice in the control group administered with vehicle PBS exhibited less severe injuries, no obvious purine crystal was observed, and the pus casts were less, though glomerular atrophy and tubular necrosis were still severe; and compared with the PBS control group, mice in the group administered with plasminogen exhibited less atrophy areas of renal tubules and less severe dilatation of renal tubules, with no pus casts found. It indicates that plasminogen can alleviate the renal injury in chronic renal failure model mice.

**Figure 2** shows a representative image of Sirius red staining of kidney after administration of plasminogen to purine-induced chronic renal injury model mice for 10 days. A represents the control group administered with vehicle PBS, B represents the group administered with plasminogen, C represents the $PLG^{-/-}$ group, and D represents the quantitative analysis results. The results showed that the collagen deposition in the group administered with plasminogen and the control group administered with vehicle PBS was remarkably less than that in the $PLG^{-/-}$ group, and the statistical difference was significant (* indicates $P < 0.05$, and ** indicates $P < 0.01$). In addition, the collagen deposition in the group administered with plasminogen was remarkably less than that in the control group administered with vehicle PBS. It indicates that plasminogen plays a key role in the repair of renal fibrosis in a chronic renal failure model.

**Figure 3** shows a representative image of Bcl-2 immunohistochemical staining of kidney after administration of plasminogen to purine-induced chronic renal injury model mice for 10 days. A represents a blank control group, B represents a control group administered with vehicle PBS, and C represents a group administered with plasminogen. The results showed that in the group administered with plasminogen, the positive staining of kidneys was remarkably darker than that in the control group administered with vehicle PBS, and the expression level was similar to that in mice of the blank control group. It indicates that plasminogen can promote the expression of Bcl-2, an apoptosis inhibitory molecule, in the kidneys of chronic renal failure model mice, and thus can inhibit apoptosis in the renal tissues of mice.

**Figure 4** shows an image of IgM immunohistochemical staining of kidney after administration of plasminogen to purine-induced chronic renal injury model mice for 10 days. A represents a blank control group, B represents a control group administered with vehicle PBS, and C represents a group administered with plasminogen. The results showed that the renal IgM-positive staining of mice in the group administered with plasminogen was lighter than that in the control group administered with vehicle PBS, and the staining range in the former group was smaller than that in the control group, and the staining was closer to that of normal mice. It indicates that the renal injury has been significantly improved after the administration of plasminogen, indicating that plasminogen has a significant repair effect on the renal injury in mice with a chronic renal injury.

**Figure 5** shows a representative image of immunohistochemical staining of renal fibrin after administration of plasminogen to purine-induced chronic renal injury model mice for 4 days. A represents the control group administered with vehicle PBS, B represents the group administered with plasminogen, and C represents the $PLG^{-/-}$ group. The results showed that the renal fibrin-positive staining in the control group administered with vehicle PBS was darker than that of mice in the group administered with plasminogen, and the staining in the $PLG^{-/-}$ group was darker than that in the control group administered with vehicle PBS. It indicates that plasminogen can alleviate a renal injury.

**Figure 6** shows an observed result of IgM immunostaining of kidney after administration of plasminogen to cisplatin injury model mice for 7 days. A represents the control group administered with vehicle PBS, B represents the group administered with plasminogen, and C represents the quantitative analysis results. The results showed that the positive expression of IgM in the group administered with plasminogen was remarkably lower than that in the control group administered with vehicle PBS, and the statistical difference was significant (* indicates $P < 0.05$). It indicates that plasminogen can promote repair of a cisplatin-induced renal injury.

**Figure 7** shows a representative image of renal type IV collagen immunostaining after administration of plasminogen to cisplatin injury model mice for 7 days. A represents the control group administered with vehicle PBS, and B represents the group administered with plasminogen. The results showed that the positive expression of type IV collagen in the group administered with plasminogen was remarkably lower than that in the control group administered with vehicle PBS. It indicates that plasminogen can ameliorate cisplatin-induced renal fibrosis.

**Figure 8** shows a representative image of immunostaining of type IV collagen in the kidney after administration of plasminogen to 24- to 25-week-old diabetic mice for 31 days. A represents the control group administered with vehicle PBS, and B represents the group administered with plasminogen. The results showed that the positive staining of IV collagen in the group administered with plasminogen was remarkably lighter than that in the control group administered with vehicle PBS, indicating that plasminogen can ameliorate renal fibrosis in diabetic mice.

**Figure 9** shows a representative image of masson staining of the kidney after administration of plasminogen to 26-week-old diabetic mice for 35 days. A represents the control group administered with vehicle PBS, and B represents the group administered with plasminogen. The results showed that in the control group administered with vehicle PBS, renal interstitial fibrosis was mild, and the hyperplastic fibrosis was blue. In the group administered with plasminogen, renal interstitial fibrosis was remarkably reduced. It indicates that plasminogen can reduce renal interstitial fibrosis in diabetic mice.

**Figure 10** shows a representative image of Sirius red staining of kidney after administration of plasminogen to bleomycin-induced systemic sclerosis model mice for 21 days. A represents the control group administered with vehicle PBS, and B represents the group administered with plasminogen. The results showed that in the bleomycin-induced systemic sclerosis mouse model, the collagen fibrosis in the kidney in the control group administered with vehicle PBS was remarkably greater than that in the group administered with plasminogen. It indicates that plasminogen effectively lowers renal fibrosis in systemic sclerosis mice.

**Figure 11** shows detection results of serum urea nitrogen in mice with a purine-induced chronic renal injury. The results showed that the concentration of urea nitrogen in sera in the $PLG^{+/+}$ group was remarkably lower than that in the $PLG^{-/-}$ group, and the statistical difference was significant (* indicates $P < 0.05$). It indicates that plasminogen can significantly ameliorate the renal function of chronic renal failure model mice.

**Figure 12** shows detection results of serum creatinine concentration after administration of plasminogen to purine-induced chronic renal injury model mice for 4 days. The results showed that the concentration of creatinine in sera of mice in each of the control group administered with vehicle PBS and the group administered with plasminogen was remarkably lower than that in the $PLG^{-/-}$ group, and the statistical difference was significant (* indicates P < 0.05). In addition, the concentration of creatinine in sera in the group administered with plasminogen was remarkably lower than that in the control group administered with vehicle PBS. It indicates that plasminogen can significantly ameliorate the renal function of chronic renal injury model mice.

**Figure 13** shows detection results of serum urea nitrogen in mice with a folate-induced acute renal injury. The results showed that the concentration of urea nitrogen in sera in the group administered with plasminogen was remarkably lower than that in the control group administered with vehicle PBS, and the statistical difference was nearly significant ($P = 0.06$). It indicates that plasminogen can significantly ameliorate the renal function of acute renal injury model mice.

**Figure 14** shows observed results of oil red O staining of the kidney after administration of plasminogen to 3% cholesterol hyperlipemia model mice for 30 days. A represents the blank control group, B represents the control group administered with vehicle PBS, C represents the group administered with plasminogen, and D represents the quantitative analysis results. The results showed that the fat deposition in kidney (indicated by arrow) of mice in the group administered with plasminogen was remarkably less than that in the control group administered with vehicle PBS, and the quantitative analysis showed significant statistical difference; in addition, the lipid deposition level in the group administered with plasminogen was similar to that in mice in the blank control group. It indicates that plasminogen can reduce the fat deposition in kidney of hyperlipemia model mice, and thus reduce renal injury caused by fat deposition.

**Figure 15** shows a representative image of HE staining of the kidney after administration of plasminogen to folate-induced acute renal injury model mice for 7 days. A represents a blank control group, B represents a control group administered with vehicle PBS, and C represents a group administered with plasminogen. The results showed that in the blank control group, the renal cell nuclei were round or oval, the cytoplasm was red-stained, and the glomeruli and tubules were normal in morphology; in the control group administered with vehicle PBS, a large number of flattened epithelial cells (indicated by a thick arrow), shed brush borders, and condensation of some cell nuclei in renal tubules were observed in the kidneys, cytoplasm was stained lightly only in some renal tubules, and pus casts (indicated by a thin arrow) were also observed in some renal tubules, accompanied by mild inflammatory cell infiltration in glomeruli and renal interstitium; and compared with the control group administered with vehicle PBS, dilatation of renal tubules and flattening of epithelial cells were remarkably improved in the group administered with plasminogen, in which most of the renal tubular cytoplasm was red-stained, with no pus casts. It indicates that plasmin can ameliorate a folate-induced acute renal injury.

**Figure 16** shows observed Bcl-2 immunohistochemical results of the kidney after administration of plasminogen to folate-induced acute renal injury model mice for 7 days. A represents the control group administered with vehicle PBS, B represents the group administered with plasminogen, and C represents the quantitative analysis results. The results showed that the renal Bcl-2-positive staining in the group administered with plasminogen was remarkably greater than that in the control group administered with vehicle PBS, and the statistical difference was significant (* indicates $P < 0.05$). It indicates that plasminogen can promote expression of Bcl-2, an apoptosis inhibitory protein, in the kidneys of acute renal injury model mice, and thus protect the renal tissue cells of mice with an acute renal injury from apoptosis.

**Figure 17** shows observed IgM immunohistochemical results of the kidney after administration of plasminogen to folate-induced acute renal injury model mice for 7 days. A represents the control group administered with vehicle PBS, and B represents the group administered with plasminogen. The results showed that the renal IgM-positive staining of mice in the group administered with plasminogen was lighter than that in the control group administered with vehicle PBS, and the staining range in the former group was smaller than that in the control group. It indicates that the expression of renal IgM has been significantly decreased after injection of plasminogen, reflecting that plasminogen can effectively reduce the renal injury in mice with a folate-induced acute renal injury.

**Figure 18** shows observed results of Sirius red staining of kidney after administration of plasminogen to 3% cholesterol hyperlipemia model mice for 30 days. A represents the blank control group, B represents the control group administered with vehicle PBS, C represents the group administered with plasminogen, and D represents the quantitative analysis results. The results showed that the collagen deposition in kidney (indicated by arrow) in the group administered with plasminogen was remarkably less than that in the control group administered with vehicle PBS, and the statistical difference was significant; and in the group administered with plasminogen, fibrosis was substantially restored to a normal level. It indicates that plasminogen can effectively reduce renal fibrosis in 3% cholesterol hyperlipemia model mice.

**Figure 19** shows a representative image of HE staining of the kidney after administration of plasminogen to ischemic reperfusion-induced acute renal injury model mice for 7 days. A represents the sham operation group, B represents the control group administered with vehicle PBS, and C represents the group administered with plasminogen. The results showed that in the sham operation group, the glomerular capillaries were unobstructed, and the cytoplasm was red-stained in renal tubules that were normal in morphology; in the control group administered with vehicle PBS, mild inflammatory cell infiltration (indicated by a triangle) in glomeruli, extensive inflammatory cell infiltration in renal interstitium, pus casts (indicated by a thin arrow) in some renal tubules, condensation of a few cell nuclei in renal tubules, great areas of flattened epithelial cells (indicated by a thick arrow), and dilated renal tubules were observed; and compared with the control group administered with vehicle PBS, there were only a few flattened epithelial cells in the group administered with plasminogen, in which most of the renal tubules had returned to a normal tubular morphology, the cytoplasm was red-stained, and no obvious renal tubular atrophy had been found, with mild inflammatory cell infiltration in renal interstitium only, all of which were close to the morphologies in the sham operation group. It indicates that plasminogen can ameliorate the renal injury in ischemic reperfusion-induced acute renal injury model mice.

## Examples

### Example 1. Plasminogen protects the kidney in a chronic renal injury model

Twenty 8- to 9-week-old $PLG^{+/+}$ mice and six $PLG^{-/-}$ mice were taken. $PLG^{+/+}$ mice were randomly divided into two groups, 10 mice in each of the group administered with plasminogen and the control group administered with vehicle PBS. Mice in the group administered with plasminogen, the control group administered with vehicle PBS, and the $PLG^{-/-}$ group were fed with a 0.25% purine diet (Nantong TROPHIC) every day to establish the chronic renal injury model[26]. The day of model establishment was recorded as Day 1, and administration began at the same time. Mice in the group administered with plasminogen were administered with plasminogen at a dose of 1 mg/0.1 mL/mouse/day via the tail vein, and an equal volume of PBS was administered to mice in the control group administered with vehicle PBS in the same manner, both lasting for 10 consecutive days for model establishment. $PLG^{-/-}$ mice were not treated. The mice were sacrificed on Day 11. The kidneys were fixed in 4% paraformaldehyde for 24 hours. The fixed kidneys were paraffin-embedded after dehydration with alcohol gradient and permeabilization with xylene. The tissue sections were 3 $\mu$m thick. The sections were dewaxed and rehydrated, stained with hematoxylin and eosin (HE staining), differentiated with 1% hydrochloric acid in alcohol, and returned to blue with ammonia water. The sections were dehydrated with alcohol gradient, permeabilized with xylene, sealed with a neutral gum, and observed under an optical microscope at 200× (Figures 1A, B and C) and 400× (Figure ID).

The results showed that kidneys of $PLG^{-/-}$mice (Figures 1C and ID) were most heavily injured, in which a large number of pus casts (indicated by a arrow), a small number of purine crystals (indicated by a triangle), great atrophy areas of renal tubules and flattened epithelial cells were observed; compared with $PLG^{-/-}$mice, kidneys of mice in the control group administered with vehicle PBS (Figure 1A) exhibited less severe injuries, no obvious purine crystal was observed, and the pus casts were less, though glomerular atrophy and tubular necrosis were still very severe; and compared with the PBS control group, mice in the group administered with plasminogen (Figure 1B) exhibited less atrophy areas of renal tubules and less severe dilatation of renal tubules, with no pus casts found. It indicates that plasminogen can repair the renal injury in chronic renal injury model mice.

### Example 2. Plasminogen repairs renal fibrosis in a chronic renal injury model

Twelve 8- to 9-week-old male $PLG^{+/+}$ mice and six $PLG^{-/-}$ mice were taken. $PLG^{+/+}$ mice were randomly divided into two groups, 6 mice in each of the group administered with plasminogen and the control group administered with vehicle PBS. The modelling method for the chronic renal injury model was the same as described in Example 1. The day of model establishment was recorded as Day 1, and administration began at the same time. Mice in the group administered with plasminogen were administered with plasminogen at a dose of 1 mg/0.1 mL/mouse/day via the tail vein, and an equal volume of PBS was administered to mice in the control group administered with vehicle PBS in the same manner, both lasting for 10 consecutive days for model establishment. $PLG^{-/-}$ mice were not treated. The mice were sacrificed on Day 11. The kidneys were fixed in 4% paraformaldehyde for 24 hours. The fixed kidneys were paraffin-embedded after dehydration with alcohol gradient and permeabilization with xylene. The tissue sections was 3 $\mu$m thick. The sections were dewaxed and rehydrated and washed with water once. After stained with 0.1% Sirius red for 60 min, the sections were flushed with running water. After stained with hematoxylin for 1 min, the sections were flushed with running water, differentiated with 1% hydrochloric acid in alcohol and returned to blue with ammonia water, flushed with running water, dried and sealed. The sections were observed under an optical microscope at 200×.

Sirius red staining allows for long-lasting staining of collagen. As a special staining method for pathological sections, Sirius red staining can show the collagen tissue specifically.

The results showed that the collagen deposition in the group administered with plasminogen (Figure 2B) and the control group administered with vehicle PBS (Figure 2A) was remarkably less than that in the $PLG^{-/-}$ group (Figure 2C), and the statistical difference was significant (Figure 2D). In addition, the collagen deposition in the group administered with plasminogen was remarkably less than that in the control group administered with vehicle PBS. It indicates that plasminogen plays a key role in the repair of renal fibrosis in a chronic renal injury model.

**Example 3. Plasminogen promotes the expression of apoptosis inhibitory protein Bcl-2 in kidneys of mice having a chronic renal injury**

Eighteen 8- to 9-week-old male $PLG^{+/+}$ mice were randomly divided into three groups, 6 mice in each of the blank control group, the group administered with plasminogen, and the control group administered with vehicle PBS. The modelling method for the chronic renal injury model was the same as described in Example 1. The day of model establishment was recorded as Day 1, and administration began at the same time. The blank control group was fed with a normal maintenance diet. Mice in the group administered with plasminogen were administered with plasminogen at a dose of 1 mg/0.1 mL/mouse/day via the tail vein, and an equal volume of PBS was administered to mice in the control group administered with vehicle PBS in the same manner, both lasting for 10 consecutive days for model establishment. Mice in the blank control group received no treatment. The day of model establishment and administration was recorded as Day 1. The mice were sacrificed on Day 11. The kidneys were fixed in 4% paraformaldehyde for 24 hours. The fixed kidneys were paraffin-embedded after dehydration with alcohol gradient and permeabilization with xylene. The thickness of the tissue sections was 3 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Rabbit anti-mouse Bcl-2 antibody (Abcam) was added to the sections dropwise, incubated at 4°C overnight, and washed with 0.01 M PBS twice for 5 minutes each time. The sections were incubated with a secondary antibody, goat anti-rabbit IgG (HRP) antibody (Abcam), for 1 hour at room temperature and washed with 0.01 M PBS twice for 5 minutes each time. The sections were developed with a DAB kit (Vector laboratories, Inc., USA). After washing with water three times, the sections were counterstained with hematoxylin for 30 seconds and flushed with running water for 5 minutes. After dehydration with alcohol gradient, permeabilization with xylenehe, and sealing with a neutral gum, the sections were observed under an optical microscope at 200×.

Bcl-2 is an apoptosis inhibitory protein, and its expression will be down-regulated under the action of an apoptosis stimulating factor[27,28]. The Bcl-2 immunohistochemical results showed that the renal Bcl-2-positive staining of the group administered with plasminogen (Figure 3C) was significantly darker than that in the control group administered with vehicle PBS (Figure 3B) and was similar to the Bcl-2 positive staining degree in a blank control group (Figure 3A). It indicates that plasminogen can promote the expression of Bcl-2, an apoptosis inhibitory molecule, in the kidneys of chronic renal injury model mice, and thus facilitate protection of the renal tissue cells of mice with a chronic renal injury from apoptosis.

**Example 4. Plasminogen improves local injuries of kidneys of mice with a chronic renal injury**

Eighteen 8- to 9-week-old male $PLG^{+/+}$ mice were randomly divided into three groups, 6 mice in each of the blank control group, the group administered with plasminogen, and the control group administered with vehicle PBS. The modelling method for the chronic renal injury model was the same as described in Example 1. The day of model establishment was recorded as Day 1, and administration began at the same time. The blank control group was fed with a normal maintenance diet. Mice in the group administered with plasminogen were administered with plasminogen at a dose of 1 mg/0.1 mL/mouse/day via the tail vein, and an equal volume of PBS was administered to mice in the control group administered with vehicle PBS in the same manner, both lasting for 10 consecutive days for model establishment. The blank control group received no treatment. The mice were sacrificed on Day 11. The kidneys were fixed in 4% paraformaldehyde for 24 hours. The fixed kidneys were paraffin-embedded after dehydration with alcohol gradient and permeabilization with xylene. The thickness of the tissue sections was 3 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Goat anti-mouse IgM (HRP) antibody (Abcam) was added to the sections dropwise, incubated for 1 hour at room temperature and washed with PBS twice for 5 minutes each time. The sections were developed with a DAB kit (Vector laboratories, Inc., USA). After washed with water three times, the sections were counterstained with hematoxylin for 30 seconds and flushed with running water for 5 minutes. After dehydration with a gradient, permeabilization and sealing, the sections were observed under an optical microscope at 200×.

IgM antibodies play an important role during the clearance of apoptotic and necrotic cells, and the local level of IgM antibodies in damaged tissues and organs is positively correlated with the degree of injury[29,30]. Therefore, detection of local level of IgM antibodies in tissues and organs can reflect the extent of injury of the tissues and organs.

The results showed that the renal IgM-positive staining of mice in the group administered with plasminogen (Figure 4C) was lighter than that in the control group administered with vehicle PBS (Figure 4B), and the staining range in the former was smaller than that in the control group, and the staining was very close to that of mice in the blank control group (Figure 4A). It indicates that the glomerular injury has been significantly improved after the injection of plasminogen, indicating that plasminogen has a significant repair effect on the renal injury in mice with a chronic renal injury.

**Example 5. Plasminogen reduces the expression of renal fibrin in mice with a chronic renal injury**

Twelve 8- to 9-week-old male $PLG^{+/+}$ mice and six $PLG^{-/-}$ mice were taken. $PLG^{+/+}$ mice were randomly divided into two groups, 6 mice in each of the group administered with plasminogen and the control group administered with vehicle PBS. The modelling method for the chronic renal injury model was the same as described in Example 1. The day of model establishment was recorded as Day 1, and administration began at the same time. Both model establishment and administration lasted for a period of 4 days. The blank control group was fed with a normal maintenance diet. Mice in the group administered with plasminogen were administered with plasminogen at a dose of 1 mg/0.1 mL/mouse/day via the tail vein, and an equal volume of PBS was administered to mice in the control group administered with vehicle PBS in the same manner. $PLG^{-/-}$ mice received no treatment. The mice were sacrificed on Day 5. The kidneys were fixed in 4% paraformaldehyde for 24 hours. The fixed kidneys were paraffin-embedded after dehydration with alcohol gradient and permeabilization with xylene. The thickness of the tissue sections was 3 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Rabbit anti-mouse fibrin antibody (Abcam) was added to the sections dropwise, incubated at 4°C overnight, and washed with PBS twice for 5 minutes each time. The sections were incubated with a secondary antibody, goat anti-rabbit IgG (HRP) antibody (Abcam), for 1 hour at room temperature and washed with PBS twice for 5 minutes each time. The sections were developed with a DAB kit (Vector laboratories, Inc., USA). After washed with water three times, the sections were counterstained with hematoxylin for 30 seconds and flushed with running water for 5 minutes. After dehydration with a gradient, permeabilization and sealing, the sections were observed under an optical microscope at 200×.

Fibrinogen is the precursor of fibrin, and in the presence of tissue injury, as a stress response to the body's injury, fibrinogen is hydrolyzed into fibrin and deposited at the injury site[31,32]. Therefore, the local fibrin level at the injury site can be used as a sign of the degree of injury.

The results showed that the renal fibrin-positive staining in the control group administered with vehicle PBS (Figure 5A) was darker than that of mice in the group administered with plasminogen (Figure 5B), and the staining in the $PLG^{-/-}$ group (Figure 5C) was darker than that in the control group administered with vehicle PBS. It indicates that plasminogen can repair a renal tissue injury to some extent.

**Example 6. Plasminogen promotes repair of a renal injury caused by cisplatin**

Ten healthy 8- to 9-week-old male C57 mice were used and randomly divided into two groups, five in the control group administered with vehicle PBS and five in the group administered with plasminogen, respectively. After the grouping was completed, a chemotherapy-induced injury model was established by single intraperitoneal injection of cisplatin at 10 mg/Kg body weight[33]. After the model was established, mice in the group administered with plasminogen were administered with plasminogen at a dose of 1 mg/0.1 mL/mouse/day via the tail vein, and an equal volume of PBS was administered to mice in the control group administered with vehicle PBS in the same manner. The day when the experiment began was Day 0, and the mice were weighed and grouped. The mice were injected with cisplatin intraperitoneally from day 1 for model establishment. Plasminogen or vehicle PBS was administered to the mice within 3 hours after completion of model establishment, and the administration period was 7 days. Mice were sacrificed on Day 8, and kidneys were fixed in 10% neutral formalin fixative for 24-48 hours. The fixed kidney tissues were paraffin-embedded after dehydration with alcohol gradient and permeabilization with xylene. The thickness of the tissue sections was 4 μm. The sections were dewaxed and rehydrated and washed with water once. The sections were repaired with citric acid for 30 minutes, and gently rinsed with water after cooling at room temperature for 10 minutes. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Goat anti-mouse IgM (HRP) antibody (Abcam) was added to the sections dropwise, incubated for 1 hour at room temperature and washed with PBS twice for 5 minutes each time. The sections were developed with a DAB kit (Vector laboratories, Inc., USA). After washed with water three times, the sections were counterstained with hematoxylin for 30 seconds and flushed with running water for 5 minutes. After dehydration with a gradient, permeabilization and sealing, the sections were observed under an optical microscope at 200×.

IgM antibodies play an important role during the clearance of apoptotic and necrotic cells, and the local level of IgM antibodies at the injury site in tissues and organs are positively correlated with the degree of injury[29,30]. Therefore, detection of local level of IgM antibodies in tissues and organs can reflect the injury of the tissues and organs.

The results showed that the IgM-positive expression in the group administered with plasminogen (Figure 6B) was remarkably lower than that in the control group administered with vehicle PBS (Figure 6A), and the statistical difference was significant (Figure 6C). It indicates that plasminogen can promote repair of a renal injury.

### Example 7. Plasminogen alleviates renal fibrosis in cisplatin chemotherapeutic injury model mice

Ten healthy 8-9-week-old male C57 mice were used and randomly divided into two groups, five in the control group administered with vehicle PBS and five in the group administered with plasminogen, respectively. After the grouping was completed, a chemotherapy-induced injury model was established by single intraperitoneal injection of cisplatin at 10 mg/Kg body weight[33]. After the model was established, mice in the group administered with plasminogen were administered with plasminogen at a dose of 1 mg/0.1 mL/mouse/day via tail vein injection, and an equal volume of PBS was administered to mice in the control group administered with vehicle PBS via tail vein injection. The day when the experiment began was Day 0, and the mice were weighed and grouped. The mice were injected with cisplatin intraperitoneally from day 1 for model establishment. Plasminogen or vehicle PBS was administered to the mice within 3 hours after completion of model establishment, and the administration period was 7 days. The mice were sacrificed on Day 8. The kidneys were fixed in 4% paraformaldehyde fixative for 24 hours. The fixed kidney tissues were paraffin-embedded after dehydration with alcohol gradient and permeabilization with xylene. The thickness of the tissue sections was 4 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Rabbit anti-mouse IV collagen antibody (Abcam) was added to the sections dropwise, incubated at 4°C overnight, and washed with TBS twice for 5 minutes each time. The sections were incubated with a secondary antibody, goat anti-rabbit IgG (HRP) antibody (Abcam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds, returned to blue with running water for 5 minutes, and washed with TBS once. After dehydration with a gradient, permeabilization and sealing, the sections were observed under an optical microscope at 200×.

The results showed that the renal type IV collagen-positive expression in the control group administered with vehicle PBS (Figure 7A) was remarkably higher than that in the group administered with plasminogen (Figure 7B). It indicates that plasminogen can ameliorate renal fibrosis in cisplatin-induced injury model mice.

### Example 8. Plasminogen alleviates renal fibrosis in diabetic mice

Ten 24- to 25-week-old male db/db mice were randomly divided into two groups, five in the control group administered with vehicle PBS and five in the group administered with plasminogen, respectively. The mice were weighed and grouped on the day when the experiment began, i.e. Day 0. Plasminogen or PBS was administered from day 1 for 31 consecutive days. Mice in the group administered with plasminogen were injected with plasminogen at a dose of 2 mg/0.2 mL/mouse/day via the tail vein, and an equal volume of PBS was administered to mice in the control group administered with vehicle PBS via the tail vein. The mice were sacrificed after administration of plasminogen for 31 days. The kidney tissues were fixed in 4% paraformaldehyde fixative for 24 hours. The fixed kidney tissues were paraffin-embedded after dehydration with alcohol gradient and permeabilization with xylene. The thickness of the tissue sections was 4 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Rabbit polyclonal antibody (Abcam) against IV collagen was added to the sections dropwise, incubated at 4°C overnight, and washed with TBS twice for 5 minutes each time. The sections were incubated with a secondary antibody, goat anti-rabbit IgG (HRP) antibody (Abcam), for 1 hour at room temperature and washed with TBS twice. The sections were developed with a DAB kit (Vector laboratories, Inc., USA). After washed with water three times, the sections were counterstained with hematoxylin for 30 seconds and flushed with running water for 5 minutes. After gradient dehydration, permeabilization and sealing, the sections were observed under an optical microscope at 200×.

Diabetic nephropathy is a chronic complication of diabetes mellitus, and glomerular sclerosis and renal interstitial fibrosis are typical pathological changes [34]. The experimental results of the present invention showed that the positive staining of IV collagen in the group administered with plasminogen (Figure 8B) was remarkably lighter than that in the control group administered with vehicle PBS (Figure 8A), indicating that plasminogen can alleviate renal fibrosis in diabetic mice.

**Example 9. Plasminogen alleviates renal fibrosis in diabetic mice**

Ten 26-week-old male db/db mice were randomly divided into two groups, 5 mice in each of the control group administered with vehicle PBS and the group administered with plasminogen. The mice were weighed and grouped on the day when the experiment began, i.e. Day 0. Plasminogen or PBS was administered from day 1 for 35 consecutive days. Mice in the group administered with plasminogen were injected with plasminogen at a dose of 2 mg/0.2 mL/mouse/day via the tail vein, and an equal volume of PBS was administered to mice in the control group administered with vehicle PBS via the tail vein. The mice were sacrificed on Day 36. The kidney tissues were fixed in 4% paraformaldehyde fixative for 24 hours. The fixed kidney tissues were paraffin-embedded after dehydration with alcohol gradient and permeabilization with xylene. The thickness of the tissue sections was 4 μm. The sections were dewaxed and rehydrated and then put into a potassium dichromate solution overnight. The sections were stained with iron hematocylin for 3 to 5 minutes, and flushed with running water. The sections were differentiated with 1% hydrochloric acid in alcohol, treated with ammonia water for 1 second, and rinsed with water. The sections were stained in ponceau acid fuchsin fluid for 8 minutes, and rinsed rapidly in water. The sections were treated with 1% phosphomolybdic acid aqueous solution for about 2 minutes, and counterstained with aniline blue solution for 6 minutes. The sections were rinsed with 1% glacial acetic acid for about 1 minute. The sections were sealed after dehydration with absolute ethanol, and permeabilization with xylene, and were observed under an optical microscope at 200×.

Masson staining can reveal tissue fibrosis. The results showed that in the control group administered with vehicle PBS (Figure 9A), renal interstitial fibrosis was mild, and the hyperplastic fibrosis was blue. Compared with the control group administered with vehicle PBS, renal interstitial fibrosis was remarkably reduced in the group administered with plasminogen (Figure 9B). It indicates that plasminogen can reduce renal fibrosis in diabetic mice.

**Example 10. Plasminogen lowers renal fibrosis in systemic sclerosis mice**

Ten 12-week-old male C57 mice were weighed and then randomly divided into two groups, 5 mice in each of the control group administered with vehicle PBS and the group administered with plasminogen. All mice were injected with bleomycin subcutaneously at a dose of 0.1 mg/0.1 mL/mouse/day to induce systemic sclerosis[35], and plasminogen or PBS was administered on the same day and this day was recorded as Day 1. The administration lasted for 21 consecutive days. Mice in the group administered with plasminogen were injected with plasminogen at a dose of 1mg/0.1 mL/mouse/day via the tail vein, and an equal volume of PBS was administered to mice in the control group administered with vehicle PBS via the tail vein. The mice were sacrificed on Day 22. The kidneys were fixed in 4% paraformaldehyde fixative for 24 hours. The fixed kidneys were paraffin-embedded after dehydration with alcohol gradient and permeabilization with xylene. The tissue sections was 3 μm thick. The sections were dewaxed and rehydrated and washed with water once. After stained with 0.1% Sirius red in saturated picric acid for 30 min, the sections were flushed with running water for 2 min. After stained with hematoxylin for 1 min, the sections were flushed with running water, differentiated with 1% hydrochloric acid in alcohol, returned to blue with ammonia water, flushed with running water, dried and sealed with a neutral gum. The sections were observed under an optical microscope at 200×.

The results showed that in the bleomycin-induced systemic sclerosis mouse model, the collagen fibrosis in the kidney in the control group administered with vehicle PBS (Figure 10A) was remarkably greater than that in the group administered with plasminogen (Figure 10B). It indicates that plasminogen effectively lowers renal fibrosis in systemic sclerosis mice.

**Example 11. Plasminogen promotes the repair of renal function in chronic renal injury model mice**

Ten 8- to 9-week-old $PLG^{+/+}$ mice and six $PLG^{-/-}$ mice were taken. The modelling method for the chronic renal injury model was the same as described in Example 1. The day of model establishment was recorded as Day 1. The model establishment lasted for a period of 10 days. On Day 11, the blood was collected from removed eyeballs, and centrifuged to obtain a supernatant, which was detected for the concentration of urea nitrogen in the serum. The content of urea nitrogen was detected using a urea nitrogen detection kit (Nanjing Jiancheng Bioengineering Institute, Cat# C013-2) according to the method of the urea nitrogen detection kit.

The results showed that the concentration of urea nitrogen in sera in the $PLG^{+/+}$ group was remarkably lower than that in the $PLG^{-/-}$ group, and the statistical difference was significant (Figure 11). It indicates that plasminogen can significantly ameliorate the renal function of chronic renal injury model mice.

**Example 12. Plasminogen promotes the repair of renal function in chronic renal injury model mice**

Twenty 8- to 9-week-old $PLG^{+/+}$ mice and six $PLG^{-/-}$ mice were taken. $PLG^{+/+}$ mice were randomly divided into two groups, 10 mice in each of the group administered with plasminogen and the control group administered with vehicle PBS. The modelling method for the chronic renal injury model was the same as described in Example 1. The day of model establishment was recorded as Day 1, and administration began at the same time, for an administration period of 4 days. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein. $PLG^{-/-}$ mice received no treatment. On Day 5, the blood was collected from removed eyeballs, and centrifuged to obtain a supernatant, which was detected for the concentration of creatinine in the serum. The serum creatinine concentration was detected using a creatinine detection kit (Nanjing Jiancheng Bioengineering Institute, Cat# C011-2) according to the method of the detection kit.

The results showed that the concentration of creatinine in sera of mice in each of the control group administered with vehicle PBS and the group administered with plasminogen was remarkably lower than that in the $PLG^{-/-}$ group, and the statistical difference was significant. In addition, the concentration of creatinine in sera in the group administered with plasminogen was remarkably lower than that in the control group administered with vehicle PBS (Figure 12). It indicates that plasminogen can significantly ameliorate the renal function of chronic renal injury model mice.

**Example 13. Plasminogen promotes the repair of renal function in acute renal injury model mice**

Nine 7-week-old male C57 mice were randomly divided into two groups, 5 mice in the group administered with plasminogen, and 4 mice in the control group administered with vehicle PBS. All mice received a single intraperitoneal injection of a folate (sigma A7876) solution at 250 mg/kg body weight to induce an acute renal injury[36]. Folate was dissolved in 0.3 mol/L $NaHCO_3$. The day of model establishment was recorded as Day 1, and plasminogen or vehicle PBS was administered at the same time. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein, both lasting for 7 days. On Day 8, the blood was collected from removed eyeballs, and centrifuged to obtain a supernatant, which was detected for the concentration of urea nitrogen in the serum. The content of urea nitrogen was detected using a urea nitrogen detection kit (Nanjing Jiancheng Bioengineering Institute, Cat# C013-2) according to the method of the urea nitrogen detection kit.

The results showed that the concentration of urea nitrogen in sera in the group administered with plasminogen was remarkably lower than that in the control group administered with vehicle PBS, and the statistical difference was nearly significant ($P = 0.06$) (Figure 13). It indicates that plasminogen can significantly ameliorate the renal function of acute renal injury model mice.

**Example 14 Plasminogen lowers fat deposition in kidney of 3% cholesterol hyperlipemia model mice**

Sixteen 9-week-old male C57 mice were fed with a 3% cholesterol high-fat diet (Nantong TROPHIC) for 4 weeks to induce hyperlipemia[37, 38]. This model was designated as the 3% cholesterol hyperlipemia model. The model mice continued to be fed with the 3% cholesterol high-fat diet. Another five male C57 mice of the same week age were taken as the blank control group, and were fed with a normal maintenance diet during the experiment. 50 $\mu$L of blood was taken from each mouse three days before administration, and the total cholesterol was detected. The model mice were randomly divided into two groups based on the total cholesterol concentration and the body weight, i.e., the group administered with plasminogen, and the control group administered with vehicle PBS, 8 mice in each group. The first day of administration was recorded as Day 1. Mice in the group administered with plasminogen were injected with human plasminogen at a dose of 1 mg/0.1 mL/mouse/day via the tail vein, and an equal volume of PBS was administered to mice in the control group administered with vehicle PBS via the tail vein, both lasting for 30 days. The mice were sacrificed on Day 31. The kidneys were fixed in 4% paraformaldehyde for 24 to 48 hours, then sedimented in 15% and 30% sucrose at 4°C overnight, respectively, and embedded in OCT. The frozen sections were 8 $\mu$m thick, stained with oil red O for 15 min, differentiated with 75% ethanol for 5 s, followed by nuclear staining with hematoxylin for 30 s, and sealing with glycerine and gelatin. The sections were observed under an optical microscope at 400×.

Oil red O staining can show lipid deposition and reflect the extent of lipid deposition[37]. The results showed that the fat deposition in kidney (indicated by arrow) of mice in the group administered with plasminogen (Figure 14C) was remarkably less than that in the control group administered with vehicle PBS (Figure 14B), and the quantitative analysis showed significant statistical difference (Figure 14D); in addition, the lipid deposition level in the group administered with plasminogen was similar to that in mice in the blank control group (Figure 14A). It indicates that plasminogen can reduce the fat deposition in kidney of hyperlipemia model mice, and thus reduce renal injury caused by fat deposition.

**Example 15. Plasminogen ameliorates renal injuries in folate-induced acute renal injury model mice**

Fifteen 7-week-old male C57 mice were randomly divided into three groups, 3 mice in the blank control group, 7 mice in the group administered with plasminogen, and 5 mice in the control group administered with vehicle PBS. Mice in the group administered with plasminogen and the control group administered with vehicle PBS received a single intraperitoneal injection of a folate (sigma A7876) solution at 250 mg/kg body weight to induce the acute renal injury model [36]. Mice in the blank control group received a single intraperitoneal injection of $NaHCO_3$ solution of corresponding volume. Folate was dissolved in 0.3 mol/L $NaHCO_3$ solution. The day of model establishment was recorded as Day 1, and plasminogen or vehicle PBS was administered at the same time. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein, both lasting for 7 days. The mice were sacrificed on Day 8. The kidneys were fixed in 4% paraformaldehyde for 24 hours. The tissue sections were 3 μm thick. The sections were dewaxed and rehydrated, stained with hematoxylin and eosin (HE staining), differentiated with 1% hydrochloric acid in alcohol, and returned to blue with ammonia water. The sections were dehydrated with alcohol gradient, permeabilized with xylene, sealed with a neutral gum, and observed under an optical microscope at 200×.

The results showed that in the blank control group (Figure 15A), the renal cell nuclei were round or oval, the cytoplasm was red-stained, and the glomeruli and tubules were normal in morphology; in the kidney of the control group administered with vehicle PBS (Figure 15B), a large proportion of flattened epithelial cells (indicated by a thick arrow), shed brush borders, and condensation of some cell nuclei in renal tubules were observed in the kidneys, cytoplasm was stained lightly only in some renal tubules, and pus casts (indicated by a thin arrow) were also observed in some renal tubules, accompanied by mild inflammatory cell infiltration in glomeruli and renal interstitium; and compared with the control group administered with vehicle PBS, dilatation of renal tubules and flattening of epithelial cells were remarkably improved in the group administered with plasminogen (Figure 15C), in which most of the renal tubular cytoplasm was red-stained, with no pus casts. It indicates that plasmin can ameliorate a folate-induced acute renal injury.

**Example 16. Plasminogen promotes the expression of Bcl-2 in kidneys of folate-induced acute renal injury model mice**

Twelve 7-week-old male C57 mice were randomly divided into two groups, 7 mice in the group administered with plasminogen, and 5 mice in the control group administered with vehicle PBS. All mice received a single intraperitoneal injection of a folate (sigma A7876) solution at 250 mg/kg body weight to induce an acute renal injury [36]. Folate was dissolved in 0.3 mol/L $NaHCO_3$. The day of model establishment was recorded as Day 1, and plasminogen or vehicle PBS was administered at the same time. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein, both lasting for 7 days. The mice were sacrificed on Day 8. The kidneys were fixed in 4% paraformaldehyde for 24 hours. The fixed kidneys were paraffin-embedded after dehydration with alcohol gradient and permeabilization with xylene. The thickness of the tissue sections was 3 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Rabbit anti-mouse Bcl-2 antibody (Abcam) was added to the sections dropwise, incubated at 4°C overnight, and washed with 0.01 M PBS twice for 5 minutes each time. The sections were incubated with a secondary antibody, goat anti-rabbit IgG (HRP) antibody (Abcam), for 1 hour at room temperature and washed with 0.01 M PBS twice for 5 minutes each time. The sections were developed with a DAB kit (Vector laboratories, Inc., USA). After washing with water three times, the sections were counterstained with hematoxylin for 30 seconds and flushed with running water for 5 minutes. After dehydration with alcohol gradient, permeabilization with xylenehe, and sealing with a neutral gum, the sections were observed under an optical microscope at 200×.

The Bcl-2 immunohistochemical results showed that the renal Bcl-2-positive staining in the group administered with plasminogen (Figure 16B) was remarkably greater than that in the control group administered with vehicle PBS (Figure 16A), and the statistical difference was significant (Figure 16C). It indicates that plasminogen can promote expression of Bcl-2, an apoptosis inhibitory protein, in the kidneys of acute renal injury model mice, and thus facilitate protection of the renal tissue cells of mice with an acute renal injury from apoptosis.

**Example 17. Plasminogen reduces renal injuries in folate-induced acute renal injury model mice**

Twelve 7-week-old male C57 mice were randomly divided into two groups, 7 mice in the group administered with plasminogen, and 5 mice in the control group administered with vehicle PBS. All mice received a single intraperitoneal injection of a folate (sigma A7876) solution at 250 mg/kg body weight to induce an acute renal injury [36]. Folate was dissolved in 0.3 mol/L $NaHCO_3$ solution. The day of model establishment was recorded as Day 1, and plasminogen or vehicle PBS was administered at the same time. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein, both lasting for 7 days. The mice were sacrificed on Day 8. The kidneys were fixed in 4% paraformaldehyde for 24 hours. The fixed kidneys were paraffin-embedded after dehydration with alcohol gradient and permeabilization with xylene. The thickness of the tissue sections was 3 μm. The sections were dewaxed and rehydrated and washed with water once. The tissues were circled with a PAP pen, incubated with 3% hydrogen peroxide for 15 minutes, and washed with 0.01M PBS twice for 5 minutes each time. The sections were blocked with 5% normal goat serum (Vector laboratories, Inc., USA) for 30 minutes, and after the time was up, the goat serum liquid was discarded. Goat anti-mouse IgM (HRP) antibody (Abcam) was added to the sections dropwise, incubated for 1 hour at room temperature and washed with PBS twice for 5 minutes each time. The sections were developed with a DAB kit (Vector laboratories, Inc., USA). After washed with water three times, the sections were counterstained with hematoxylin for 30 seconds and flushed with running water for 5 minutes. After dehydration with a gradient, permeabilization and sealing, the sections were observed under an optical microscope at 200×.

The results showed that the renal IgM-positive staining of mice in the group administered with plasminogen (Figure 17B) was lighter than that in the control group administered with vehicle PBS (Figure 17A), and the staining range in the former group was smaller than that in the control group. It indicates that the expression of renal IgM has been significantly decreased after injection of plasminogen, reflecting that plasminogen can effectively reduce the renal injury in mice with a folate-induced acute renal injury.

**Example 18. Plasminogen lowers renal fibrosis in 3% cholesterol hyperlipemia model mice**

Sixteen 9-week-old male C57 mice were fed with a 3% cholesterol high-fat diet (Nantong TROPHIC) for 4 weeks to induce hyperlipemia[37, 38]. This model was designated as the 3% cholesterol hyperlipemia model. The model mice continued to be fed with the 3% cholesterol high-fat diet. Another five male C57 mice of the same week age were taken as the blank control group, and were fed with a normal maintenance diet during the experiment. 50 μL of blood was taken from each mouse three days before administration, and the total cholesterol was detected. The model mice were randomly divided into two groups based on the total cholesterol concentration and the body weight, i.e., the group administered with plasminogen, and the control group administered with vehicle PBS, 8 mice in each group. The first day of administration was recorded as Day 1. Mice in the group administered with plasminogen were injected with human plasminogen at a dose of 1 mg/0.1 mL/mouse/day via the tail vein, and an equal volume of PBS was administered to mice in the control group administered with vehicle PBS via the tail vein. The mice were administered for 30 days. After the mice were administered on day 30, the mice were sacrificed on Day 31. The kidneys were fixed in 4% paraformaldehyde for 24 to 48 hours. The fixed tissues were paraffin-embedded after dehydration with alcohol gradient and permeabilization with xylene. The sections was 3 μm thick. The sections were dewaxed and rehydrated and washed with water once. After stained with 0.1% Sirius red in saturated picric acid for 30 min, the sections were flushed with running water for 2 min. After stained with hematoxylin for 1 min, the sections were flushed with running water, differentiated with 1% hydrochloric acid in alcohol, returned to blue with ammonia water, flushed with running water, dried and sealed with a neutral gum. The sections were observed under an optical microscope at 200×.

The results showed that the collagen deposition in kidney (indicated by arrow) in the group administered with plasminogen (Figure 18C) was remarkably less than that in the control group administered with vehicle PBS (Figure 18B), and the statistical difference was significant (Figure 18D); while in the group administered with plasminogen, fibrosis was substantially restored to a normal level (Figure 18A). It indicates that plasminogen can effectively reduce renal fibrosis in 3% cholesterol hyperlipemia model mice.

**Example 19. Plasminogen reduces renal injuries in ischemic reperfusion-induced acute renal injury model mice**

Nine 7- to 9-week-old male $PLG^{+/+}$ mice were randomly divided into three groups, 3 mice in each of the sham operation group, the group administered with plasminogen, and the control group administered with vehicle PBS. All mice were anesthetized by intraperitoneal injection of pentobarbital sodium at 50 mg/kg body weight. Incisions were made in the abdomens of the mice in the group administered with plasminogen and the control group administered with vehicle PBS for the exposure of kidneys, bilateral arteries and veins were isolated and clamped by vascular clamps, then the kidney were moved back into the abdominal cavity, and the wound was closed. After the time was up, the kidneys were exposed again, the vascular clamps were removed, the renal situations were observed, and the wounds were sutured after confirmation of reperfusion. In the sham operation group, an incisions was made in the abdomen for the exposure of the kidney only without ischemic treatment, and the wounds were sutured after the time was up[39]. After the operation was completed, each mouse received an intraperitoneal injection of 1 mL of normal saline at 37°C. The body temperature were kept at 36.5°C to 38°C during the operation. The day of model establishment was recorded as Day 1, and plasminogen or vehicle PBS was administered at the same time. Mice in the group administered with plasminogen were administered with plasminogen at a dose of 1 mg/0.1 mL/mouse/day via the tail vein, and an equal volume of PBS was administered to mice in the control group administered with vehicle PBS via the tail vein, both lasting for 7 days. Sham-operated mice received no injection treatment The mice were sacrificed on Day 8. The kidneys were fixed in 4% paraformaldehyde for 24 hours. The tissue sections were 3 μm thick. The sections were dewaxed and rehydrated, stained with hematoxylin and eosin (HE staining), differentiated with 1% hydrochloric acid in alcohol, and returned to blue with ammonia water. The sections were dehydrated with alcohol gradient, permeabilized with xylene, sealed with a neutral gum, and observed under an optical microscope at 200×.

The results showed that in the sham operation group (Figure 19A), the glomerular capillaries were unobstructed, and the cytoplasm was red-stained in renal tubules that were normal in morphology; in the control group administered with vehicle PBS (Figure 19B), mild inflammatory cell infiltration (indicated by a triangle) in glomeruli, extensive inflammatory cell infiltration in renal interstitium, pus casts (indicated by a thin arrow) in some renal tubules, condensation of a few cell nuclei in renal tubules, great areas of flattened epithelial cells (indicated by a thick arrow), and dilated renal tubules were observed; and compared with the control group administered with vehicle PBS, there were only a few flattened epithelial cells in the group administered with plasminogen (Figure 19C), in which most of the renal tubules had returned to a normal tubular morphology, the cytoplasm was red-stained, and no obvious renal tubular atrophy had been found, with mild inflammatory cell infiltration in renal interstitium only, all of which were close to the morphologies in the sham operation group. It indicates that plasminogen can ameliorate the renal injury in ischemic reperfusion-induced acute renal injury model mice.

References:

[1] James L. Pirkle, MD1 and Barry I. Freedman, MD. Hypertension and chronic kidney disease: controversies in pathogenesis and treatment, Minerva UrolNefrol. 2013 March ; 65(1): 37-50.

[2]Nguyen DCA,Touyz RM. A new look at the renin-angiotensin system-focusing on the vascular system [J]. Peptides,2011,32 (10) : 2141-2150.

[3]Kimoto E, Shoji T. Shinohara K, et al. Regional arterialstiffness inpatient with type 2 diabetes and chronic kidney disease [J]. J Am Soc Nephrol, 2006, 17(8):2245-2252.

[4]Mogensen CE, DamsgaardEm, Frland A, et al, Microalbuminuria in non-insulin depedent diabetes [J]. Clin Nephrol. 1992, 38(1):28-39.

[5] Choudhury D, Ahmed Z. Drug-associated renal dysfunction and injury [J]. Nat Clin PractNephrol, 2006, 2: 80-91.

[6] Colares VS, Oliveira RB, Abdulkader RC. Nephrotoxicity of vancomycin in patients with normal serum creatinine [J]. Nephrol Dial Transplant, 2006, 21: 3608.

[7] Izzedine H, Launay-Vacher V, Deray G. Antiviral drug-induced nephrotoxicity [J]. Am J Kidney Dis, 2005, 45: 804-817.

[8] Uwai Y, Ida H, Tsuji Y, et al. Renal transport of adefovir, cidofovir, and tenofovir by SLC22A family members (hOAT1, hOAT3, and hOCT2) [J]. Pharm Res, 2007, 24: 811-815.

[9] Servais H, Ortiz A, Devuyst O, et al. Renal cell apoptosis induced by nephrotoxic drugs: cellular and molecular mechanisms and potential approaches to modulation [J]. Apoptosis, 2008, 13: 11-32.

[10] Taguchi T, Nazneen A, Abid MR, et al. Cisplatin-associated nephrotoxicity and pathological events [J]. ContribNephrol, 2005, 148: 107-121.

[11] Harirforoosh S, Jamali F. Renal adverse effects of nonsteroidal anti-inflammatory drugs [J]. Expert Opin Drug Saf, 2009, 8: 669-681.

[12] Lincoln T. Toxicology: danger in the diet [J]. Nature, 2007,448: 148.

[13] Guitard J, Kamar N, Mouzin M, et al. Sulfadiazine-related obstructive urinary tract lithiasis: an unusual cause of acute renal failure after kidney transplantation [J]. Clin Nephrol, 2005, 63: 405-407.

[14] Boucher BJ. Renal failure and rhabdomyolysis associated with sitagliptin and simvastatin use. But what about the amiodarone? [J]. Diabet Med, 2009, 26: 192-193.

[15] Hsu CY, McCulloch CE, Fan D, et al. Community-based incidence of acute renal failure[J]. Kidney Int, 2007, 72(2): 208-212.

[16] Liangos O, Wald R, O'Bell JW, et al. Epidemiology and outcomes of acute renal failure in hospitalized patients: a national survey[J]. Clin J Am Soc Nephrol, 2006, 1(1): 43-51.

[17] Hoste EA, Clermont G, Kersten A, et al. RIFLE criteria for acute kidney injury are associated with hospital mortality in critically ill patients: a cohort analysis[J]. Crit Care, 2006, 10(3): R73.

[18] Zhang L, Yang YY, Fu P. The development and problems of continuous renal replacement therapy[J]. Chin J Pract Inter Med, 2011, 31(4):301-304.

[19] RENAL Replacement Therapy Study Investigators, Bellomo R, Cass A, et al. Intensity of continuous renal-replacement therapy in critically ill patients[J]. N Engl J Med, 2009, 361(17): 1627-1638.

[20] VA/NIH Acute Renal Failure Trial Network, Palevsky PM, Zhang JH, et al. Intensity of renal support in critically ill patients with acute kidney injury[J]. N Engl J Med, 2008, 359(1): 7-20.

[21] Coca SG, Yusuf B, Shlipak MG, et al. Long-term risk of mortality and other adverse outcomes after acute kidney injury: a systematic review and meta-analysis[J]. Am J Kidney Dis, 2009, 53(6): 961-973.

[22]Marder V J, Novokhatny V. Direct fibrinolytic agents: biochemical attributes, preclinical foundation and clinical potential [J]. Journal of Thrombosis and Haemostasis, 2010, 8(3): 433-444.

[23] Hunt J A, Petteway Jr S R, Scuderi P, et al. Simplified recombinant plasmin: production and functional comparison of a novel thrombolytic molecule with plasma-derived plasmin [J]. ThrombHaemost, 2008, 100(3): 413-419.

[24]Sottrup-Jensen L, Claeys H, Zajdel M, et al. The primary structure of human plasminogen: Isolation of two lysine-binding fragments and one "mini"-plasminogen (MW, 38,000) by elastase-catalyzed-specific limited proteolysis [J]. Progress in chemical fibrinolysis and thrombolysis, 1978, 3: 191-209.

[25]Nagai N, Demarsin E, Van Hoef B, et al. Recombinant human microplasmin: production and potential therapeutic properties [J]. Journal of Thrombosis and Haemostasis, 2003, 1(2): 307-313.

[26] CristhianeFaveroAguiar,CristianeNaffah-de-Souza, Angela Castoldi et al. Administration of α-Galactosylceramide Improves Adenine-Induced Renal Injury. Mol Med. 2015 Jun 18;21:553-62.

[27]Moungjaroen J, Nimmannit U, Callery PS, Wang L, Azad N, Lipipun V, Chanvorachote P, Rojanasakul Y (2006). Reactive oxygen species mediate caspase activation and apoptosis induced by lipoic acid in human lung epithelial cancer cells through Bcl-2 downregulation. J Pharmacol Exp Ther 319, 1062-1069.

[28]Wang L, Chanvorachote P, Toledo D, Stehlik C, Mercer RR, Castranova V, Rojanasakul Y (2008). Peroxide is a key mediator of Bcl-2 down-regulation and apoptosis induction by cisplatinin human lung cancer cells. Mol Pharmacol 73, 119-127.

[29]Zhang M, Takahashi K, Alicot EM, Vorup-Jensen T, Kessler B, et al. (2006) Activation of the lectin pathway by natural IgM in a model of ischemia/ reperfusion injury. J Immunol 177: 4727-4734.

[30] Kim SJ, Gershov D, Ma X, Brot N, Elkon KB (2002) I-PLA2 Activation during Apoptosis Promotes the Exposure of Membrane LysophosphatidylcholineLeading to Binding by Natural Immunoglobulin M Antibodies and Complement Activation. The Journal of Experimental Medicine 196: 655-665.

[31]Dimitrios Davalos , Katerina Akassoglou. Fibrinogen as a key regulator of inflammation in disease. Seminars in Immunopathology,2012. 34(1):43-62.

[32]Valvi D, Mannino DM, Mullerova H, et al.Fibrinogen, chronic obstructive pulmonary disease (COPD) and outcomes in two United States cohorts. Int J Chron Obstruct Pulmon Dis 2012;7:173-82.

[33]Blanca Humanes,AlbertoLazaro,Sonia Camano et al. Cilastatin protects against cisplatin-induced nephrotoxicity without compromising its anticancer efficiency in rats. Kidney Int. 2012 Sep;82(6):652-63.

[34] Donnelly SM, Zhou XP, Hu ang JT et al. Prevention of early glomerulopathy with tolrestat in the streptozotocin-induced diabetic rat. Biochem Cell Biol. 1996;74(3):355-62.

[35] Yosuke Kanno, En Shu, Hiroyuki Kanoh and Mariko Seishima. The Antifibrotic Effect of a2AP Neutralization in Systemic Sclerosis Dermal Fibroblasts and Mouse Models of Systemic Sclerosis. Journal of Investigative Dermatology (2016) 136, 762e769

[36]SzczypkaMS, WestoverAJ, ClouthierSGetal. Rare incorporation of bone marrow-derived cells into kidney after folic acid-induced injury. Stem Cells. 2005;23(1):44-54.

[37] Dominika Nackiewicz, Paromita Dey, Barbara Szczerba et al. Inhibitor of differentiation 3, a transcription factor regulates hyperlipidemia associated kidney disease. Nephron Exp Nephrol. 2014 ; 126(3): 141-147.

[38] Ming Gul, Yu Zhang., Shengjie Fan et al. Extracts of RhizomaPolygonatiOdorati Prevent High-Fat Diet-Induced Metabolic Disorders in C57BL/6 Mice. PLoS ONE 8(11): e81724.

[39] Li Yang, Craig R. Brooks,Sheng Xiao et al. KIM-1-mediated phagocytosis reduces acute injuryto the kidney. J Clin Invest. 2015 Apr;125(4):1620-36.

ÐòÁÐ±í

<110> ÉîÛÚÈð½¡ÉúÃü¿ÆÑ§ÑÐ¾¿ÔºÓÐÏÞ¹«Ë¾

<120> Ò»ÖÖÔ¤·ÀºÍÖÎÁÆ²¡ÀíÐÔÉö×éÖ¯ËðÉËµÄ·½·¨

<130> F17W0389

<160> 14

<170> PatentIn version 3.5

<210> 1
<211> 2376
<212> DNA
<213> ²»º¬ÓÐÐÅºÅëÄµÄÌìÈ»ÏËÈÜÃ¸Ô(Glu-PLG£¬Glu-ÏËÎ¬µ°°×ÈÜÃ¸Ô)ºËËáÐòÁÐ

<400> 1
gagcctctgg atgactatgt gaatacccag ggggcttcac tgttcagtgt cactaagaag 60

cagctgggag caggaagtat agaagaatgt gcagcaaaat gtgaggagga cgaagaattc 120

acctgcaggg cattccaata tcacagtaaa gagcaacaat gtgtgataat ggctgaaaac 180

aggaagtcct ccataatcat taggatgaga gatgtagttt tatttgaaaa gaaagtgtat 240

ctctcagagt gcaagactgg gaatggaaag aactacagag ggacgatgtc caaaacaaaa 300

aatggcatca cctgtcaaaa atggagttcc acttctcccc acagacctag attctcacct 360

gctacacacc cctcagaggg actggaggag aactactgca ggaatccaga caacgatccg 420

caggggccct ggtgctatac tactgatcca gaaaagagat atgactactg cgacattctt 480

gagtgtgaag aggaatgtat gcattgcagt ggagaaaact atgacggcaa aatttccaag 540

accatgtctg gactggaatg ccaggcctgg gactctcaga gcccacacgc tcatggatac 600

attccttcca aatttccaaa caagaacctg aagaagaatt actgtcgtaa ccccgatagg 660

gagctgcggc cttggtgttt caccaccgac cccaacaagc gctgggaact ttgtgacatc 720

ccccgctgca caacacctcc accatcttct ggtcccacct accagtgtct gaagggaaca 780

ggtgaaaact atcgcgggaa tgtggctgtt accgtgtccg ggcacacctg tcagcactgg 840

agtgcacaga cccctcacac acataacagg acaccagaaa acttcccctg caaaaatttg 900

gatgaaaact actgccgcaa tcctgacgga aaaagggccc catggtgcca tacaaccaac 960

agccaagtgc ggtgggagta ctgtaagata ccgtcctgtg actcctcccc agtatccacg 1020

gaacaattgg ctcccacagc accacctgag ctaacccctg tggtccagga ctgctaccat 1080

ggtgatggac agagctaccg aggcacatcc tccaccacca ccacaggaaa gaagtgtcag 1140

tcttggtcat ctatgacacc acaccggcac cagaagaccc cagaaaacta cccaaatgct 1200

ggcctgacaa tgaactactg caggaatcca gatgccgata aaggcccctg gtgttttacc 1260

acagacccca gcgtcaggtg ggagtactgc aacctgaaaa aatgctcagg aacagaagcg 1320

agtgttgtag cacctccgcc tgttgtcctg cttccagatg tagagactcc ttccgaagaa 1380

gactgtatgt ttgggaatgg gaaaggatac cgaggcaaga gggcgaccac tgttactggg 1440

acgccatgcc aggactgggc tgcccaggag ccccatagac acagcatttt cactccagag 1500

acaaatccac gggcgggtct ggaaaaaaat tactgccgta accctgatgg tgatgtaggt 1560

ggtccctggt gctacacgac aaatccaaga aaactttacg actactgtga tgtccctcag 1620

tgtgcggccc cttcatttga ttgtgggaag cctcaagtgg agccgaagaa atgtcctgga 1680

agggttgtag gggggtgtgt ggcccaccca cattcctggc cctggcaagt cagtcttaga 1740

acaaggtttg gaatgcactt ctgtggaggc accttgatat ccccagagtg ggtgttgact 1800

gctgcccact gcttggagaa gtccccaagg ccttcatcct acaaggtcat cctgggtgca 1860

caccaagaag tgaatctcga accgcatgtt caggaaatag aagtgtctag gctgttcttg 1920

gagcccacac gaaaagatat tgccttgcta aagctaagca gtcctgccgt catcactgac 1980

aaagtaatcc cagcttgtct gccatcccca aattatgtgg tcgctgaccg gaccgaatgt 2040

ttcatcactg gctgggggaga aacccaaggt acttttggag ctggccttct caaggaagcc 2100

cagctccctg tgattgagaa taaagtgtgc aatcgctatg agtttctgaa tggaagagtc 2160

caatccaccg aactctgtgc tgggcatttg gccggaggca ctgacagttg ccagggtgac 2220

agtggaggtc ctctggtttg cttcgagaag gacaaataca ttttacaagg agtcacttct 2280

tggggtcttg gctgtgcacg ccccaataag cctggtgtct atgttcgtgt ttcaaggttt 2340

gttacttgga ttgagggagt gatgagaaat aattaa 2376

<210> 2
<211> 791
<212> PRT
<213> ²»º¬ÓÐÐÅºÅëÄµÄÌìÈ»ÏËÈÜÃ¸Ô-(Glu-PLG£¬Glu-ÏËÎ¬µ°°×ÈÜÃ¸Ô-)°±»ùËáÐòÁÐ

<400> 2

Glu Pro Leu Asp Asp Tyr Val Asn Thr Gln Gly Ala Ser Leu Phe Ser
1 5 10 15

Val Thr Lys Lys Gln Leu Gly Ala Gly Ser Ile Glu Glu Cys Ala Ala
20 25 30

Lys Cys Glu Glu Asp Glu Glu Phe Thr Cys Arg Ala Phe Gln Tyr His
35 40 45

Ser Lys Glu Gln Gln Cys Val Ile Met Ala Glu Asn Arg Lys Ser Ser
50 55 60

Ile Ile Ile Arg Met Arg Asp Val Val Leu Phe Glu Lys Lys Val Tyr
65 70 75 80

```
Leu Ser Glu Cys Lys Thr Gly Asn Gly Lys Asn Tyr Arg Gly Thr Met
                85                  90                  95

Ser Lys Thr Lys Asn Gly Ile Thr Cys Gln Lys Trp Ser Ser Thr Ser
            100                 105                 110

Pro His Arg Pro Arg Phe Ser Pro Ala Thr His Pro Ser Glu Gly Leu
        115                 120                 125

Glu Glu Asn Tyr Cys Arg Asn Pro Asp Asn Asp Pro Gln Gly Pro Trp
    130                 135                 140

Cys Tyr Thr Thr Asp Pro Glu Lys Arg Tyr Asp Tyr Cys Asp Ile Leu
145                 150                 155                 160

Glu Cys Glu Glu Glu Cys Met His Cys Ser Gly Glu Asn Tyr Asp Gly
                165                 170                 175

Lys Ile Ser Lys Thr Met Ser Gly Leu Glu Cys Gln Ala Trp Asp Ser
            180                 185                 190

Gln Ser Pro His Ala His Gly Tyr Ile Pro Ser Lys Phe Pro Asn Lys
        195                 200                 205

Asn Leu Lys Lys Asn Tyr Cys Arg Asn Pro Asp Arg Glu Leu Arg Pro
    210                 215                 220

Trp Cys Phe Thr Thr Asp Pro Asn Lys Arg Trp Glu Leu Cys Asp Ile
225                 230                 235                 240

Pro Arg Cys Thr Thr Pro Pro Pro Ser Ser Gly Pro Thr Tyr Gln Cys
                245                 250                 255

Leu Lys Gly Thr Gly Glu Asn Tyr Arg Gly Asn Val Ala Val Thr Val
            260                 265                 270

Ser Gly His Thr Cys Gln His Trp Ser Ala Gln Thr Pro His Thr His
        275                 280                 285

Asn Arg Thr Pro Glu Asn Phe Pro Cys Lys Asn Leu Asp Glu Asn Tyr
    290                 295                 300

Cys Arg Asn Pro Asp Gly Lys Arg Ala Pro Trp Cys His Thr Thr Asn
305                 310                 315                 320

Ser Gln Val Arg Trp Glu Tyr Cys Lys Ile Pro Ser Cys Asp Ser Ser
                325                 330                 335
```

```
Pro Val Ser Thr Glu Gln Leu Ala Pro Thr Ala Pro Pro Glu Leu Thr
            340                 345                 350

Pro Val Val Gln Asp Cys Tyr His Gly Asp Gly Gln Ser Tyr Arg Gly
        355                 360                 365

Thr Ser Ser Thr Thr Thr Thr Gly Lys Lys Cys Gln Ser Trp Ser Ser
    370                 375                 380

Met Thr Pro His Arg His Gln Lys Thr Pro Glu Asn Tyr Pro Asn Ala
385                 390                 395                 400

Gly Leu Thr Met Asn Tyr Cys Arg Asn Pro Asp Ala Asp Lys Gly Pro
                405                 410                 415

Trp Cys Phe Thr Thr Asp Pro Ser Val Arg Trp Glu Tyr Cys Asn Leu
            420                 425                 430

Lys Lys Cys Ser Gly Thr Glu Ala Ser Val Val Ala Pro Pro Pro Val
        435                 440                 445

Val Leu Leu Pro Asp Val Glu Thr Pro Ser Glu Glu Asp Cys Met Phe
    450                 455                 460

Gly Asn Gly Lys Gly Tyr Arg Gly Lys Arg Ala Thr Thr Val Thr Gly
465                 470                 475                 480

Thr Pro Cys Gln Asp Trp Ala Ala Gln Glu Pro His Arg His Ser Ile
                485                 490                 495

Phe Thr Pro Glu Thr Asn Pro Arg Ala Gly Leu Glu Lys Asn Tyr Cys
            500                 505                 510

Arg Asn Pro Asp Gly Asp Val Gly Gly Pro Trp Cys Tyr Thr Thr Asn
        515                 520                 525

Pro Arg Lys Leu Tyr Asp Tyr Cys Asp Val Pro Gln Cys Ala Ala Pro
    530                 535                 540

Ser Phe Asp Cys Gly Lys Pro Gln Val Glu Pro Lys Lys Cys Pro Gly
545                 550                 555                 560

Arg Val Val Gly Gly Cys Val Ala His Pro His Ser Trp Pro Trp Gln
                565                 570                 575

Val Ser Leu Arg Thr Arg Phe Gly Met His Phe Cys Gly Gly Thr Leu
            580                 585                 590
```

Ile Ser Pro Glu Trp Val Leu Thr Ala Ala His Cys Leu Glu Lys Ser
595 600 605

Pro Arg Pro Ser Ser Tyr Lys Val Ile Leu Gly Ala His Gln Glu Val
610 615 620

Asn Leu Glu Pro His Val Gln Glu Ile Glu Val Ser Arg Leu Phe Leu
625 630 635 640

Glu Pro Thr Arg Lys Asp Ile Ala Leu Leu Lys Leu Ser Ser Pro Ala
645 650 655

Val Ile Thr Asp Lys Val Ile Pro Ala Cys Leu Pro Ser Pro Asn Tyr
660 665 670

Val Val Ala Asp Arg Thr Glu Cys Phe Ile Thr Gly Trp Gly Glu Thr
675 680 685

Gln Gly Thr Phe Gly Ala Gly Leu Leu Lys Glu Ala Gln Leu Pro Val
690 695 700

Ile Glu Asn Lys Val Cys Asn Arg Tyr Glu Phe Leu Asn Gly Arg Val
705 710 715 720

Gln Ser Thr Glu Leu Cys Ala Gly His Leu Ala Gly Gly Thr Asp Ser
725 730 735

Cys Gln Gly Asp Ser Gly Gly Pro Leu Val Cys Phe Glu Lys Asp Lys
740 745 750

Tyr Ile Leu Gln Gly Val Thr Ser Trp Gly Leu Gly Cys Ala Arg Pro
755 760 765

Asn Lys Pro Gly Val Tyr Val Arg Val Ser Arg Phe Val Thr Trp Ile
770 775 780

Glu Gly Val Met Arg Asn Asn
785 790

<210> 3
<211> 2433
<212> DNA
<213> º¬ÓÐÐÅºÅëÄµÄÌìÈ»ÏËÈÜÃ¸Ô–£¨À´Ô´ÓÚswiss prot£©µÄºËËáÐòÁÐ

<400> 3
atggaacata aggaagtggt tcttctactt cttttatttc tgaaatcagg tcaaggagag 60

cctctggatg actatgtgaa tacccagggg gcttcactgt tcagtgtcac taagaagcag 120

ctgggagcag gaagtataga agaatgtgca gcaaaatgtg aggaggacga agaattcacc 180
tgcagggcat tccaatatca cagtaaagag caacaatgtg tgataatggc tgaaaacagg 240
aagtcctcca taatcattag gatgagagat gtagttttat ttgaaaagaa agtgtatctc 300
tcagagtgca agactgggaa tggaaagaac tacagaggga cgatgtccaa aacaaaaaat 360
ggcatcacct gtcaaaaatg gagttccact tctccccaca gacctagatt ctcacctgct 420
acacaccccct cagagggact ggaggagaac tactgcagga atccagacaa cgatccgcag 480
gggccctggt gctatactac tgatccagaa aagagatatg actactgcga cattcttgag 540
tgtgaagagg aatgtatgca ttgcagtgga gaaaactatg acggcaaaat ttccaagacc 600
atgtctggac tggaatgcca ggcctgggac tctcagagcc cacacgctca tggatacatt 660
ccttccaaat ttccaaacaa gaacctgaag aagaattact gtcgtaaccc cgatagggag 720
ctgcggcctt ggtgtttcac caccgacccc aacaagcgct gggaactttg tgacatcccc 780
cgctgcacaa cacctccacc atcttctggt cccacctacc agtgtctgaa gggaacaggt 840
gaaaactatc gcgggaatgt ggctgttacc gtgtccgggc acacctgtca gcactggagt 900
gcacagaccc ctcacacaca taacaggaca ccagaaaact tcccctgcaa aaatttggat 960
gaaaactact gccgcaatcc tgacggaaaa agggccccat ggtgccatac aaccaacagc 1020
caagtgcggt gggagtactg taagataccg tcctgtgact cctccccagt atccacggaa 1080
caattggctc ccacagcacc acctgagcta acccctgtgg tccaggactg ctaccatggt 1140
gatggacaga gctaccgagg cacatcctcc accaccacca caggaaagaa gtgtcagtct 1200
tggtcatcta tgacaccaca ccggcaccag aagaccccag aaaactaccc aaatgctggc 1260
ctgacaatga actactgcag gaatccagat gccgataaag gcccctggtg ttttaccaca 1320
gaccccagcg tcaggtggga gtactgcaac ctgaaaaaat gctcaggaac agaagcgagt 1380
gttgtagcac ctccgcctgt tgtcctgctt ccagatgtag agactccttc cgaagaagac 1440
tgtatgtttg ggaatgggaa aggataccga ggcaagaggg cgaccactgt tactgggacg 1500
ccatgccagg actgggctgc ccaggagccc catagacaca gcattttcac tccagagaca 1560
aatccacggg cgggtctgga aaaaaattac tgccgtaacc ctgatggtga tgtaggtggt 1620
ccctggtgct acacgacaaa tccaagaaaa ctttacgact actgtgatgt ccctcagtgt 1680
gcggcccctt catttgattg tgggaagcct caagtggagc cgaagaaatg tcctggaagg 1740
gttgtagggg ggtgtgtggc ccacccacat tcctggccct ggcaagtcag tcttagaaca 1800
aggtttggaa tgcacttctg tggaggcacc ttgatatccc cagagtgggt gttgactgct 1860
gcccactgct tggagaagtc cccaaggcct tcatcctaca aggtcatcct gggtgcacac 1920
caagaagtga atctcgaacc gcatgttcag gaaatagaag tgtctaggct gttcttggag 1980

cccacacgaa aagatattgc cttgctaaag ctaagcagtc ctgccgtcat cactgacaaa 2040

gtaatcccag cttgtctgcc atccccaaat tatgtggtcg ctgaccggac cgaatgtttc 2100

atcactggct ggggagaaac ccaaggtact tttggagctg gccttctcaa ggaagcccag 2160

ctccctgtga ttgagaataa agtgtgcaat cgctatgagt ttctgaatgg aagagtccaa 2220

tccaccgaac tctgtgctgg gcatttggcc ggaggcactg acagttgcca gggtgacagt 2280

ggaggtcctc tggtttgctt cgagaaggac aaatacattt tacaaggagt cacttcttgg 2340

ggtcttggct gtgcacgccc caataagcct ggtgtctatg ttcgtgtttc aaggtttgtt 2400

acttggattg agggagtgat gagaaataat taa 2433

<210> 4
<211> 810
<212> PRT
<213> º¬ÓÐÐÅºÅëÄµÄÌìÈ»ÏËÈÜÃ¸Ô–£¨À´Ô´ÓÚswiss prot£©µÄº±»ùËáÐòÁÐ

<400> 4

Met Glu His Lys Glu Val Val Leu Leu Leu Leu Leu Phe Leu Lys Ser
1 5 10 15

Gly Gln Gly Glu Pro Leu Asp Asp Tyr Val Asn Thr Gln Gly Ala Ser
20 25 30

Leu Phe Ser Val Thr Lys Lys Gln Leu Gly Ala Gly Ser Ile Glu Glu
35 40 45

Cys Ala Ala Lys Cys Glu Glu Asp Glu Glu Phe Thr Cys Arg Ala Phe
50 55 60

Gln Tyr His Ser Lys Glu Gln Gln Cys Val Ile Met Ala Glu Asn Arg
65 70 75 80

Lys Ser Ser Ile Ile Ile Arg Met Arg Asp Val Val Leu Phe Glu Lys
85 90 95

Lys Val Tyr Leu Ser Glu Cys Lys Thr Gly Asn Gly Lys Asn Tyr Arg
100 105 110

Gly Thr Met Ser Lys Thr Lys Asn Gly Ile Thr Cys Gln Lys Trp Ser
115 120 125

Ser Thr Ser Pro His Arg Pro Arg Phe Ser Pro Ala Thr His Pro Ser
130 135 140

Glu Gly Leu Glu Glu Asn Tyr Cys Arg Asn Pro Asp Asn Asp Pro Gln
145 150 155 160

```
Gly Pro Trp Cys Tyr Thr Thr Asp Pro Glu Lys Arg Tyr Asp Tyr Cys
                165                 170                 175

Asp Ile Leu Glu Cys Glu Glu Glu Cys Met His Cys Ser Gly Glu Asn
            180                 185                 190

Tyr Asp Gly Lys Ile Ser Lys Thr Met Ser Gly Leu Glu Cys Gln Ala
        195                 200                 205

Trp Asp Ser Gln Ser Pro His Ala His Gly Tyr Ile Pro Ser Lys Phe
    210                 215                 220

Pro Asn Lys Asn Leu Lys Lys Asn Tyr Cys Arg Asn Pro Asp Arg Glu
225                 230                 235                 240

Leu Arg Pro Trp Cys Phe Thr Thr Asp Pro Asn Lys Arg Trp Glu Leu
                245                 250                 255

Cys Asp Ile Pro Arg Cys Thr Thr Pro Pro Pro Ser Ser Gly Pro Thr
            260                 265                 270

Tyr Gln Cys Leu Lys Gly Thr Gly Glu Asn Tyr Arg Gly Asn Val Ala
        275                 280                 285

Val Thr Val Ser Gly His Thr Cys Gln His Trp Ser Ala Gln Thr Pro
    290                 295                 300

His Thr His Asn Arg Thr Pro Glu Asn Phe Pro Cys Lys Asn Leu Asp
305                 310                 315                 320

Glu Asn Tyr Cys Arg Asn Pro Asp Gly Lys Arg Ala Pro Trp Cys His
                325                 330                 335

Thr Thr Asn Ser Gln Val Arg Trp Glu Tyr Cys Lys Ile Pro Ser Cys
            340                 345                 350

Asp Ser Ser Pro Val Ser Thr Glu Gln Leu Ala Pro Thr Ala Pro Pro
        355                 360                 365

Glu Leu Thr Pro Val Val Gln Asp Cys Tyr His Gly Asp Gly Gln Ser
    370                 375                 380

Tyr Arg Gly Thr Ser Ser Thr Thr Thr Thr Gly Lys Lys Cys Gln Ser
385                 390                 395                 400

Trp Ser Ser Met Thr Pro His Arg His Gln Lys Thr Pro Glu Asn Tyr
                405                 410                 415
```

```
Pro Asn Ala Gly Leu Thr Met Asn Tyr Cys Arg Asn Pro Asp Ala Asp
            420                 425                 430

Lys Gly Pro Trp Cys Phe Thr Thr Asp Pro Ser Val Arg Trp Glu Tyr
        435                 440                 445

Cys Asn Leu Lys Lys Cys Ser Gly Thr Glu Ala Ser Val Val Ala Pro
    450                 455                 460

Pro Pro Val Val Leu Leu Pro Asp Val Glu Thr Pro Ser Glu Glu Asp
465                 470                 475                 480

Cys Met Phe Gly Asn Gly Lys Gly Tyr Arg Gly Lys Arg Ala Thr Thr
                485                 490                 495

Val Thr Gly Thr Pro Cys Gln Asp Trp Ala Ala Gln Glu Pro His Arg
            500                 505                 510

His Ser Ile Phe Thr Pro Glu Thr Asn Pro Arg Ala Gly Leu Glu Lys
        515                 520                 525

Asn Tyr Cys Arg Asn Pro Asp Gly Asp Val Gly Gly Pro Trp Cys Tyr
    530                 535                 540

Thr Thr Asn Pro Arg Lys Leu Tyr Asp Tyr Cys Asp Val Pro Gln Cys
545                 550                 555                 560

Ala Ala Pro Ser Phe Asp Cys Gly Lys Pro Gln Val Glu Pro Lys Lys
                565                 570                 575

Cys Pro Gly Arg Val Val Gly Gly Cys Val Ala His Pro His Ser Trp
            580                 585                 590

Pro Trp Gln Val Ser Leu Arg Thr Arg Phe Gly Met His Phe Cys Gly
        595                 600                 605

Gly Thr Leu Ile Ser Pro Glu Trp Val Leu Thr Ala Ala His Cys Leu
    610                 615                 620

Glu Lys Ser Pro Arg Pro Ser Ser Tyr Lys Val Ile Leu Gly Ala His
625                 630                 635                 640

Gln Glu Val Asn Leu Glu Pro His Val Gln Glu Ile Glu Val Ser Arg
                645                 650                 655

Leu Phe Leu Glu Pro Thr Arg Lys Asp Ile Ala Leu Leu Lys Leu Ser
```

```
            660                 665                 670

Ser Pro Ala Val Ile Thr Asp Lys Val Ile Pro Ala Cys Leu Pro Ser
        675                 680                 685

Pro Asn Tyr Val Val Ala Asp Arg Thr Glu Cys Phe Ile Thr Gly Trp
    690                 695                 700

Gly Glu Thr Gln Gly Thr Phe Gly Ala Gly Leu Leu Lys Glu Ala Gln
705                 710                 715                 720

Leu Pro Val Ile Glu Asn Lys Val Cys Asn Arg Tyr Glu Phe Leu Asn
                725                 730                 735

Gly Arg Val Gln Ser Thr Glu Leu Cys Ala Gly His Leu Ala Gly Gly
            740                 745                 750

Thr Asp Ser Cys Gln Gly Asp Ser Gly Gly Pro Leu Val Cys Phe Glu
        755                 760                 765

Lys Asp Lys Tyr Ile Leu Gln Gly Val Thr Ser Trp Gly Leu Gly Cys
    770                 775                 780

Ala Arg Pro Asn Lys Pro Gly Val Tyr Val Arg Val Ser Arg Phe Val
785                 790                 795                 800

Thr Trp Ile Glu Gly Val Met Arg Asn Asn
                805                 810


<210>  5
<211>  2145
<212>  DNA
<213>  LYS77-PLG£¨Lys-ÏËÈÜÃ¸Ô-£©ºËËáÐòÁÐ

<400>  5
aaagtgtatc tctcagagtg caagactggg aatggaaaga actacagagg gacgatgtcc      60

aaaacaaaaa atggcatcac ctgtcaaaaa tggagttcca cttctcccca cagacctaga     120

ttctcacctg ctacacaccc ctcagaggga ctggaggaga actactgcag gaatccagac     180

aacgatccgc aggggccctg gtgctatact actgatccag aaaagagata tgactactgc     240

gacattcttg agtgtgaaga ggaatgtatg cattgcagtg gagaaaacta tgacggcaaa     300

atttccaaga ccatgtctgg actggaatgc caggcctggg actctcagag cccacacgct     360

catggataca ttccttccaa atttccaaac aagaacctga agaagaatta ctgtcgtaac     420

cccgataggg agctgcggcc ttggtgtttc accaccgacc ccaacaagcg ctgggaactt     480

tgtgacatcc cccgctgcac aacacctcca ccatcttctg gtcccaccta ccagtgtctg     540
```

aagggaacag gtgaaaacta tcgcgggaat gtggctgtta ccgtgtccgg gcacacctgt 600

cagcactgga gtgcacagac ccctcacaca cataacagga caccagaaaa cttcccctgc 660

aaaaatttgg atgaaaacta ctgccgcaat cctgacggaa aaagggcccc atggtgccat 720

acaaccaaca gccaagtgcg gtgggagtac tgtaagatac cgtcctgtga ctcctcccca 780

gtatccacgg aacaattggc tcccacagca ccacctgagc taacccctgt ggtccaggac 840

tgctaccatg gtgatggaca gagctaccga ggcacatcct ccaccaccac cacaggaaag 900

aagtgtcagt cttggtcatc tatgacacca caccggcacc agaagacccc agaaaactac 960

ccaaatgctg gcctgacaat gaactactgc aggaatccag atgccgataa aggcccctgg 1020

tgttttacca cagaccccag cgtcaggtgg gagtactgca acctgaaaaa atgctcagga 1080

acagaagcga gtgttgtagc acctccgcct gttgtcctgc ttccagatgt agagactcct 1140

tccgaagaag actgtatgtt tgggaatggg aaaggatacc gaggcaagag ggcgaccact 1200

gttactggga cgccatgcca ggactgggct gcccaggagc cccatagaca cagcattttc 1260

actccagaga caaatccacg ggcgggtctg gaaaaaaatt actgccgtaa ccctgatggt 1320

gatgtaggtg gtccctggtg ctacacgaca aatccaagaa aactttacga ctactgtgat 1380

gtccctcagt gtgcggcccc ttcatttgat tgtgggaagc ctcaagtgga gccgaagaaa 1440

tgtcctggaa gggttgtagg ggggtgtgtg gcccacccac attcctggcc ctggcaagtc 1500

agtcttagaa caaggtttgg aatgcacttc tgtggaggca ccttgatatc cccagagtgg 1560

gtgttgactg ctgcccactg cttggagaag tccccaaggc cttcatccta caaggtcatc 1620

ctgggtgcac accaagaagt gaatctcgaa ccgcatgttc aggaaataga agtgtctagg 1680

ctgttcttgg agcccacacg aaaagatatt gccttgctaa agctaagcag tcctgccgtc 1740

atcactgaca aagtaatccc agcttgtctg ccatccccaa attatgtggt cgctgaccgg 1800

accgaatgtt tcatcactgg ctggggagaa acccaaggta cttttggagc tggccttctc 1860

aaggaagccc agctccctgt gattgagaat aaagtgtgca atcgctatga gtttctgaat 1920

ggaagagtcc aatccaccga actctgtgct gggcatttgg ccggaggcac tgacagttgc 1980

cagggtgaca gtggaggtcc tctggtttgc ttcgagaagg acaaatacat tttacaagga 2040

gtcacttctt ggggtcttgg ctgtgcacgc cccaataagc ctggtgtcta tgttcgtgtt 2100

tcaaggtttg ttacttggat tgagggagtg atgagaaata attaa 2145

<210> 6
<211> 714
<212> PRT
<213> LYS77-PLG£¨Lys-ÏÈÈÜÃ¸Ô-£©°±»ùËáÐòÁÐ

<400> 6

Lys Val Tyr Leu Ser Glu Cys Lys Thr Gly Asn Gly Lys Asn Tyr Arg

```
1               5                   10                  15

Gly Thr Met Ser Lys Thr Lys Asn Gly Ile Thr Cys Gln Lys Trp Ser
            20                  25                  30

Ser Thr Ser Pro His Arg Pro Arg Phe Ser Pro Ala Thr His Pro Ser
        35                  40                  45

Glu Gly Leu Glu Glu Asn Tyr Cys Arg Asn Pro Asp Asn Asp Pro Gln
    50                  55                  60

Gly Pro Trp Cys Tyr Thr Thr Asp Pro Glu Lys Arg Tyr Asp Tyr Cys
65                  70                  75                  80

Asp Ile Leu Glu Cys Glu Glu Glu Cys Met His Cys Ser Gly Glu Asn
                85                  90                  95

Tyr Asp Gly Lys Ile Ser Lys Thr Met Ser Gly Leu Glu Cys Gln Ala
            100                 105                 110

Trp Asp Ser Gln Ser Pro His Ala His Gly Tyr Ile Pro Ser Lys Phe
        115                 120                 125

Pro Asn Lys Asn Leu Lys Lys Asn Tyr Cys Arg Asn Pro Asp Arg Glu
    130                 135                 140

Leu Arg Pro Trp Cys Phe Thr Thr Asp Pro Asn Lys Arg Trp Glu Leu
145                 150                 155                 160

Cys Asp Ile Pro Arg Cys Thr Thr Pro Pro Pro Ser Ser Gly Pro Thr
                165                 170                 175

Tyr Gln Cys Leu Lys Gly Thr Gly Glu Asn Tyr Arg Gly Asn Val Ala
            180                 185                 190

Val Thr Val Ser Gly His Thr Cys Gln His Trp Ser Ala Gln Thr Pro
        195                 200                 205

His Thr His Asn Arg Thr Pro Glu Asn Phe Pro Cys Lys Asn Leu Asp
    210                 215                 220

Glu Asn Tyr Cys Arg Asn Pro Asp Gly Lys Arg Ala Pro Trp Cys His
225                 230                 235                 240

Thr Thr Asn Ser Gln Val Arg Trp Glu Tyr Cys Lys Ile Pro Ser Cys
                245                 250                 255
```

```
Asp Ser Ser Pro Val Ser Thr Glu Gln Leu Ala Pro Thr Ala Pro Pro
            260                 265                 270

Glu Leu Thr Pro Val Val Gln Asp Cys Tyr His Gly Asp Gly Gln Ser
        275                 280                 285

Tyr Arg Gly Thr Ser Ser Thr Thr Thr Thr Gly Lys Lys Cys Gln Ser
    290                 295                 300

Trp Ser Ser Met Thr Pro His Arg His Gln Lys Thr Pro Glu Asn Tyr
305                 310                 315                 320

Pro Asn Ala Gly Leu Thr Met Asn Tyr Cys Arg Asn Pro Asp Ala Asp
                325                 330                 335

Lys Gly Pro Trp Cys Phe Thr Thr Asp Pro Ser Val Arg Trp Glu Tyr
            340                 345                 350

Cys Asn Leu Lys Lys Cys Ser Gly Thr Glu Ala Ser Val Val Ala Pro
        355                 360                 365

Pro Pro Val Val Leu Leu Pro Asp Val Glu Thr Pro Ser Glu Glu Asp
    370                 375                 380

Cys Met Phe Gly Asn Gly Lys Gly Tyr Arg Gly Lys Arg Ala Thr Thr
385                 390                 395                 400

Val Thr Gly Thr Pro Cys Gln Asp Trp Ala Ala Gln Glu Pro His Arg
                405                 410                 415

His Ser Ile Phe Thr Pro Glu Thr Asn Pro Arg Ala Gly Leu Glu Lys
            420                 425                 430

Asn Tyr Cys Arg Asn Pro Asp Gly Asp Val Gly Gly Pro Trp Cys Tyr
        435                 440                 445

Thr Thr Asn Pro Arg Lys Leu Tyr Asp Tyr Cys Asp Val Pro Gln Cys
    450                 455                 460

Ala Ala Pro Ser Phe Asp Cys Gly Lys Pro Gln Val Glu Pro Lys Lys
465                 470                 475                 480

Cys Pro Gly Arg Val Val Gly Gly Cys Val Ala His Pro His Ser Trp
                485                 490                 495

Pro Trp Gln Val Ser Leu Arg Thr Arg Phe Gly Met His Phe Cys Gly
            500                 505                 510
```

```
Gly Thr Leu Ile Ser Pro Glu Trp Val Leu Thr Ala Ala His Cys Leu
        515                 520                 525

Glu Lys Ser Pro Arg Pro Ser Ser Tyr Lys Val Ile Leu Gly Ala His
    530                 535                 540

Gln Glu Val Asn Leu Glu Pro His Val Gln Glu Ile Glu Val Ser Arg
545                 550                 555                 560

Leu Phe Leu Glu Pro Thr Arg Lys Asp Ile Ala Leu Leu Lys Leu Ser
                565                 570                 575

Ser Pro Ala Val Ile Thr Asp Lys Val Ile Pro Ala Cys Leu Pro Ser
            580                 585                 590

Pro Asn Tyr Val Val Ala Asp Arg Thr Glu Cys Phe Ile Thr Gly Trp
        595                 600                 605

Gly Glu Thr Gln Gly Thr Phe Gly Ala Gly Leu Leu Lys Glu Ala Gln
    610                 615                 620

Leu Pro Val Ile Glu Asn Lys Val Cys Asn Arg Tyr Glu Phe Leu Asn
625                 630                 635                 640

Gly Arg Val Gln Ser Thr Glu Leu Cys Ala Gly His Leu Ala Gly Gly
                645                 650                 655

Thr Asp Ser Cys Gln Gly Asp Ser Gly Gly Pro Leu Val Cys Phe Glu
            660                 665                 670

Lys Asp Lys Tyr Ile Leu Gln Gly Val Thr Ser Trp Gly Leu Gly Cys
        675                 680                 685

Ala Arg Pro Asn Lys Pro Gly Val Tyr Val Arg Val Ser Arg Phe Val
    690                 695                 700

Thr Trp Ile Glu Gly Val Met Arg Asn Asn
705                 710
```

<210> 7
<211> 1245
<212> DNA
<213> delta-plg£¨delta-ÏËÈÜÃ¸Ô£©ºËËáÐòÁÐ

<400> 7

```
gagcctctgg atgactatgt gaatacccag gggcttcac tgttcagtgt cactaagaag      60

cagctgggag caggaagtat agaagaatgt gcagcaaaat gtgaggagga cgaagaattc     120
```

acctgcaggg cattccaata tcacagtaaa gagcaacaat gtgtgataat ggctgaaaac 180
aggaagtcct ccataatcat taggatgaga gatgtagttt tatttgaaaa gaaagtgtat 240
ctctcagagt gcaagactgg gaatggaaag aactacagag ggacgatgtc caaaacaaaa 300
aatggcatca cctgtcaaaa atggagttcc acttctcccc acagacctag attctcacct 360
gctacacacc cctcagaggg actggaggag aactactgca ggaatccaga caacgatccg 420
caggggccct ggtgctatac tactgatcca gaaaagagat atgactactg cgacattctt 480
gagtgtgaag aggcggcccc ttcatttgat tgtgggaagc ctcaagtgga gccgaagaaa 540
tgtcctggaa gggttgtagg ggggtgtgtg gcccacccac attcctggcc ctggcaagtc 600
agtcttagaa caaggtttgg aatgcacttc tgtggaggca ccttgatatc cccagagtgg 660
gtgttgactg ctgcccactg cttggagaag tccccaaggc cttcatccta caaggtcatc 720
ctgggtgcac accaagaagt gaatctcgaa ccgcatgttc aggaaataga agtgtctagg 780
ctgttcttgg agcccacacg aaaagatatt gccttgctaa agctaagcag tcctgccgtc 840
atcactgaca aagtaatccc agcttgtctg ccatccccaa attatgtggt cgctgaccgg 900
accgaatgtt tcatcactgg ctggggagaa acccaaggta cttttggagc tggccttctc 960
aaggaagccc agctccctgt gattgagaat aaagtgtgca atcgctatga gtttctgaat 1020
ggaagagtcc aatccaccga actctgtgct gggcatttgg ccggaggcac tgacagttgc 1080
cagggtgaca gtggaggtcc tctggtttgc ttcgagaagg acaaatacat tttacaagga 1140
gtcacttctt ggggtcttgg ctgtgcacgc cccaataagc ctggtgtcta tgttcgtgtt 1200
tcaaggtttg ttacttggat tgagggagtg atgagaaata attaa 1245

<210> 8
<211> 414
<212> PRT
<213> delta-plg£¨delta-ÏËÈÜÃ¸Ô-£©°±»ùËáÐòÁÐ

<400> 8

Glu Pro Leu Asp Asp Tyr Val Asn Thr Gln Gly Ala Ser Leu Phe Ser
1 5 10 15

Val Thr Lys Lys Gln Leu Gly Ala Gly Ser Ile Glu Glu Cys Ala Ala
20 25 30

Lys Cys Glu Glu Asp Glu Glu Phe Thr Cys Arg Ala Phe Gln Tyr His
35 40 45

Ser Lys Glu Gln Gln Cys Val Ile Met Ala Glu Asn Arg Lys Ser Ser
50 55 60

Ile Ile Ile Arg Met Arg Asp Val Val Leu Phe Glu Lys Lys Val Tyr

```
65                  70                  75                  80

Leu Ser Glu Cys Lys Thr Gly Asn Gly Lys Asn Tyr Arg Gly Thr Met
                85                  90                  95

Ser Lys Thr Lys Asn Gly Ile Thr Cys Gln Lys Trp Ser Ser Thr Ser
            100                 105                 110

Pro His Arg Pro Arg Phe Ser Pro Ala Thr His Pro Ser Glu Gly Leu
        115                 120                 125

Glu Glu Asn Tyr Cys Arg Asn Pro Asp Asn Asp Pro Gln Gly Pro Trp
    130                 135                 140

Cys Tyr Thr Thr Asp Pro Glu Lys Arg Tyr Asp Tyr Cys Asp Ile Leu
145                 150                 155                 160

Glu Cys Glu Glu Ala Ala Pro Ser Phe Asp Cys Gly Lys Pro Gln Val
                165                 170                 175

Glu Pro Lys Lys Cys Pro Gly Arg Val Val Gly Gly Cys Val Ala His
            180                 185                 190

Pro His Ser Trp Pro Trp Gln Val Ser Leu Arg Thr Arg Phe Gly Met
        195                 200                 205

His Phe Cys Gly Gly Thr Leu Ile Ser Pro Glu Trp Val Leu Thr Ala
    210                 215                 220

Ala His Cys Leu Glu Lys Ser Pro Arg Pro Ser Ser Tyr Lys Val Ile
225                 230                 235                 240

Leu Gly Ala His Gln Glu Val Asn Leu Glu Pro His Val Gln Glu Ile
                245                 250                 255

Glu Val Ser Arg Leu Phe Leu Glu Pro Thr Arg Lys Asp Ile Ala Leu
            260                 265                 270

Leu Lys Leu Ser Ser Pro Ala Val Ile Thr Asp Lys Val Ile Pro Ala
        275                 280                 285

Cys Leu Pro Ser Pro Asn Tyr Val Val Ala Asp Arg Thr Glu Cys Phe
    290                 295                 300

Ile Thr Gly Trp Gly Glu Thr Gln Gly Thr Phe Gly Ala Gly Leu Leu
305                 310                 315                 320
```

```
Lys Glu Ala Gln Leu Pro Val Ile Glu Asn Lys Val Cys Asn Arg Tyr
                325                 330                 335

Glu Phe Leu Asn Gly Arg Val Gln Ser Thr Glu Leu Cys Ala Gly His
            340                 345                 350

Leu Ala Gly Gly Thr Asp Ser Cys Gln Gly Asp Ser Gly Gly Pro Leu
        355                 360                 365

Val Cys Phe Glu Lys Asp Lys Tyr Ile Leu Gln Gly Val Thr Ser Trp
    370                 375                 380

Gly Leu Gly Cys Ala Arg Pro Asn Lys Pro Gly Val Tyr Val Arg Val
385                 390                 395                 400

Ser Arg Phe Val Thr Trp Ile Glu Gly Val Met Arg Asn Asn
                405                 410


<210>  9
<211>  1104
<212>  DNA
<213>  Mini-plg£¨Ð¡ÏËÎ¬µ°°×ÈÜÃ¸Ô£©ºËËáÐòÁÐ

<400>  9
gtcaggtggg agtactgcaa cctgaaaaaa tgctcaggaa cagaagcgag tgttgtagca      60

cctccgcctg ttgtcctgct tccagatgta gagactcctt ccgaagaaga ctgtatgttt     120

gggaatggga aaggataccg aggcaagagg gcgaccactg ttactgggac gccatgccag     180

gactgggctg cccaggagcc ccatagacac agcattttca ctccagagac aaatccacgg     240

gcgggtctgg aaaaaaatta ctgccgtaac cctgatggtg atgtaggtgg tccctggtgc     300

tacacgacaa atccaagaaa actttacgac tactgtgatg tccctcagtg tgcggcccct     360

tcatttgatt gtgggaagcc tcaagtggag ccgaagaaat gtcctggaag ggttgtaggg     420

gggtgtgtgg cccacccaca ttcctggccc tggcaagtca gtcttagaac aaggtttgga     480

atgcacttct gtggaggcac cttgatatcc ccagagtggg tgttgactgc tgcccactgc     540

ttggagaagt ccccaaggcc ttcatcctac aaggtcatcc tgggtgcaca ccaagaagtg     600

aatctcgaac cgcatgttca ggaaatagaa gtgtctaggc tgttcttgga gcccacacga     660

aaagatattg ccttgctaaa gctaagcagt cctgccgtca tcactgacaa agtaatccca     720

gcttgtctgc catccccaaa ttatgtggtc gctgaccgga ccgaatgttt catcactggc     780

tggggagaaa cccaaggtac ttttggagct ggccttctca aggaagccca gctccctgtg     840

attgagaata aagtgtgcaa tcgctatgag tttctgaatg gaagagtcca atccaccgaa     900

ctctgtgctg ggcatttggc cggaggcact gacagttgcc agggtgacag tggaggtcct     960

ctggtttgct tcgagaagga caaatacatt ttacaaggag tcacttcttg ggtcttggc    1020
```

tgtgcacgcc ccaataagcc tggtgtctat gttcgtgttt caaggtttgt tacttggatt 1080

gagggagtga tgagaaataa ttaa 1104

<210> 10
<211> 367
<212> PRT
<213> Mini-plg£¨Ð¡ÏËÎ¬µºº×ÈÜÃ¸Ô£©º±»ùËáÐòÁÐ

<400> 10

Val Arg Trp Glu Tyr Cys Asn Leu Lys Lys Cys Ser Gly Thr Glu Ala
1 5 10 15

Ser Val Val Ala Pro Pro Pro Val Val Leu Leu Pro Asp Val Glu Thr
20 25 30

Pro Ser Glu Glu Asp Cys Met Phe Gly Asn Gly Lys Gly Tyr Arg Gly
35 40 45

Lys Arg Ala Thr Thr Val Thr Gly Thr Pro Cys Gln Asp Trp Ala Ala
50 55 60

Gln Glu Pro His Arg His Ser Ile Phe Thr Pro Glu Thr Asn Pro Arg
65 70 75 80

Ala Gly Leu Glu Lys Asn Tyr Cys Arg Asn Pro Asp Gly Asp Val Gly
85 90 95

Gly Pro Trp Cys Tyr Thr Thr Asn Pro Arg Lys Leu Tyr Asp Tyr Cys
100 105 110

Asp Val Pro Gln Cys Ala Ala Pro Ser Phe Asp Cys Gly Lys Pro Gln
115 120 125

Val Glu Pro Lys Lys Cys Pro Gly Arg Val Val Gly Gly Cys Val Ala
130 135 140

His Pro His Ser Trp Pro Trp Gln Val Ser Leu Arg Thr Arg Phe Gly
145 150 155 160

Met His Phe Cys Gly Gly Thr Leu Ile Ser Pro Glu Trp Val Leu Thr
165 170 175

Ala Ala His Cys Leu Glu Lys Ser Pro Arg Pro Ser Ser Tyr Lys Val
180 185 190

Ile Leu Gly Ala His Gln Glu Val Asn Leu Glu Pro His Val Gln Glu
195 200 205

```
Ile Glu Val Ser Arg Leu Phe Leu Glu Pro Thr Arg Lys Asp Ile Ala
    210                 215                 220

Leu Leu Lys Leu Ser Ser Pro Ala Val Ile Thr Asp Lys Val Ile Pro
225                 230                 235                 240

Ala Cys Leu Pro Ser Pro Asn Tyr Val Val Ala Asp Arg Thr Glu Cys
                245                 250                 255

Phe Ile Thr Gly Trp Gly Glu Thr Gln Gly Thr Phe Gly Ala Gly Leu
            260                 265                 270

Leu Lys Glu Ala Gln Leu Pro Val Ile Glu Asn Lys Val Cys Asn Arg
        275                 280                 285

Tyr Glu Phe Leu Asn Gly Arg Val Gln Ser Thr Glu Leu Cys Ala Gly
    290                 295                 300

His Leu Ala Gly Gly Thr Asp Ser Cys Gln Gly Asp Ser Gly Gly Pro
305                 310                 315                 320

Leu Val Cys Phe Glu Lys Asp Lys Tyr Ile Leu Gln Gly Val Thr Ser
                325                 330                 335

Trp Gly Leu Gly Cys Ala Arg Pro Asn Lys Pro Gly Val Tyr Val Arg
            340                 345                 350

Val Ser Arg Phe Val Thr Trp Ile Glu Gly Val Met Arg Asn Asn
        355                 360                 365


<210>  11
<211>  750
<212>  DNA
<213>  Micro-plg£¨Î¢ÏËÎ¬µ°°×ÈÜÃ¸Ô£©ºËËáÐòÁÐ

<400>  11
gccccttcat ttgattgtgg gaagcctcaa gtggagccga agaaatgtcc tggaagggtt      60

gtaggggggt gtgtggccca cccacattcc tggccctggc aagtcagtct tagaacaagg     120

tttggaatgc acttctgtgg aggcaccttg atatccccag agtgggtgtt gactgctgcc     180

cactgcttgg agaagtcccc aaggccttca tcctacaagg tcatcctggg tgcacaccaa     240

gaagtgaatc tcgaaccgca tgttcaggaa atagaagtgt ctaggctgtt cttggagccc     300

acacgaaaag atattgcctt gctaaagcta agcagtcctg ccgtcatcac tgacaaagta     360

atcccagctt gtctgccatc cccaaattat gtggtcgctg accggaccga atgtttcatc     420

actggctggg gagaaaccca aggtactttt ggagctggcc ttctcaagga agcccagctc     480
```

cctgtgattg agaataaagt gtgcaatcgc tatgagtttc tgaatggaag agtccaatcc 540

accgaactct gtgctgggca tttggccgga ggcactgaca gttgccaggg tgacagtgga 600

ggtcctctgg tttgcttcga gaaggacaaa tacattttac aaggagtcac ttcttggggt 660

cttggctgtg cacgccccaa taagcctggt gtctatgttc gtgtttcaag gtttgttact 720

tggattgagg gagtgatgag aaataattaa 750

<210> 12
<211> 249
<212> PRT
<213> Micro-plg£¨Î¢ÏËÎ¬µººÍÈÜÃ¸Ô£©º±»ùËáÐòÁÐ

<400> 12

Ala Pro Ser Phe Asp Cys Gly Lys Pro Gln Val Glu Pro Lys Lys Cys
1 5 10 15

Pro Gly Arg Val Val Gly Gly Cys Val Ala His Pro His Ser Trp Pro
20 25 30

Trp Gln Val Ser Leu Arg Thr Arg Phe Gly Met His Phe Cys Gly Gly
35 40 45

Thr Leu Ile Ser Pro Glu Trp Val Leu Thr Ala Ala His Cys Leu Glu
50 55 60

Lys Ser Pro Arg Pro Ser Ser Tyr Lys Val Ile Leu Gly Ala His Gln
65 70 75 80

Glu Val Asn Leu Glu Pro His Val Gln Glu Ile Glu Val Ser Arg Leu
85 90 95

Phe Leu Glu Pro Thr Arg Lys Asp Ile Ala Leu Leu Lys Leu Ser Ser
100 105 110

Pro Ala Val Ile Thr Asp Lys Val Ile Pro Ala Cys Leu Pro Ser Pro
115 120 125

Asn Tyr Val Val Ala Asp Arg Thr Glu Cys Phe Ile Thr Gly Trp Gly
130 135 140

Glu Thr Gln Gly Thr Phe Gly Ala Gly Leu Leu Lys Glu Ala Gln Leu
145 150 155 160

Pro Val Ile Glu Asn Lys Val Cys Asn Arg Tyr Glu Phe Leu Asn Gly
165 170 175

```
Arg Val Gln Ser Thr Glu Leu Cys Ala Gly His Leu Ala Gly Gly Thr
            180                 185                 190

Asp Ser Cys Gln Gly Asp Ser Gly Gly Pro Leu Val Cys Phe Glu Lys
        195                 200                 205

Asp Lys Tyr Ile Leu Gln Gly Val Thr Ser Trp Gly Leu Gly Cys Ala
    210                 215                 220

Arg Pro Asn Lys Pro Gly Val Tyr Val Arg Val Ser Arg Phe Val Thr
225                 230                 235                 240

Trp Ile Glu Gly Val Met Arg Asn Asn
                245


<210>  13
<211>  684
<212>  DNA
<213>  Ë¿º±Ëáµººx Ã¸£¨½á¹¹£©ÓòµÄºËËáÐòÁÐ

<400>  13
gttgtagggg ggtgtgtggc ccacccacat tcctggccct ggcaagtcag tcttagaaca        60

aggtttggaa tgcacttctg tggaggcacc ttgatatccc cagagtgggt gttgactgct       120

gcccactgct tggagaagtc cccaaggcct tcatcctaca aggtcatcct gggtgcacac       180

caagaagtga atctcgaacc gcatgttcag gaaatagaag tgtctaggct gttcttggag       240

cccacacgaa aagatattgc cttgctaaag ctaagcagtc ctgccgtcat cactgacaaa       300

gtaatcccag cttgtctgcc atccccaaat tatgtggtcg ctgaccggac cgaatgtttc       360

atcactggct ggggagaaac ccaaggtact tttggagctg gccttctcaa ggaagcccag       420

ctccctgtga ttgagaataa agtgtgcaat cgctatgagt ttctgaatgg aagagtccaa       480

tccaccgaac tctgtgctgg gcatttggcc ggaggcactg acagttgcca gggtgacagt       540

ggaggtcctc tggtttgctt cgagaaggac aaatacattt tacaaggagt cacttcttgg       600

ggtcttggct gtgcacgccc caataagcct ggtgtctatg ttcgtgtttc aaggtttgtt       660

acttggattg agggagtgat gaga                                              684


<210>  14
<211>  228
<212>  PRT
<213>  Ë¿º±Ëáµººx Ã¸£¨½á¹¹£©ÓòµÄº±»ùËáÐòÁÐ

<400>  14

Val Val Gly Gly Cys Val Ala His Pro His Ser Trp Pro Trp Gln Val
1               5                   10                  15

Ser Leu Arg Thr Arg Phe Gly Met His Phe Cys Gly Gly Thr Leu Ile
```

```
                20                  25                  30

Ser Pro Glu Trp Val Leu Thr Ala Ala His Cys Leu Glu Lys Ser Pro
        35                  40                  45

Arg Pro Ser Ser Tyr Lys Val Ile Leu Gly Ala His Gln Glu Val Asn
    50                  55                  60

Leu Glu Pro His Val Gln Glu Ile Glu Val Ser Arg Leu Phe Leu Glu
65                  70                  75                  80

Pro Thr Arg Lys Asp Ile Ala Leu Leu Lys Leu Ser Ser Pro Ala Val
                85                  90                  95

Ile Thr Asp Lys Val Ile Pro Ala Cys Leu Pro Ser Pro Asn Tyr Val
            100                 105                 110

Val Ala Asp Arg Thr Glu Cys Phe Ile Thr Gly Trp Gly Glu Thr Gln
        115                 120                 125

Gly Thr Phe Gly Ala Gly Leu Leu Lys Glu Ala Gln Leu Pro Val Ile
    130                 135                 140

Glu Asn Lys Val Cys Asn Arg Tyr Glu Phe Leu Asn Gly Arg Val Gln
145                 150                 155                 160

Ser Thr Glu Leu Cys Ala Gly His Leu Ala Gly Gly Thr Asp Ser Cys
                165                 170                 175

Gln Gly Asp Ser Gly Gly Pro Leu Val Cys Phe Glu Lys Asp Lys Tyr
            180                 185                 190

Ile Leu Gln Gly Val Thr Ser Trp Gly Leu Gly Cys Ala Arg Pro Asn
        195                 200                 205

Lys Pro Gly Val Tyr Val Arg Val Ser Arg Phe Val Thr Trp Ile Glu
    210                 215                 220

Gly Val Met Arg
225
```

## Claims

1. A method for preventing and/or treating a renal tissue injury in a subject, comprising administering an effective amount of plasminogen to the subject, wherein the subject has a risk of the renal tissue injury, is suspected of having the renal tissue injury or suffers from the renal tissue injury.

2. The method of claim 1, wherein the renal tissue injury comprises a renal tissue injury caused by an infection, an inflammation, an allergic reaction, autoimmunity, ischemia, a thrombus, microangiopathy, a trauma, a radiation injury, a glucose metabolic disorder, an electrolyte disorder, a fat metabolism disorder, and a cancer.

3. The method of claim 1 or 2, wherein the renal tissue injury is a renal tissue injury caused by a systemic disease selected from hypertension, diabetes mellitus, atherosclerosis, systemic sclerosis, systemic lupus erythematosus, hyperlipemia, non-Hodgkin's lymphoma, multiple myeloma, systemic vasculitis, anaphylactoid purpura, polymyositis, and thrombotic microangiopathies.

4. The method of claim 1 or 2, wherein the renal tissue injury is a renal tissue injury caused by a chronic kidney disease.

5. The method of claim 4, wherein the chronic kidney disease is chronic glomerulonephritis, chronic pyelonephritis, nephrotic syndrome, renal insufficiency, renal failure or uremia.

6. The method of claim 1 or 2, wherein the chronic kidney disease is a drug-induced chronic renal injury.

7. The method of claim 6, wherein the drug comprises a chemotherapeutic drug, an antihypertensive drug, a hypolipidemic drug, a hypoglycemic drug, a nonsteroid anti-inflammatory drug, an antibiotic drug, and an antiviral drug.

8. The method of claim 7, wherein the drug is a chemotherapeutic drug, in particular cisplatin.

9. A method for preventing and/or treating an acute renal tissue injury in a subject, comprising administering an effective amount of plasminogen to the subject to protect a renal tissue.

10. The method of claim 9, wherein the plasminogen alleviates apoptosis in a renal tissue caused by an acute renal tissue injury.

11. The method of claim 9 or 10, wherein the plasminogen promotes repairs of the injured renal tissue.

12. The method of any one of claims 9 to 11, wherein the plasminogen alleviates fibrosis of the injured renal tissue.

13. The method of any one of claims 9 to 12, wherein the plasminogen promotes recovery of renal function.

14. The method of any one of claims 1 to 13, wherein the renal injury is acute glomerulonephritis, acute pyelonephritis, an acute renal injury, acute renal failure, acute renal insufficiency, and acute tubular necrosis.

15. The method of claims 1 to 13, wherein the acute renal injury is an acute renal injury induced by a chemotherapeutic drug.

16. A method for preventing and/or treating chronic renal tissue injury in a subject, comprising administering an effective amount of plasminogen to the subject to protect a renal tissue.

17. The method of claim 16, wherein the plasminogen alleviates apoptosis in a renal tissue.

18. The method of claim 16 or 17, wherein the plasminogen promotes repairs of the injured renal tissue.

19. The method of any one of claims 16 to 18, wherein the plasminogen alleviates fibrosis of the injured renal tissue.

20. The method of any one of claims 16 to 19, wherein the plasminogen promotes recovery of renal function.

21. The method of any one of claims 16 to 20, wherein the chronic renal injury is a renal tissue injury caused by a chronic renal tissue disease.

22. The method of any one of claims 16 to 20, wherein the chronic renal injury is a renal tissue injury elicited or accompanied by other diseases.

23. The method of claim 22, wherein the other diseases comprise hypertension, diabetes mellitus, atherosclerosis, hyperlipemia, hepatitis, hepatic cirrhosis, coronary heart disease, angina pectoris, and myocardial infarction.

**24.** The method of claim 23, wherein the other diseases are hypertension, diabetes mellitus, atherosclerosis, and hyperlipemia.

**25.** A method for preventing and/or treating a lipid deposition-induced renal tissue injury in a subject, comprising administering an effective amount of plasminogen to the subject.

**26.** The method of claim 25, wherein the lipid deposition is induced by hyperlipemia caused by abnormal fat or glucose metabolism in the subject.

**27.** A method for preventing and/or treating a renal tissue injury caused or accompanied by diabetes mellitus in a subject, comprising administering an effective amount of plasminogen to the subject.

**28.** A method for preventing and/or treating a renal tissue injury caused or accompanied by hyperlipemia in a subject, comprising administering an effective amount of plasminogen to the subject.

**29.** A method for preventing and/or treating a renal tissue injury caused or accompanied by atherosclerosis in a subject, comprising administering an effective amount of plasminogen to the subject.

**30.** A method for preventing and/or treating an ischemic renal tissue injury in a subject, comprising administering an effective amount of plasminogen to the subj ect.

**31.** The method of claim 30, wherein the plasminogen alleviates apoptosis in a renal tissue.

**32.** The method of claim 30 or 31, wherein the plasminogen promotes repair of an injured renal tissue.

**33.** The method of any one of claims 30 to 32, wherein the plasminogen alleviates fibrosis of the injured renal tissue.

**34.** The method of any one of claims 30 to 33, wherein the plasminogen promotes recovery of renal function.

**35.** The method of any one of claims 30 to 34, wherein the ischemia is caused by stenosis of a vascular lumen.

**36.** The method of any one of claims 30 to 34, wherein the ischemia is caused by a thrombus blocking a blood vessel.

**37.** The method of claim 35 or 36, wherein the ischemia is caused by hypertension, diabetes mellitus, atherosclerosis, and a heart disease.

**38.** A method for preventing and/or treating a renal tissue injury induced by ischemic reperfusion in a subject, comprising administering an effective amount of plasminogen to the subject.

**39.** A method for preventing and/or treating a renal tissue injury induced by an autoimmune response in a subject, comprising administering an effective amount of plasminogen to the subject.

**40.** The method of claim 39, wherein the renal tissue injury induced by an autoimmune response is caused by systemic sclerosis.

**41.** A method for preventing and/or treating an inflammation-induced renal tissue injury in a subject, comprising administering an effective amount of plasminogen to the subject.

**42.** The method of claim 41, wherein the inflammation is an acute and chronic renal parenchymal inflammation or renal interstitial inflammation.

**43.** The method of claim 42, wherein the plasminogen alleviates apoptosis in a renal tissue.

**44.** The method of claim 42 or 43, wherein the plasminogen promotes repair of an injured renal tissue.

**45.** The method of any one of claims 41 to 44, wherein the plasminogen alleviates fibrosis of the injured renal tissue.

**46.** The method of any one of claims 41 to 45, wherein the plasminogen promotes recovery of renal function.

**47.** The method of any one of claims 1 to 46, wherein the plasminogen has at least 75%, 80%, 85%, 90%, 95%, 96%, 97%, 98% or 99% sequence identity with SEQ ID No. 2, 6, 8, 10 or 12, and still has the plasminogen activity.

**48.** The method of any one of claims 1 to 47, wherein the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No. 2, 6, 8, 10 or 12, and still has the plasminogen activity.

**49.** The method of any one of claims 1 to 48, wherein the plasminogen is a protein that comprises a plasminogen active fragment and still has the plasminogen activity.

**50.** The method of any one of claims 1 to 49, wherein the plasminogen is selected from Glu-plasminogen, Lys-plasminogen, mini-plasminogen, micro-plasminogen, delta-plasminogen or their variants that retain the plasminogen activity.

**51.** The method of any one of claims 1 to 50, wherein the plasminogen can be administered in combination with one or more drugs selected from an antihypertensive drug, a hypolipidemic drug, an antibiotic drug, an anticoagulant drug, a thrombolytic drug, a diuretic drug, an anti-tumor drug, a hypoglycemic drug, a non-steroidal anti-inflammatory drug, an immunomodulatory drug, an anti-infective drug, an antiviral drug, a hormone, and an active ingredient of a natural product.

**52.** The method of any one of claims 1 to 51, wherein the plasminogen is a natural or synthetic human plasminogen, or a variant or fragment thereof that still retains the plasminogen activity.

**53.** The method of any one of claims 1 to 51, wherein the plasminogen is an ortholog of human plasminogen from a primate or a rodent, or a variant or fragment thereof that still retains the plasminogen activity.

**54.** The method of any one of claims 1 to 53, wherein the amino acids of the plasminogen are as shown in SEQ ID No. 2, 6, 8, 10 or 12.

**55.** The method of any one of claims 1 to 54, wherein the plasminogen is a natural human plasminogen.

**56.** The method of any one of claims 1 to 55, wherein the subject is a human.

**57.** The method of any one of claims 1 to 26, wherein the subject has a lack or deficiency of plasminogen.

**58.** The method of claim 57, wherein the lack or deficiency is congenital, secondary and/or local.

**59.** A plasminogen for use in the method of any one of claims 1 to 58.

**60.** A pharmaceutical composition, comprising a pharmaceutically acceptable carrier and the plasminogen for use in the method of any one of claims 1 to 58.

**61.** A preventive or therapeutic kit comprising: (i) the plasminogen for use in the method of any one of claims 1 to 58, and (ii) a means for delivering the plasminogen to the subject.

**62.** The kit of claim 61, wherein the means is a syringe or a vial.

**63.** The kit of claim 61 or 62, further comprising a label or an instruction for use indicating the administration of the plasminogen to the subject to implement the method of any one of claims 1 to 58.

**64.** An article of manufacture, comprising:
a container comprising a label; and

(i) the plasminogen for use in the method of any one of claims 1 to 58 or a pharmaceutical composition comprising the plasminogen, wherein the label indicates the administration of the plasminogen or the composition to the subject to implement the method of any one of claims 1 to 58.

**65.** The kit of any one of claims 61 to 63 or the article of manufacture of claim 64, further comprising one or more additional means or containers containing other drugs.

**66.** The kit or the article of manufacture of claim 65, wherein the other drugs are selected from a group of: a hypolipidemic drug, an anti-platelet drug, an antihypertensive drug, a vasodilator, a hypoglycemic drug, an anticoagulant drug, a thrombolytic drug, a hepatoprotective drug, an anti-arrhythmia drug, a cardiotonic drug, a diuretic drug, an anti-infective drug, an antiviral drug, an immunomodulatory drug, an inflammatory regulatory drug, an anti-tumor drug, a hormone drug, and thyroxine.

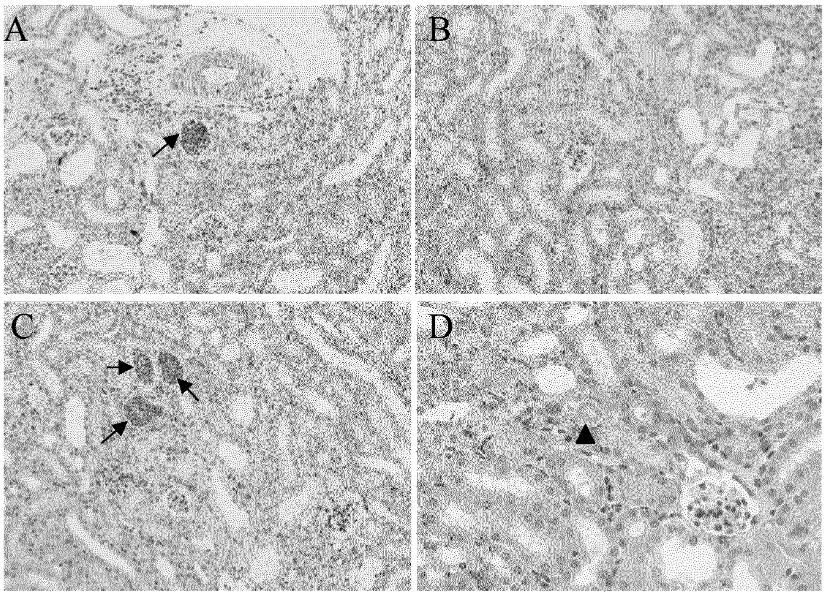


Fig.1

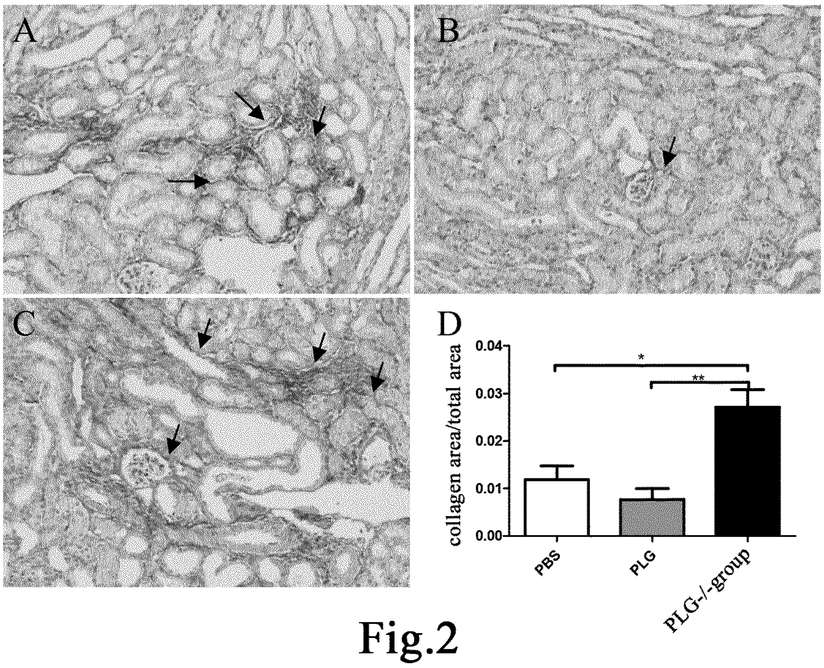


Fig.2

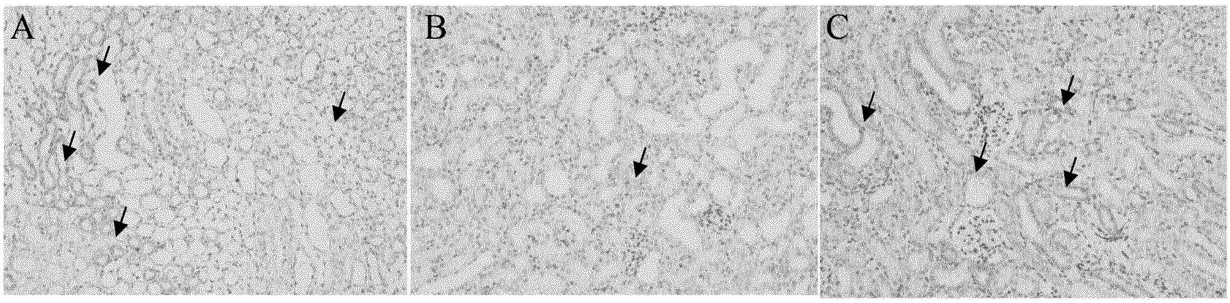


Fig.3

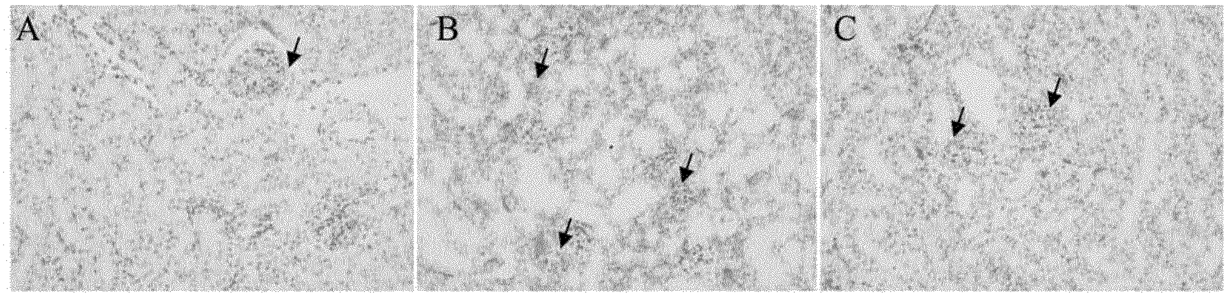


Fig.4

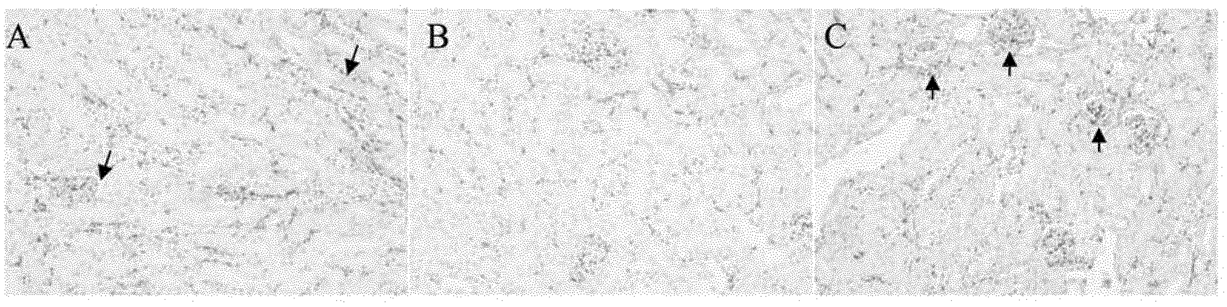


Fig.5

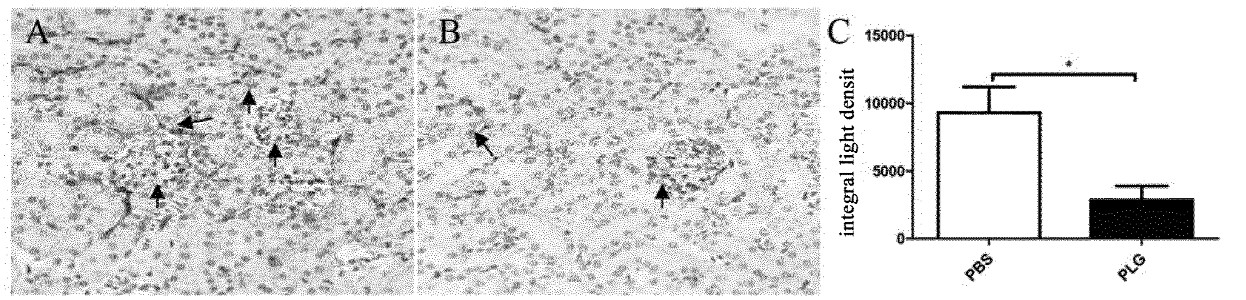


Fig.6

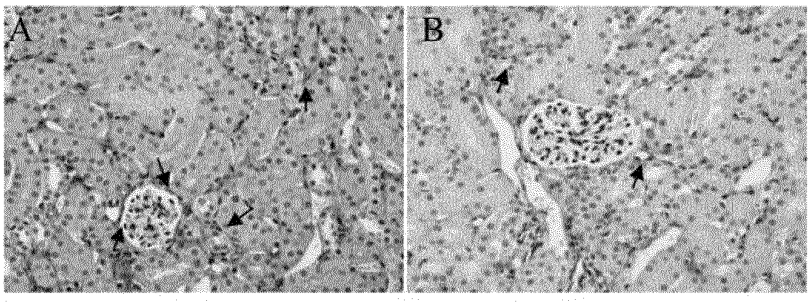


Fig.7

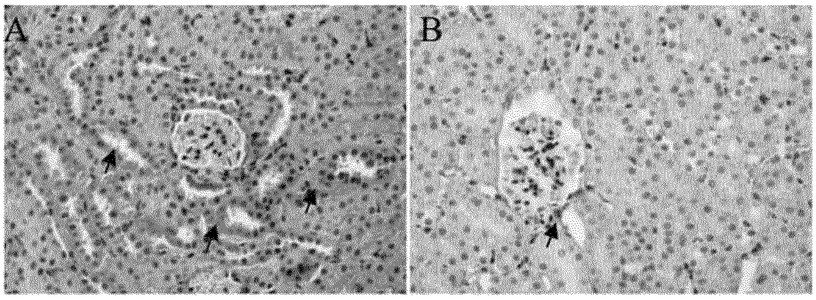


Fig.8

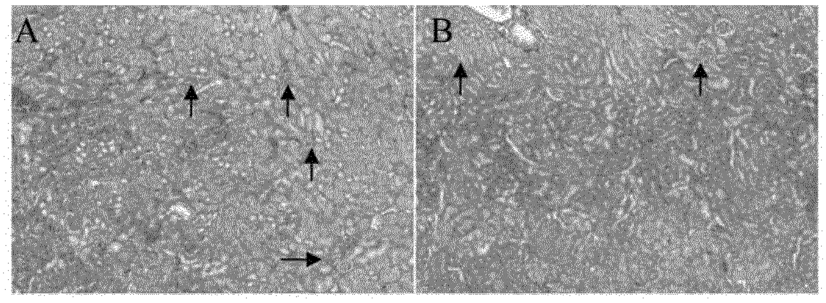


Fig.9

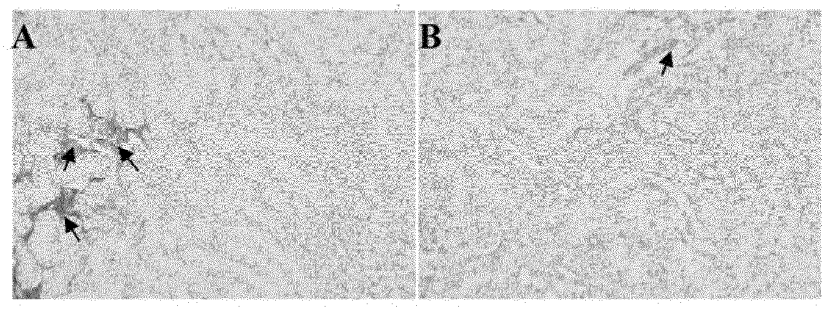


Fig.10

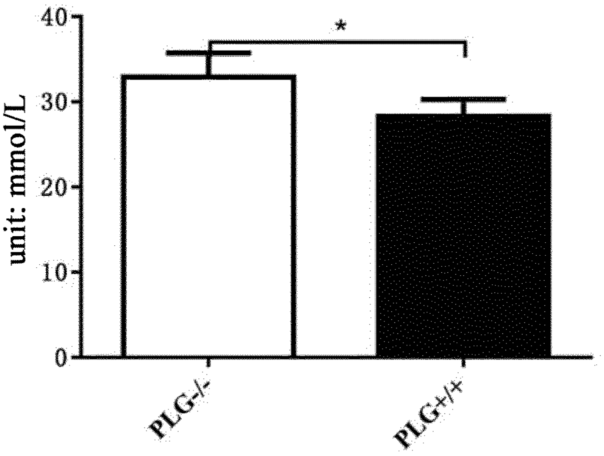


Fig.11

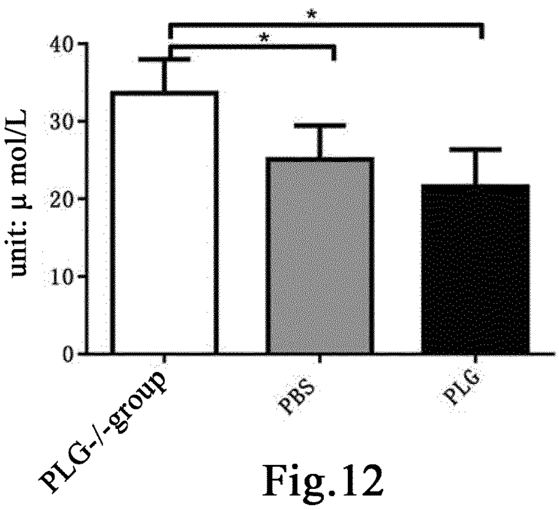


Fig.12

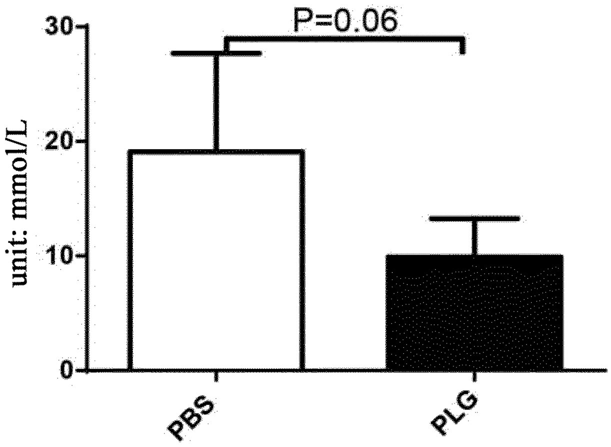


Fig.13

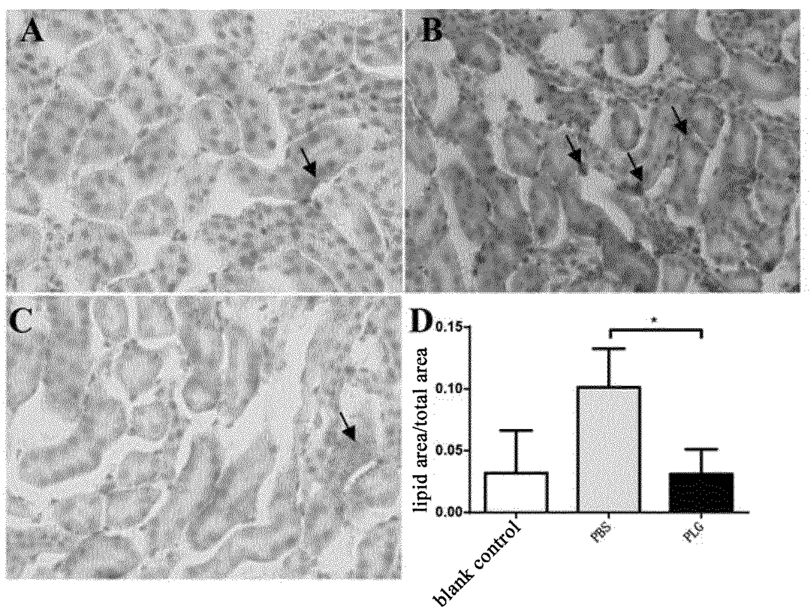


Fig.14

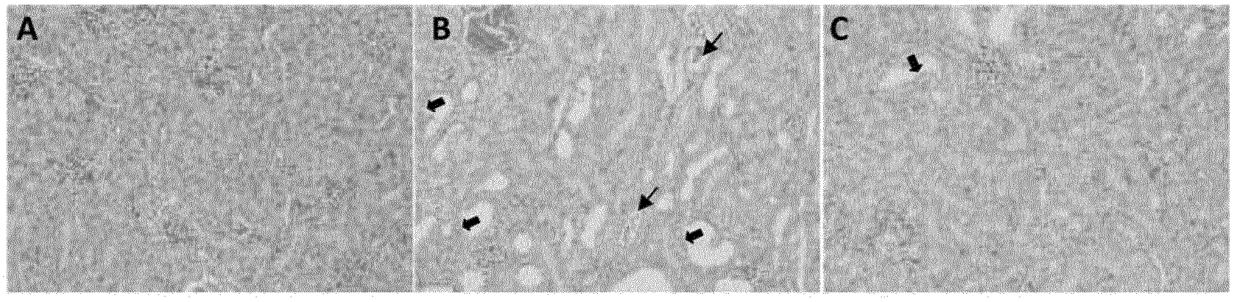


Fig.15

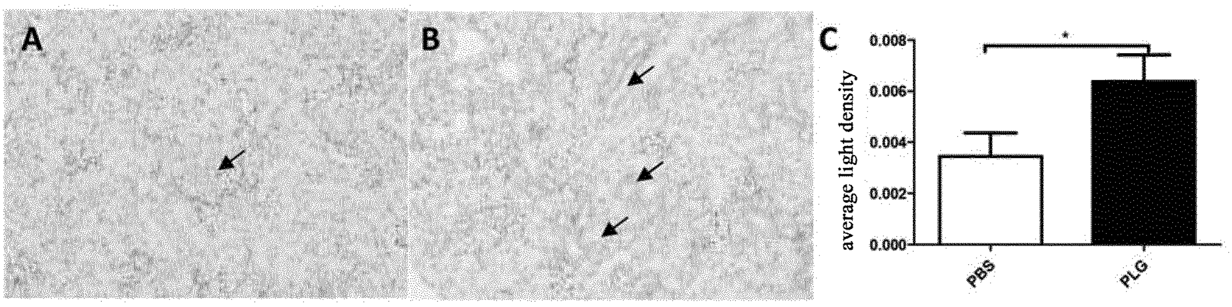


Fig.16

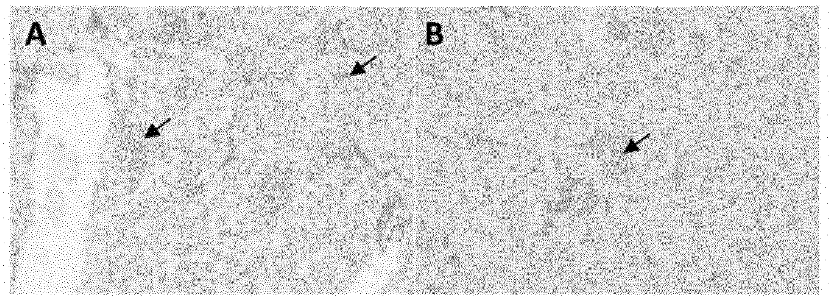


Fig.17

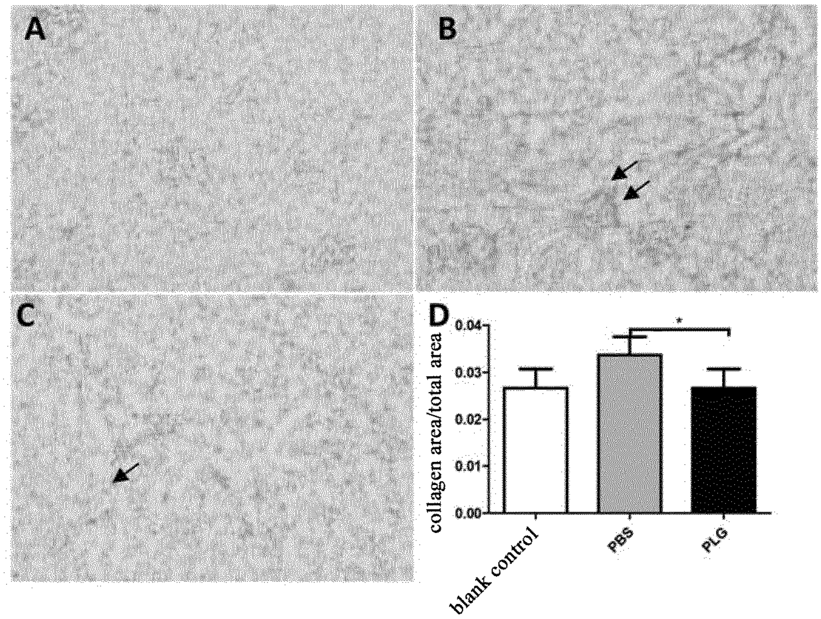
A
B
C
D
collagen area/total area
0.04
0.03
0.02
0.01
0.00
*
blank control
PBS
PLG

Fig.18

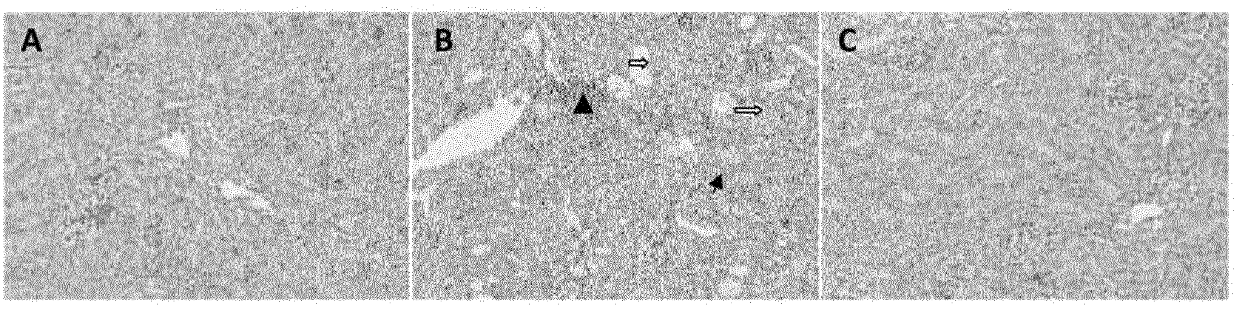
A
B
C

Fig.19

# INTERNATIONAL SEARCH REPORT

International application No.
PCT/CN2017/089060

**A. CLASSIFICATION OF SUBJECT MATTER**

A61K 38/48 (2006.01) i; A61P 13/12 (2006.01) i

According to International Patent Classification (IPC) or to both national classification and IPC

B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CJFD; CNABS; DWPI; SIPOABS; VEN; CNTXT; WOTXT; USTXT; EPTXT; GOOGLE; PUBMED; GenBank; EBI; ISI Web of knowledge; CNKI: profibrinolysin, 肾损伤, plasminogen, 损伤, 肾组织损伤, plg, 纤维溶酶原, 肾炎, 纤维蛋白溶解酶原, 肾功能衰竭, kidney injury, 纤溶酶原, profibrolysin, 肾小管坏死, 纤维蛋白溶酶原, SEQ ID NOs: 2, 6, 8, 10, 12

C. DOCUMENTS CONSIDERED TO BE RELEVANT

| Category* | Citation of document, with indication, where appropriate, of the relevant passages | Relevant to claim No. |
|---|---|---|
| X | CN 101573134 A (OMNIO HEALER AB) 04 November 2009 (04.11.2009), description, page 5, paragraph 5, page 10, paragraphs 2-7, page 12, paragraph 5, page 15, paragraphs 3, 4, 6 and 7 and page 34, paragraph 4 | 1-5, 9-14, 16-46, 49-53, 55-66 |
| Y | CN 101573134 A (OMNIO HEALER AB) 04 November 2009 (04.11.2009), description, page 5, paragraph 5, page 10, paragraphs 2-7, page 12, paragraph 5, page 15, paragraphs 3, 4, 6 and 7 and page 34, paragraph 4 | 6-8, 15, 47, 48, 54 |
| Y | 孙海鸥. 药物性肾损害的机制及其临床表现. 肾脏病与透析肾移植杂志. 30 June 2006 (30.06.2006), 15(3), page 252, left column, paragraph 1, page 253, left column, paragraph 2, and page 255, table 1. (Journal of Nephrology, Dialysis & Transplantation), non-official translation (Sun, Haiou, The Mechanism and Clinical Manifestations of Drug-induced Renal Damage.) | 6-8, 15 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 04 September 2017 | 14 September 2017 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br>ZHANG, Yanqing<br>Telephone No. (86-10) 61648130 |

Form PCT/ISA /210 (second sheet) (July 2009)

## INTERNATIONAL SEARCH REPORT

International application No.
PCT/CN2017/089060

C (Continuation). DOCUMENTS CONSIDERED TO BE RELEVANT

| Category* | Citation of document, with indication, where appropriate, of the relevant passages | Relevant to claim No. |
|---|---|---|
| Y | CN 1768138 A (N ZYME BIOTECHNOLOGY GMBH) 03 May 2006 (03.05.2006), description, sequence list, sequence 17, 18 and 32 | 47, 48, 54 |
| X | CN 102121023 A (SUN YAT-SEN UNIVERSITY) 13 July 2011 (13.07.2011), description, paragraphs [0002], [0005], [0008], [0009], and [0023]-[0025] | 1-3, 16, 22-24, 27, 49-53, 55-66 |
| A | CN 101563100 A (OMNIO HEALER AB) 21 October 2009 (21.10.2009), entire document | 1-66 |
| A | CN 87104683 A (EMORY UNIVERSITY) 21 December 1988 (21.12.1988), entire document | 1-66 |

Form PCT/ISA /210 (continuation of second sheet) (July 2009)

| INTERNATIONAL SEARCH REPORT | International application No. PCT/CN2017/089060 |
|---|---|

**Box No. I Nucleotide and/or amino acid sequence(s) (Continuation of item item 1.c of the first sheet)**

1. With regard to any nucleotide and/or amino acid sequence disclosed in the international application, the international search was carried out on the basis of a sequence listing filed or furnished:

   a. (means)

   ☐ on paper

   ☒ in electronic form

   b. (time)

   ☒ in the international application as filed

   ☐ together with the international application in electronic form

   ☐ subsequently to this Authority for the purposes of search

2. ☐ In addition, in the case that more than one version or copy of a sequence listing has been filed or furnished, the required statements that the information in the subsequent or additional copies is identical to that in the application as filed or does not go beyond the application as filed, as appropriate, were furnished.

3. Additional comments:

Form PCT/ISA /210 (continuation of first sheet (1)) (July 2009)

## INTERNATIONAL SEARCH REPORT

International application No.
PCT/CN2017/089060

**Box No. II Observations where certain claims were found unsearchable (Continuation of item 2 of first sheet)**

This international search report has not been established in respect of certain claims under Article 17(2)(a) for the following reasons:

1. ☒ Claims Nos.:1-58
because they relate to subject matter not required to be searched by this Authority, namely:
[1] Claims 1-58 are related to methods of treatment of diseases and therefore does not warrant an international search according to the criteria set out in PCT Rule 39.1(iv). This search report has been made based on the pharmaceutical uses of plasminogen.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2017/089060

| Patent Documents referred in the Report | Publication Date | Patent Family | Publication Date |
|---|---|---|---|
| CN 101573134 A | 04 November 2009 | CN 101563100 B | 07 August 2013 |
| | | CA 2662101 A1 | 06 March 2008 |
| | | EP 2056864 A2 | 13 May 2009 |
| | | KR 20090059122 A | 10 June 2009 |
| | | US 2013149321 A1 | 13 June 2013 |
| | | CN 101573134 B | 06 March 2013 |
| | | JP 2010502600 A | 28 January 2010 |
| | | EP 2056864 A4 | 25 January 2012 |
| | | JP 5566105 B2 | 06 August 2014 |
| | | DK 2056864 T3 | 10 March 2014 |
| | | AU 2007290881 B2 | 07 March 2013 |
| | | EA 200970233 A1 | 28 August 2009 |
| | | EA 016250 B1 | 30 March 2012 |
| | | EP 2056864 B1 | 11 December 2013 |
| | | CA 2662101 C | 07 July 2015 |
| | | WO 2008026999 A2 | 06 March 2008 |
| | | WO 2008026999 A3 | 22 May 2008 |
| | | AU 2007290881 A1 | 06 March 2008 |
| | | US 8318661 B2 | 27 November 2012 |
| | | ES 2451015 T3 | 26 March 2014 |
| | | US 2016243204 A1 | 25 August 2016 |
| | | CN 101563100 A | 21 October 2009 |
| | | MX 2009002226 A | 07 September 2009 |
| | | US 2010099600 A1 | 22 April 2010 |
| CN 1768138 A | 03 May 2006 | AU 2003210137 A1 | 02 September 2003 |
| | | WO 03066842 A3 | 10 June 2004 |
| | | JP 2005525798 A | 02 September 2005 |
| | | WO 03066842 A2 | 14 August 2003 |

Form PCT/ISA /210 (patent family annex) (July 2009)

# INTERNATIONAL SEARCH REPORT
Information on patent family members

International application No.
PCT/CN2017/089060

| Patent Documents referred in the Report | Publication Date | Patent Family | Publication Date |
|---|---|---|---|
| | | MX PA04007585 A | 20 September 2005 |
| | | CA 2475277 A1 | 14 August 2003 |
| | | AU 2003210137 A8 | 02 September 2003 |
| | | EP 1472346 A2 | 03 November 2004 |
| CN 102121023 A | 13 July 2011 | CN 102121023 B | 04 July 2012 |
| CN 101563100 A | 21 October 2009 | CN 101563100 B | 07 August 2013 |
| | | CA 2662101 A1 | 06 March 2008 |
| | | CN 101573134 A | 04 November 2009 |
| | | EP 2056864 A2 | 13 May 2009 |
| | | KR 20090059122 A | 10 June 2009 |
| | | US 2013149321 A1 | 13 June 2013 |
| | | CN 101573134 B | 06 March 2013 |
| | | JP 2010502600 A | 28 January 2010 |
| | | EP 2056864 A4 | 25 January 2012 |
| | | JP 5566105 B2 | 06 August 2014 |
| | | DK 2056864 T3 | 10 March 2014 |
| | | AU 2007290881 B2 | 07 March 2013 |
| | | EA 200970233 A1 | 28 August 2009 |
| | | EA 016250 B1 | 30 March 2012 |
| | | EP 2056864 B1 | 11 December 2013 |
| | | CA 2662101 C | 07 July 2015 |
| | | WO 2008026999 A2 | 06 March 2008 |
| | | WO 2008026999 A3 | 22 May 2008 |
| | | AU 2007290881 A1 | 06 March 2008 |
| | | US 8318661 B2 | 27 November 2012 |
| | | ES 2451015 T3 | 26 March 2014 |
| | | US 2016243204 A1 | 25 August 2016 |
| | | MX 2009002226 A | 07 September 2009 |

Form PCT/ISA /210 (patent family annex) (July 2009)

**INTERNATIONAL SEARCH REPORT**
Information on patent family members

International application No.
PCT/CN2017/089060

| Patent Documents referred in the Report | Publication Date | Patent Family | Publication Date |
|---|---|---|---|
| | | US 2010099600 A1 | 22 April 2010 |
| CN 87104683 A | 21 December 1988 | None | |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102154253 A
- WO 9704801 A
- US 3773919 A
- EP 58481 A
- US 20090156498 A

### Non-patent literature cited in the description


- The Peptides: Analysis, Synthesis, Biology. Vol. 2: Special Methods in Peptide Synthesis. *Barany and Solid-Phase Peptide Synthesis,* vol. 2, 3-284
- **MERRIFIELD et al.** *J. Am. Chem. Soc.,* 1963, vol. 85, 2149-2156
- **STEWART et al.** olid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **JAMES L. PIRKLE ; MD1 AND BARRY ; I. FREEDMAN, MD.** Hypertension and chronic kidney disease: controversies in pathogenesis and treatment. *Minerva UrolNefrol.,* March 2013, vol. 65 (1), 37-50
- **NGUYEN DCA ; TOUYZ RM.** A new look at the renin-angiotensin system-focusing on the vascular system [J. *Peptides,* 2011, vol. 32 (10), 2141-2150
- **KIMOTO E ; SHOJI T. SHINOHARA K et al.** Regional arterialstiffness inpatient with type 2 diabetes and chronic kidney disease [J. *J Am Soc Nephrol,* 2006, vol. 17 (8), 2245-2252
- **MOGENSEN CE ; DAMSGAARDEM ; FRLAND A et al.** Microalbuminuria in non-insulin depedent diabetes [J. *Clin Nephrol.,* 1992, vol. 38 (1), 28-39
- **CHOUDHURY D ; AHMED Z.** Drug-associated renal dysfunction and injury [J. *Nat Clin PractNephrol,* 2006, vol. 2, 80-91
- **COLARES VS ; OLIVEIRA RB ; ABDULKADER RC.** Nephrotoxicity of vancomycin in patients with normal serum creatinine [J. *Nephrol Dial Transplant,* 2006, vol. 21, 3608
- **IZZEDINE H ; LAUNAY-VACHER V ; DERAY G.** Antiviral drug-induced nephrotoxicity [J. *Am J Kidney Dis,* 2005, vol. 45, 804-817
- **UWAI Y ; IDA H ; TSUJI Y et al.** Renal transport of adefovir, cidofovir, and tenofovir by SLC22A family members (hOAT1, hOAT3, and hOCT2) [J. *Pharm Res,* 2007, vol. 24, 811-815
- **SERVAIS H ; ORTIZ A ; DEVUYST O et al.** Renal cell apoptosis induced by nephrotoxic drugs: cellular and molecular mechanisms and potential approaches to modulation [J. *Apoptosis,* 2008, vol. 13, 11-32
- **TAGUCHI T ; NAZNEEN A ; ABID MR et al.** Cisplatin-associated nephrotoxicity and pathological events [J. *ContribNephrol,* 2005, vol. 148, 107-121
- **HARIRFOROOSH S ; JAMALI F.** Renal adverse effects of nonsteroidal anti-inflammatory drugs [J. *Expert Opin Drug Saf,* 2009, vol. 8, 669-681
- **LINCOLN T.** Toxicology: danger in the diet [J. *Nature,* 2007, vol. 448, 148
- **GUITARD J ; KAMAR N ; MOUZIN M et al.** Sulfadiazine-related obstructive urinary tract lithiasis: an unusual cause of acute renal failure after kidney transplantation [J. *Clin Nephrol,* 2005, vol. 63, 405-407
- **BOUCHER BJ.** Renal failure and rhabdomyolysis associated with sitagliptin and simvastatin use. But what about the amiodarone? [J. *Diabet Med,* 2009, vol. 26, 192-193
- **HSU CY ; MCCULLOCH CE ; FAN D et al.** Community-based incidence of acute renal failure[J. *Kidney Int,* 2007, vol. 72 (2), 208-212
- **LIANGOS O ; WALD R ; O'BELL JW et al.** Epidemiology and outcomes of acute renal failure in hospitalized patients: a national survey[J. *Clin J Am Soc Nephrol,* 2006, vol. 1 (1), 43-51

- **HOSTE EA ; CLERMONT G ; KERSTEN A et al.** RIFLE criteria for acute kidney injury are associated with hospital mortality in critically ill patients: a cohort analysis[J. *Crit Care,* 2006, vol. 10 (3), R73
- **ZHANG L ; YANG YY ; FU P.** The development and problems of continuous renal replacement therapy[J. *Chin J Pract Inter Med,* 2011, vol. 31 (4), 301-304
- **RENAL REPLACEMENT THERAPY STUDY INVESTIGATORS ; BELLOMO R ; CASS A et al.** Intensity of continuous renal-replacement therapy in critically ill patients[J. *N Engl J Med,* 2009, vol. 361 (17), 1627-1638
- **VA/NIH ACUTE RENAL FAILURE TRIAL NETWORK ; PALEVSKY PM ; ZHANG JH et al.** Intensity of renal support in critically ill patients with acute kidney injury[J. *N Engl J Med,* 2008, vol. 359 (1), 7-20
- **COCA SG ; YUSUF B ; SHLIPAK MG et al.** Long-term risk of mortality and other adverse outcomes after acute kidney injury: a systematic review and meta-analysis[J. *Am J Kidney Dis,* 2009, vol. 53 (6), 961-973
- **MARDER V J ; NOVOKHATNY V.** Direct fibrinolytic agents: biochemical attributes, preclinical foundation and clinical potential [J. *Journal of Thrombosis and Haemostasis,* 2010, vol. 8 (3), 433-444
- **HUNT J A ; PETTEWAY JR S R ; SCUDERI P et al.** Simplified recombinant plasmin: production and functional comparison of a novel thrombolytic molecule with plasma-derived plasmin [J. *ThrombHaemost,* 2008, vol. 100 (3), 413-419
- **SOTTRUP-JENSEN L ; CLAEYS H ; ZAJDEL M et al.** The primary structure of human plasminogen: Isolation of two lysine-binding fragments and one ''mini''-plasminogen (MW, 38,000) by elastase-catalyzed-specific limited proteolysis [J. *Progress in chemical fibrinolysis and thrombolysis,* 1978, vol. 3, 191-209
- **NAGAI N ; DEMARSIN E ; VAN HOEF B et al.** Recombinant human microplasmin: production and potential therapeutic properties [J. *Journal of Thrombosis and Haemostasis,* 2003, vol. 1 (2), 307-313
- **CRISTHIANEFAVEROAGUIAR ; CRISTIANENAFFAH-DE-SOUZA ; ANGELA CASTOLDI et al.** Administration of α-Galactosylceramide Improves Adenine-Induced Renal Injury. *Mol Med.,* 18 June 2015, vol. 21, 553-62
- **MOUNGJAROEN J ; NIMMANNIT U ; CALLERY PS ; WANG L ; AZAD N ; LIPIPUN V ; CHANVORACHOTE P ; ROJANASAKUL Y.** Reactive oxygen species mediate caspase activation and apoptosis induced by lipoic acid in human lung epithelial cancer cells through Bcl-2 downregulation. *J Pharmacol Exp Ther,* 2006, vol. 319, 1062-1069
- **WANG L ; CHANVORACHOTE P ; TOLEDO D ; STEHLIK C ; MERCER RR ; CASTRANOVA V ; ROJANASAKUL Y.** Peroxide is a key mediator of Bcl-2 down-regulation and apoptosis induction by cisplatinin human lung cancer cells. *Mol Pharmacol,* 2008, vol. 73, 119-127
- **ZHANG M ; TAKAHASHI K ; ALICOT EM ; VORUP-JENSEN T ; KESSLER B et al.** Activation of the lectin pathway by natural IgM in a model of ischemia/ reperfusion injury. *J Immunol,* 2006, vol. 177, 4727-4734
- **KIM SJ ; GERSHOV D ; MA X ; BROT N ; ELKON KB.** I-PLA2 Activation during Apoptosis Promotes the Exposure of Membrane Lysophosphatidylcholine-Leading to Binding by Natural Immunoglobulin M Antibodies and Complement Activation. *The Journal of Experimental Medicine,* 2002, vol. 196, 655-665
- **DIMITRIOS DAVALOS ; KATERINA AKASSOGLOU.** Fibrinogen as a key regulator of inflammation in disease. *Seminars in Immunopathology,* 2012, vol. 34 (1), 43-62
- **VALVI D ; MANNINO DM ; MULLEROVA H et al.** Fibrinogen, chronic obstructive pulmonary disease (COPD) and outcomes in two United States cohorts. *Int J Chron Obstruct Pulmon Dis,* 2012, vol. 7, 173-82
- **BLANCA HUMANES ; ALBERTOLAZARO ; SONIA CAMANO et al.** Cilastatin protects against cisplatin-induced nephrotoxicity without compromising its anticancer efficiency in rats. *Kidney Int.,* September 2012, vol. 82 (6), 652-63
- **DONNELLY SM ; ZHOU XP ; HU ANG JT et al.** Prevention of early glomerulopathy with tolrestat in the streptozotocin-induced diabetic rat. *Biochem Cell Biol.,* 1996, vol. 74 (3), 355-62
- **YOSUKE KANNO ; EN SHU ; HIROYUKI KANOH ; MARIKO SEISHIMA.** The Antifibrotic Effect of a2AP Neutralization in Systemic Sclerosis Dermal Fibroblasts and Mouse Models of Systemic Sclerosis. *Journal of Investigative Dermatology,* 2016, vol. 136, 762e769
- **SZCZYPKAMS ; WESTOVERAJ ; CLOUTHIERSG et al.** Rare incorporation of bone marrow-derived cells into kidney after folic acid-induced injury. *Stem Cells,* 2005, vol. 23 (1), 44-54
- **DOMINIKA NACKIEWICZ ; PAROMITA DEY ; BARBARA SZCZERBA et al.** Inhibitor of differentiation 3, a transcription factor regulates hyperlipidemia associated kidney disease. *Nephron Exp Nephrol.,* 2014, vol. 126 (3), 141-147
- **MING GUL ; YU ZHANG ; SHENGJIE FAN et al.** Extracts of RhizomaPolygonatiOdorati Prevent High-Fat Diet-Induced Metabolic Disorders in C57BL/6 Mice. *PLoS ONE,* vol. 8 (11), e81724

- **LI YANG ; CRAIG R. BROOKS ; SHENG XIAO et al.** KIM-1-mediated phagocytosis reduces acute injuryto the kidney. *J Clin Invest.,* April 2015, vol. 125 (4), 1620-36